# EUROPEAN PATENT APPLICATION

(11) **EP 4 567 114 A1**
(43) Date of publication of application: **11.06.2025**
(21) Application number: 23850146.4
(22) Date of filing: 03.08.2023
(51) Int. Cl.: C12N 15/12, A61K 38/17, A61K 48/00, A61P 13/12, A61P 43/00, C12N 15/63

(54) **SHORTENED POLYCYSTIN-1 POLYPEPTIDE AND USE THEREOF**

(30) Priority: 04.08.2022 JP 2022124947
(71) Applicant: Otsuka Pharmaceutical Co., Ltd., Tokyo 101-8535 (JP)
(72) Inventor: NISHIKAWA, Hiroshi, Osaka-shi, Osaka 540-0021 (JP); OGURI, Koudai, Osaka-shi, Osaka 540-0021 (JP); MIZUGUCHI, Hiroshi, Osaka-shi, Osaka 540-0021 (JP); HAYASHI, Yohei, Osaka-shi, Osaka 540-0021 (JP); SAKAMAKI, Wakako, Osaka-shi, Osaka 540-0021 (JP); MINENO, Kurumi, Osaka-shi, Osaka 540-0021 (JP); HARADA, Takeo, Osaka-shi, Osaka 540-0021 (JP)
(74) Representative: Hoffmann Eitle
(86) International application number: PCT/JP2023/028405
(87) International publication number: WO 2024/029593

(57) **Abstract**

The present disclosure includes a polynucleotide comprising a sequence encoding a shortened polycystin-1 (PC1) having cyst formation inhibitory activity, wherein the shortened PC1 comprises a C-terminal tail (CTT) region of PC1 or the part thereof; a shortened PC1 polypeptide encoded by the polynucleotide; a vector comprising the polynucleotide; and a pharmaceutical composition for treating a PKD1-related disease comprising the polynucleotide, the polypeptide, or the vector.

## Description

### TECHNICAL FIELD

The present disclosure relates to shortened polycystin-1 polypeptides and uses thereof, particularly in the treatment of PKD1-related diseases.

### BACKGROUND

Autosomal dominant polycystic kidney disease (ADPKD) is the most frequent hereditary renal disease, in which multiple cysts develop and grow progressively in both kidneys and damages to various organs other than the kidneys are also observed. ADPKD is a hereditary disease whose causal genes are PKD1 and PKD2 genes. It is said that 85% of patients develops the disease due to abnormality of the PKD1 gene. The PKD1 gene encodes an 11-transmembrane protein, polycystin-1 (PC1), which is involved in regulation of Ca²⁺ influx into cells by cooperating with polycystin-2 (PC2), a 6-transmembrane protein encoded by the PKD2 gene. It is suggested that the PKD1 or PKD2 gene mutation results in loss of function of PC1 or PC2 and causes cyst formation.

### SUMMARY

### PROBLEM TO BE SOLVED

Gene therapy can be an option for the treatment of hereditary diseases such as ADPKD. The PKD1 gene, however, is as large as approximately 13 kb and too large to be transferred as a whole. Also, it has not been known which region of PC1 is necessary to restore PC1 function, and where the necessary region should be present within cells. An object of the present disclosure is to provide a shortened form of PC1 that can be used to restore PC1 function and/or use thereof.

### SOLUTION TO THE PROBLEM

In one aspect, the present disclosure provides a polynucleotide comprising a sequence encoding a shortened polycystin-1 (PC1) having cyst formation inhibitory activity, wherein the shortened PC1 comprises a C-terminal tail (CTT) region of PC1 or a part thereof.

In one aspect, the present disclosure provides a polynucleotide comprising a sequence encoding a shortened polycystin-1 (PC1), wherein the shortened PC1 is a membrane-localized polypeptide comprising a C-terminal tail (CTT) region of PC1 or a part thereof.

In one aspect, the present disclosure provides a shortened PC1 polypeptide encoded by the polynucleotide as described above.

In one aspect, the present disclosure provides a vector comprising the polynucleotide as described above.

In one aspect, the present disclosure provides a pharmaceutical composition for treating a PKD1-related disease comprising the polynucleotide or the vector as described above.

### EFFECT OF THE INVENTION

The present disclosure provides a shortened PC1 that can be used to restore PC1 function, enabling treatment, in particular by gene therapy, of a PKD1-related disease.

### BRIEF DESCRIPTION OF DRAWINGS

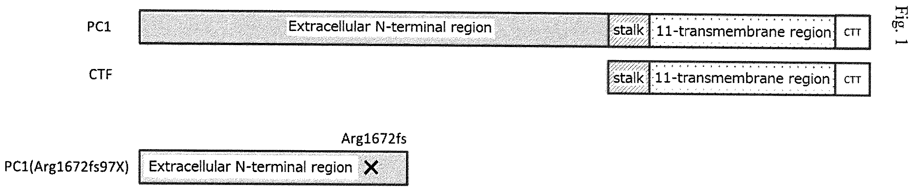

Fig. 1 shows the structures of PC1, a shortened PC1 polypeptide (CTF), and PC1 (Arg1672fs97X).

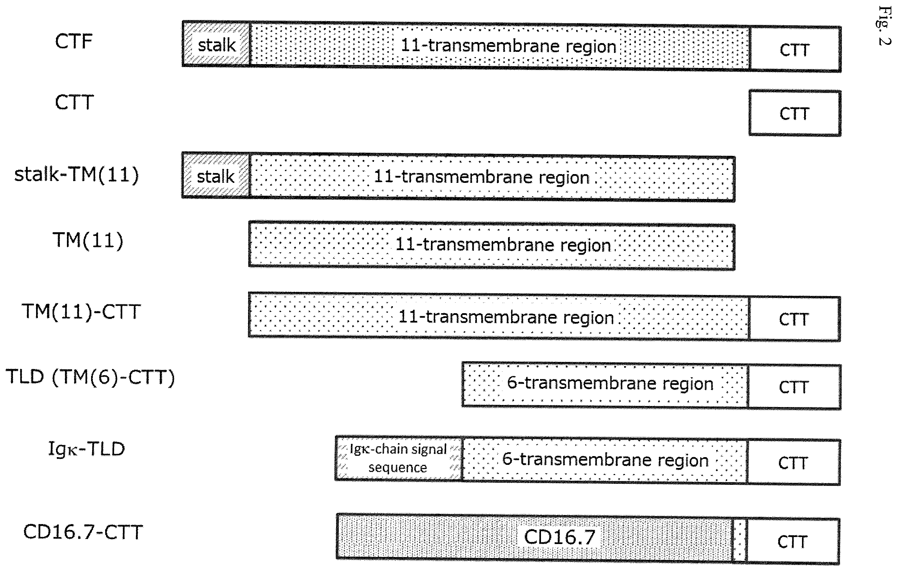

Fig. 2 shows the structures of various shortened PC1 polypeptides.
Fig. 3 shows the results of evaluation of cyst formation inhibitory effects of PC1, PC1 (Arg1672fs97X), and shortened polypeptides (CTF, CTT). The pEB indicates the control. The vertical axis indicates the total cyst area (µm²).
Fig. 4 shows the results of evaluation of cyst formation inhibitory effects of various shortened PC1 polypeptides. The pEB indicates the control. The vertical axis indicates the total cyst area (µm²).
Fig. 5 shows the structures of shortened PC1 polypeptides CD16.7-CTT and FAS-CTT.
Fig. 6 shows the results of evaluation of cyst formation inhibitory effects of CD16.7-CTT and FAS-CTT. The pEB indicates the control. The vertical axis indicates the total cyst area (µm²).
Fig. 7 shows the partial regions of a CTT region comprised in various shortened PC1 polypeptides. The number shown in the figure indicates the amino acid position in SEQ ID NO: 1. NLS: nuclear localization signal.
Fig. 8-1 shows the results of evaluation of cyst formation inhibitory effects of various shortened PC1 polypeptides each comprising a partial region of a CTT region (1). The pEB indicates the control, and the vertical axis indicates the total cyst area (µm²) (same for Figs. 8-2 to 8-4).
Fig. 8-2 shows the results of evaluation of cyst formation inhibitory effects of various shortened PC1 polypeptides each comprising a partial region of a CTT region (2).
Fig. 8-3 shows the results of evaluation of cyst formation inhibitory effects of various shortened PC1 polypeptides each comprising a partial region of a CTT region (3).
Fig. 8-4 shows the results of evaluation of cyst formation inhibitory effects of various shortened PC1 polypeptides each comprising a partial region of a CTT region (4).
Fig. 9-1 shows expression analysis of shortened PC1 polypeptides in MDCK cells by HPLC-MS (identification of peptides within the CTT region). The vertical axis (Abundance) indicates the detection intensity of peptides by HPLC-MS (same for Fig. 9-2 and Figs. 10-1 to 10-2).
Fig. 9-2 shows expression analysis of shortened PC1 polypeptides in MDCK cells by HPLC-MS (identification of peptides within the CTF 11-transmembrane region).
Fig. 10-1 shows cell membrane localization analysis of shortened PC1 polypeptides in MDCK cells by HPLC-MS (identification of peptides within the CTT region).
Fig. 10-2 shows cell membrane localization analysis of shortened PC1 polypeptides in MDCK cells by HPLC-MS (identification of peptides within the CTF 11-transmembrane region).

### DETAILED DESCRIPTION

Unless otherwise specified, the terms used herein have meanings as commonly understood by those of ordinary skill in the technical fields such as organic chemistry, medical science, pharmaceutical science, molecular biology, and microbiology. Definitions for some terms used herein are provided below and these definitions supersede general understandings herein.

The PKD1 gene encodes polycystin-1 (PC1), an 11-transmembrane protein with an extracellular N-terminus and an intracellular C-terminus. PC1 forms a complex with PC2, a 6-transmembrane protein with an intracellular N-terminus and an intracellular C-terminus, at the coiled-coil domain of its intracellular region, and signals PC2, which functions as a calcium ion channel, to induce Ca²⁺ influx into cells.

PC1 has a GPS (G protein-coupled receptor proteolytic site) as an enzymatic cleavage site and is composed of an extracellular N-terminal region on the N-terminal side of the GPS and a C-terminal fragment (CTF) region on the C-terminal side of the GPS. The CTF region has a stalk, an 11-transmembrane region, and a C-terminal tail (CTT) region. The stalk is the N-terminal extracellular region of the CTF region, i.e., the region from the N-terminus of the CTF region to the first transmembrane region (TM1). In the present disclosure, PC1 can be mammalian PC1, such as human or non-human mammalian PC1. In one embodiment, PC1 is human PC1. A representative amino acid sequence of human PC1 is shown in SEQ ID NO: 1, and a nucleotide sequence encoding the same is shown in SEQ ID NO: 2. Human PC1 and a nucleotide sequence encoding the same are not limited to those having SEQ ID NOs: 1 and 2, respectively. For example, human PC1 may have an amino acid sequence that differs from the amino acid sequence of SEQ ID NO: 1 in that it comprises naturally-occurring mutation(s) such as SNP(s). Also, the nucleotide sequence encoding human PC1 may have a sequence different from the sequence of SEQ ID NO: 2 due to degeneracy of the genetic code. A person skilled in the art can identify each region of PC1 according to existing reports (Nat Genet, 1995 Jun;10(2):151-60; Biochemistry 2003, 42, 13035-13048; Science 361, 992 (2018); Dev Cell 22, 197-210, Jan 7, 2012), or using any of amino acid domain/motif databases or transmembrane region prediction tools such as Uniprot (https://www.uniprot.org/), NCBI (https://www.ncbi.nlm.nih.gov/), and predictprotein (https://predictprotein.org/). In SEQ ID NO: 1, the extracellular N-terminal region, stalk, 11-transmembrane region, and CTT region are identified as amino acid sequences at position 1 to 3048, 3049 to 3074, 3075 to 4101, and 4102 to 4303, respectively.

A shortened PC1 is a polypeptide lacking part of an amino acid sequence of a full-length PC1. A shortened PC1 may comprise a sequence not derived from PC1 in addition to a partial sequence of PC1. The length of the partial sequence of PC1 in a shortened PC1 may be 4300, 4200, 4100, 4000, 3900, 3800, 3700, 3600, 3500, 3400, 3300, 3200, 3100, 3000, 2900, 2800, 2700, 2600, 2500, 2400, 2300, 2200, 2100, 2000, 1900, 1800, 1700, 1600, 1500, 1400, 1300, 1200, 1100, 1000, 900, 800, 700, 600, 500, 400, 300, 290, 280, 270, 260, 250, 240, 230, 220, 210, 200, 190, 180, 170, 160, 150, 140, 130, 120, 110, 100, 90, 80, 70, 60, or 50 amino acids or less, and 40, 50, 60, 70, 80, 90, 100, 110, 120, 130, 140, 150, 160, 170, 180, 190, 200, 210, 220, 230, 240, 250, 260, 270, 280, 290, 300, 400, 500, 600, 700, 800, 900, 1000, 1100, 1200, 1300, 1400, 1500, 1600, 1700, 1800, 1900, or 2000 amino acids or more. The length of the partial sequence of PC1 in a shortened PC1 may be 140 to 260, 150 to 250, 160 to 240, 170 to 230, 180 to 220, or 190 to 210 amino acids, such as 226, 202, or 190 amino acids. Alternatively, the length of the partial sequence of PC1 in a shortened PC1 may be 40 to 200, 50 to 150, or 50 to 100 amino acids. The partial sequence of PC1 in a shortened PC1 may be a continuous sequence in a full-length PC1, or may consist of a plurality of sequences that are not continued in a full-length PC1. In one embodiment, the shortened PC1 lacks at least part of the extracellular N-terminal region of PC1. In one embodiment, the shortened PC1 lacks the whole of the extracellular N-terminal region of PC1. In one embodiment, the shortened PC1 lacks the whole of the extracellular N-terminal region and also lacks at least part of the stalk and the 11-transmembrane region of PC1. In one embodiment, the shortened PC1 lacks all of the whole of extracellular N-terminal region, the stalk, and the 11-transmembrane region of PC1. The length of a shortened PC1 may be 4300, 4200, 4100, 4000, 3900, 3800, 3700, 3600, 3500, 3400, 3300, 3200, 3100, 3000, 2900, 2800, 2700, 2600, 2500, 2400, 2300, 2200, 2100, 2000, 1900, 1800, 1700, 1600, 1500, 1400, 1300, 1200, 1100, 1000, 900, 800, 700, 600, 500, 400, 300, 290, 280, 270, 260, 250, 240, 230, 220, 210, 200, 190, 180, 170, 160, 150, 140, 130, 120, 110, 100, 90, 80, 70, 60, or 50 amino acids or less, and 40, 50, 60, 70, 80, 90, 100, 110, 120, 130, 140, 150, 160, 170, 180, 190, 200, 210, 220, 230, 240, 250, 260, 270, 280, 290, 300, 400, 500, 600, 700, 800, 900, 1000, 1100, 1200, 1300, 1400, 1500, 1600, 1700, 1800, 1900, or 2000 amino acids or more.

The shortened PC1 of the present disclosure comprises a CTT region or a part thereof. As used herein, comprising "a CTT region or a part thereof" is synonymously used with comprising at least part of a CTT region. The CTT region is the intracellular region of PC1. The length of the CTT region may be 230, 220, 210, 200, 190, 180, 170, 160, 150, or 140 amino acids or less, and 140, 150, 160, 170, 180, 190, or 200 amino acids or more. For example, the length of the CTT region may be 140 to 260, 150 to 250, 160 to 240, 170 to 230, 180 to 220, 190 to 210 amino acids, such as 226, 202, or 190 amino acids.

In SEQ ID NO: 1, the CTT region can be identified as the amino acid sequence at positions 4102 to 4303 (SEQ ID NO: 3). In SEQ ID NO: 2, the sequence encoding the CTT region can be identified as the nucleotide sequence at positions 12304 to 12909 (SEQ ID NO: 4).

In one embodiment, the CTT region comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 3. In one embodiment, the CTT region comprises or consists of the sequence of SEQ ID NO: 3, or a sequence wherein 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 3. In one embodiment, the CTT region comprises or consists of the sequence of SEQ ID NO: 3. In one embodiment, the CTT region comprises or consists of the sequence at positions 3 to 202 of SEQ ID NO: 3.

The sequence of a polynucleotide encoding the CTT region is not particularly limited as long as it encodes the CTT region described herein. In one embodiment, the sequence encoding the CTT region comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 3. In one embodiment, the sequence encoding the CTT region comprises or consists of a sequence encoding the sequence of SEQ ID NO: 3 or a sequence wherein 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 3. In one embodiment, the sequence encoding the CTT region comprises or consists of a sequence encoding the sequence of SEQ ID NO: 3. In one embodiment, the sequence encoding the CTT region comprises or consists of a sequence encoding the sequence at positions 3 to 202 of SEQ ID NO: 3.

In one embodiment, the sequence encoding the CTT region comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 4. In one embodiment, the sequence encoding the CTT region comprises or consists of the sequence of SEQ ID NO: 4 or a sequence wherein 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 4. In one embodiment, the sequence encoding the CTT region comprises or consists of the sequence of SEQ ID NO: 4. In one embodiment, the sequence encoding the CTT region comprises or consists of the sequence at positions 7 to 606 of SEQ ID NO: 4. The sequence encoding the CTT region may have a sequence different from the sequence of SEQ ID NO: 4, or the sequence at positions 7 to 606 of SEQ ID NO: 4, due to degeneracy of the genetic code.

A part of a CTT region may be any portion of the CTT region. The length of the part of a CTT region may be 200, 190, 180, 170, 160, 150, 140, 130, 120, 110, 100, 90, 80, 70, 60, or 50 amino acids or less, and 40, 50, 60, 70, 80, 90, or 100 amino acids or more. For example, the length of the part of a CTT region may be 40 to 200, 50 to 150, or 50 to 100 amino acids.

In one embodiment, the part of a CTT region comprises or consists of at least part of a sequence having 70%, 75%, 80%, 85%, 90%, 91%, 92%, 93%, 94%, 95%, 96%, 97%, 98%, or 99% or more sequence identity with the sequence at positions 4154 to 4303 of SEQ ID NO: 1. In one embodiment, the part of a CTT region comprises or consists of at least part of the sequence at positions 4154 to 4303 of SEQ ID NO: 1, or a sequence wherein 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence at positions 4154 to 4303 of SEQ ID NO: 1. In one embodiment, the part of a CTT region comprises or consists of at least part of the sequence at positions 4154 to 4303 of SEQ ID NO: 1.

In one embodiment, the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 4204 to 4243 of SEQ ID NO: 1. In one embodiment, the part of a CTT region comprises or consists of the sequence at positions 4204 to 4243 of SEQ ID NO: 1, or a sequence wherein 1 to 15, 1 to 10, 1 to 5, 1 to 4, or 1, 2, or 3 amino acids are modified in the sequence at positions 4204 to 4243 of SEQ ID NO: 1. In one embodiment, the part of a CTT region comprises or consists of the sequence at positions 4204 to 4243 of SEQ ID NO: 1.

In one embodiment, the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 4104 to 4203 of SEQ ID NO: 1. In one embodiment, the part of a CTT region comprises or consists of the sequence at positions 4104 to 4203 of SEQ ID NO: 1, or a sequence wherein 1 to 30, 1 to 25, 1 to 20, 1 to 15, 1 to 10, 1 to 5, 1 to 4, or 1, 2, or 3 amino acids are modified in the sequence at positions 4104 to 4203 of SEQ ID NO: 1. In one embodiment, the part of a CTT region comprises or consists of the sequence at positions 4104 to 4203 of SEQ ID NO: 1.

In one embodiment, the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 4154 to 4253 of SEQ ID NO: 1. In one embodiment, the part of a CTT region comprises or consists of the sequence at positions 4154 to 4253 of SEQ ID NO: 1, or a sequence wherein 1 to 30, 1 to 25, 1 to 20, 1 to 15, 1 to 10, 1 to 5, 1 to 4, or 1, 2, or 3 amino acids are modified in the sequence at positions 4154 to 4253 of SEQ ID NO: 1. In one embodiment, the part of a CTT region comprises or consists of the sequence at positions 4154 to 4253 of SEQ ID NO: 1.

In one embodiment, the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 4154 to 4243 of SEQ ID NO: 1. In one embodiment, the part of a CTT region comprises or consists of the sequence at positions 4154 to 4243 of SEQ ID NO: 1, or a sequence wherein 1 to 30, 1 to 25, 1 to 20, 1 to 15, 1 to 10, 1 to 5, 1 to 4, or 1, 2, or 3 amino acids are modified in the sequence at positions 4154 to 4243 of SEQ ID NO: 1. In one embodiment, the part of a CTT region comprises or consists of the sequence at positions 4154 to 4243 of SEQ ID NO: 1.

In one embodiment, the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 4193 to 4303 of SEQ ID NO: 1. In one embodiment, the part of a CTT region comprises or consists of the sequence at positions 4193 to 4303 of SEQ ID NO: 1, or a sequence wherein 1 to 30, 1 to 25, 1 to 20, 1 to 15, 1 to 10, 1 to 5, 1 to 4, or 1, 2, or 3 amino acids are modified in the sequence at positions 4193 to 4303 of SEQ ID NO: 1. In one embodiment, the part of a CTT region comprises or consists of the sequence at positions 4193 to 4303 of SEQ ID NO: 1.

In one embodiment, the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 4204 to 4303 of SEQ ID NO: 1. In one embodiment, the part of a CTT region comprises or consists of the sequence at positions 4204 to 4303 of SEQ ID NO: 1, or a sequence wherein 1 to 30, 1 to 25, 1 to 20, 1 to 15, 1 to 10, 1 to 5, 1 to 4, or 1, 2, or 3 amino acids are modified in the sequence at positions 4204 to 4303 of SEQ ID NO: 1. In one embodiment, the part of a CTT region comprises or consists of the sequence at positions 4204 to 4303 of SEQ ID NO: 1.

In one embodiment, the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 4204 to 4253 of SEQ ID NO: 1. In one embodiment, the part of a CTT region comprises or consists of the sequence at positions 4204 to 4253 of SEQ ID NO: 1, or a sequence wherein 1 to 15, 1 to 10, 1 to 5, 1 to 4, or 1, 2, or 3 amino acids are modified in the sequence at positions 4204 to 4253 of SEQ ID NO: 1. In one embodiment, the part of a CTT region comprises or consists of the sequence at positions 4204 to 4253 of SEQ ID NO: 1.

In one embodiment, the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 4254 to 4303 of SEQ ID NO: 1. In one embodiment, the part of a CTT region comprises or consists of the sequence at positions 4254 to 4303 of SEQ ID NO: 1, or a sequence wherein 1 to 15, 1 to 10, 1 to 5, 1 to 4, or 1, 2, or 3 amino acids are modified in the sequence at positions 4254 to 4303 of SEQ ID NO: 1. In one embodiment, the part of a CTT region comprises or consists of the sequence at positions 4254 to 4303 of SEQ ID NO: 1.

The sequence of a polynucleotide encoding the part of a CTT region is not particularly limited as long as it encodes the part of a CTT region described herein. In one embodiment, the sequence encoding the part of a CTT region comprises or consists of a sequence encoding at least part of a sequence having 70%, 75%, 80%, 85%, 90%, 91%, 92%, 93%, 94%, 95%, 96%, 97%, 98%, or 99% or more sequence identity with the sequence at positions 4154 to 4303 of SEQ ID NO: 1. In one embodiment, the sequence encoding the part of a CTT region comprises or consists of a sequence encoding at least part of the sequence at positions 4154 to 4303 of SEQ ID NO: 1 or a sequence wherein 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence at positions 4154 to 4303 of SEQ ID NO: 1. In one embodiment, the sequence encoding the part of a CTT region comprises or consists of a sequence encoding at least part of the sequence at positions 4154 to 4303 of SEQ ID NO: 1.

In one embodiment, the sequence encoding the part of a CTT region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 4204 to 4243 of SEQ ID NO: 1. In one embodiment, the sequence encoding the part of a CTT region comprises or consists of a sequence encoding the sequence at positions 4204 to 4243 of SEQ ID NO: 1 or a sequence wherein 1 to 15, 1 to 10, 1 to 5, 1 to 4, or 1, 2, or 3 amino acids are modified in the sequence at positions 4204 to 4243 of SEQ ID NO: 1. In one embodiment, the sequence encoding the part of a CTT region comprises or consists of a sequence encoding the sequence at positions 4204 to 4243 of SEQ ID NO: 1.

In one embodiment, the sequence encoding the part of a CTT region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 4104 to 4203 of SEQ ID NO: 1. In one embodiment, the sequence encoding the part of a CTT region comprises or consists of a sequence encoding the sequence at positions 4104 to 4203 of SEQ ID NO: 1 or a sequence wherein 1 to 30, 1 to 25, 1 to 20, 1 to 15, 1 to 10, 1 to 5, 1 to 4, or 1, 2, or 3 amino acids are modified in the sequence at positions 4104 to 4203 of SEQ ID NO: 1. In one embodiment, the sequence encoding the part of a CTT region comprises or consists of a sequence encoding the sequence at positions 4104 to 4203 of SEQ ID NO: 1.

In one embodiment, the sequence encoding the part of a CTT region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 4154 to 4253 of SEQ ID NO: 1. In one embodiment, the sequence encoding the part of a CTT region comprises or consists of a sequence encoding the sequence at positions 4154 to 4253 of SEQ ID NO: 1 or a sequence wherein 1 to 30, 1 to 25, 1 to 20, 1 to 15, 1 to 10, 1 to 5, 1 to 4, or 1, 2, or 3 amino acids are modified in the sequence at positions 4154 to 4253 of SEQ ID NO: 1. In one embodiment, the sequence encoding the part of a CTT region comprises or consists of a sequence encoding the sequence at positions 4154 to 4253 of SEQ ID NO: 1.

In one embodiment, the sequence encoding the part of a CTT region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 4154 to 4243 of SEQ ID NO: 1. In one embodiment, the sequence encoding the part of a CTT region comprises or consists of a sequence encoding the sequence at positions 4154 to 4243 of SEQ ID NO: 1 or a sequence wherein 1 to 30, 1 to 25, 1 to 20, 1 to 15, 1 to 10, 1 to 5, 1 to 4, or 1, 2, or 3 amino acids are modified in the sequence at positions 4154 to 4243 of SEQ ID NO: 1. In one embodiment, the sequence encoding the part of a CTT region comprises or consists of a sequence encoding the sequence at positions 4154 to 4243 of SEQ ID NO: 1.

In one embodiment, the sequence encoding the part of a CTT region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 4193 to 4303 of SEQ ID NO: 1. In one embodiment, the sequence encoding the part of a CTT region comprises or consists of a sequence encoding the sequence at positions 4193 to 4303 of SEQ ID NO: 1 or a sequence wherein 1 to 30, 1 to 25, 1 to 20, 1 to 15, 1 to 10, 1 to 5, 1 to 4, or 1, 2, or 3 amino acids are modified in the sequence at positions 4193 to 4303 of SEQ ID NO: 1. In one embodiment, the sequence encoding the part of a CTT region comprises or consists of a sequence encoding the sequence at positions 4193 to 4303 of SEQ ID NO: 1.

In one embodiment, the sequence encoding the part of a CTT region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 4204 to 4303 of SEQ ID NO: 1. In one embodiment, the sequence encoding the part of a CTT region comprises or consists of a sequence encoding the sequence at positions 4204 to 4303 of SEQ ID NO: 1 or a sequence wherein 1 to 30, 1 to 25, 1 to 20, 1 to 15, 1 to 10, 1 to 5, 1 to 4, or 1, 2, or 3 amino acids are modified in the sequence at positions 4204 to 4303 of SEQ ID NO: 1. In one embodiment, the sequence encoding the part of a CTT region comprises or consists of a sequence encoding the sequence at positions 4204 to 4303 of SEQ ID NO: 1.

In one embodiment, the sequence encoding the part of a CTT region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 4204 to 4253 of SEQ ID NO: 1. In one embodiment, the sequence encoding the part of a CTT region comprises or consists of a sequence encoding the sequence at positions 4204 to 4253 of SEQ ID NO: 1 or a sequence wherein 1 to 15, 1 to 10, 1 to 5, 1 to 4, or 1, 2, or 3 amino acids are modified in the sequence at positions 4204 to 4253 of SEQ ID NO: 1. In one embodiment, the sequence encoding the part of a CTT region comprises or consists of a sequence encoding the sequence at positions 4204 to 4253 of SEQ ID NO: 1.

In one embodiment, the sequence encoding the part of a CTT region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 4254 to 4303 of SEQ ID NO: 1. In one embodiment, the sequence encoding the part of a CTT region comprises or consists of a sequence encoding the sequence at positions 4254 to 4303 of SEQ ID NO: 1 or a sequence wherein 1 to 15, 1 to 10, 1 to 5, 1 to 4, or 1, 2, or 3 amino acids are modified in the sequence at positions 4254 to 4303 of SEQ ID NO: 1. In one embodiment, the sequence encoding the part of a CTT region comprises or consists of a sequence encoding the sequence at positions 4254 to 4303 of SEQ ID NO: 1.

In one embodiment, the sequence encoding the part of a CTT region comprises or consists of at least part of a sequence having 70%, 75%, 80%, 85%, 90%, 91%, 92%, 93%, 94%, 95%, 96%, 97%, 98%, or 99% or more sequence identity with the sequence at positions 12460 to 12909 of SEQ ID NO: 2. In one embodiment, the sequence encoding the part of a CTT region comprises or consists of at least part of the sequence at positions 12460 to 12909 of SEQ ID NO: 2 or a sequence wherein 1 to 135, 1 to 130, 1 to 125, 1 to 120, 1 to 115, 1 to 110, 1 to 105, 1 to 100, 1 to 95, 1 to 90, 1 to 85, 1 to 80, 1 to 75, 1 to 70, 1 to 65, 1 to 60, 1 to 55, 1 to 50, 1 to 45,1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 12460 to 12909 of SEQ ID NO: 2. In one embodiment, the sequence encoding the part of a CTT region comprises or consists of at least part of the sequence at positions 12460 to 12909 of SEQ ID NO: 2. The sequence encoding the part of a CTT region may have a sequence different from the at least part of the sequence at positions 12460 to 12909 of SEQ ID NO: 2 due to degeneracy of the genetic code.

In one embodiment, the sequence encoding the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 12610 to 12729 of SEQ ID NO: 2. In one embodiment, the sequence encoding the part of a CTT region comprises or consists of the sequence at positions 12610 to 12729 of SEQ ID NO: 2 or a sequence wherein 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 12610 to 12729 of SEQ ID NO: 2. In one embodiment, the sequence encoding the part of a CTT region comprises or consists of the sequence at positions 12610 to 12729 of SEQ ID NO: 2. The sequence encoding the part of a CTT region may have a sequence different from the sequence at positions 12610 to 12729 of SEQ ID NO: 2 due to degeneracy of the genetic code.

In one embodiment, the sequence encoding the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 12310 to 12609 of SEQ ID NO: 2. In one embodiment, the sequence encoding the part of a CTT region comprises or consists of the sequence at positions 12310 to 12609 of SEQ ID NO: 2 or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 12310 to 12609 of SEQ ID NO: 2. In one embodiment, the sequence encoding the part of a CTT region comprises or consists of the sequence at positions 12310 to 12609 of SEQ ID NO: 2. The sequence encoding the part of a CTT region may have a sequence different from the sequence at positions 12310 to 12609 of SEQ ID NO: 2 due to degeneracy of the genetic code.

In one embodiment, the sequence encoding the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 12460 to 12759 of SEQ ID NO: 2. In one embodiment, the sequence encoding the part of a CTT region comprises or consists of the sequence at positions 12460 to 12759 of SEQ ID NO: 2 or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 12460 to 12759 of SEQ ID NO: 2. In one embodiment, the sequence encoding the part of a CTT region comprises or consists of the sequence at positions 12460 to 12759 of SEQ ID NO: 2. The sequence encoding the part of a CTT region may have a sequence different from the sequence at positions 12460 to 12759 of SEQ ID NO: 2 due to degeneracy of the genetic code.

In one embodiment, the sequence encoding the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 12460 to 12729 of SEQ ID NO: 2. In one embodiment, the sequence encoding the part of a CTT region comprises or consists of the sequence at positions 12460 to 12729 of SEQ ID NO: 2 or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 12460 to 12729 of SEQ ID NO: 2. In one embodiment, the sequence encoding the part of a CTT region comprises or consists of the sequence at positions 12460 to 12729 of SEQ ID NO: 2. The sequence encoding the part of a CTT region may have a sequence different from the sequence at positions 12460 to 12729 of SEQ ID NO: 2 due to degeneracy of the genetic code.

In one embodiment, the sequence encoding the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 12577 to 12909 of SEQ ID NO: 2. In one embodiment, the sequence encoding the part of a CTT region comprises or consists of the sequence at positions 12577 to 12909 of SEQ ID NO: 2 or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 12577 to 12909 of SEQ ID NO: 2. In one embodiment, the sequence encoding the part of a CTT region comprises or consists of the sequence at positions 12577 to 12909 of SEQ ID NO: 2. The sequence encoding the part of a CTT region may have a sequence different from the sequence at positions 12577 to 12909 of SEQ ID NO: 2 due to degeneracy of the genetic code.

In one embodiment, the sequence encoding the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 12610 to 12909 of SEQ ID NO: 2. In one embodiment, the sequence encoding the part of a CTT region comprises or consists of the sequence at positions 12610 to 12909 of SEQ ID NO: 2 or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 12610 to 12909 of SEQ ID NO: 2. In one embodiment, the sequence encoding the part of a CTT region comprises or consists of the sequence at positions 12610 to 12909 of SEQ ID NO: 2. The sequence encoding the part of a CTT region may have a sequence different from the sequence at positions 12610 to 12909 of SEQ ID NO: 2 due to degeneracy of the genetic code.

In one embodiment, the sequence encoding the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 12610 to 12759 of SEQ ID NO: 2. In one embodiment, the sequence encoding the part of a CTT region comprises or consists of the sequence at positions 12610 to 12759 of SEQ ID NO: 2 or a sequence wherein 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 12610 to 12759 of SEQ ID NO: 2. In one embodiment, the sequence encoding the part of a CTT region comprises or consists of the sequence at positions 12610 to 12759 of SEQ ID NO: 2. The sequence encoding the part of a CTT region may have a sequence different from the sequence at positions 12610 to 12759 of SEQ ID NO: 2 due to degeneracy of the genetic code.

In one embodiment, the sequence encoding the part of a CTT region comprises or consists of a sequence having 70%, 75%, 80%, 85%, 90%, 91%, 92%, 93%, 94%, 95%, 96%, 97%, 98%, or 99% or more sequence identity with the sequence at positions 12760 to 12909 of SEQ ID NO: 2. In one embodiment, the sequence encoding the part of a CTT region comprises or consists of the sequence at positions 12760 to 12909 of SEQ ID NO: 2 or a sequence wherein 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 12760 to 12909 of SEQ ID NO: 2. In one embodiment, the sequence encoding the part of a CTT region comprises or consists of the sequence at positions 12760 to 12909 of SEQ ID NO: 2. The sequence encoding the part of a CTT region may have a sequence different from the sequence at positions 12760 to 12909 of SEQ ID NO: 2 due to degeneracy of the genetic code.

In one embodiment, the shortened PC1 is a membrane-localized polypeptide. A membrane-localized polypeptide means a polypeptide that localizes to the cell membrane when expressed in a cell.

In one embodiment, the shortened PC1 further comprises at least part of the stalk of PC1 in addition to the CTT region or a part thereof. In one embodiment, the shortened PC1 further comprises, in addition to the CTT region, at least part of the stalk and at least one transmembrane region of PC1. In one embodiment, the shortened PC1 further comprises a signal sequence in addition to the CTT region. In one embodiment, the shortened PC1 further comprises, in addition to the CTT region, a signal sequence and at least one transmembrane region.

In one embodiment, the at least part of the stalk consists of 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids, such as 25 or 26 amino acids. In one embodiment, the at least part of the stalk comprises or consists of a sequence having 70%, 75%, 80%, 85%, 90%, 91%, 92%, 93%, 94%, 95%, 96%, 97%, 98%, or 99% or more sequence identity with the sequence at positions 3049 to 3074 of SEQ ID NO: 1. In one embodiment, the at least part of the stalk comprises or consists of the sequence at positions 3049 to 3074 of SEQ ID NO: 1, or a sequence wherein 1 to 5, 1 to 4, or 1, 2, or 3 amino acids are modified in the sequence at positions 3049 to 3074 of SEQ ID NO: 1. In one embodiment, the at least part of the stalk comprises or consists of the sequence at positions 3049 to 3074 of SEQ ID NO: 1.

The sequence of a polynucleotide encoding the at least part of the stalk is not particularly limited as long as it encodes the at least part of the stalk described herein. In one embodiment, the sequence encoding the at least part of the stalk comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 3049 to 3074 of SEQ ID NO: 1. In one embodiment, the sequence encoding the at least part of the stalk is a sequence encoding the sequence at positions 3049 to 3074 of SEQ ID NO: 1, or a sequence wherein 1 to 5, 1 to 4, or 1, 2, or 3 amino acids are modified in the sequence at positions 3049 to 3074 of SEQ ID NO: 1. In one embodiment, the sequence encoding the at least part of the stalk comprises or consists of a sequence encoding the sequence at positions 3049 to 3074 of SEQ ID NO: 1.

In one embodiment, the sequence encoding the at least part of the stalk comprises or consists of a sequence having 70%, 75%, 80%, 85%, 90%, 91%, 92%, 93%, 94%, 95%, 96%, 97%, 98%, or 99% or more sequence identity with the sequence at positions 9145 to 9222 of SEQ ID NO: 2. In one embodiment, the sequence encoding the at least part of the stalk is the sequence at positions 9145 to 9222 of SEQ ID NO: 2, or a sequence wherein 1 to 15, 1 to 10, 1 to 5, 1 to 4, or 1, 2, or 3 nucleotides are modified in the sequence at positions 9145 to 9222 of SEQ ID NO: 2. In one embodiment, the sequence encoding the at least part of the stalk comprises or consists of the sequence at positions 9145 to 9222 of SEQ ID NO: 2. The sequence encoding the at least part of the stalk may have a sequence different from the sequence at positions 9145 to 9222 of SEQ ID NO: 2 due to degeneracy of the genetic code.

A transmembrane region is a region of a polypeptide that spans the cell membrane, which is the lipid bilayer. In the shortened PC1 of the present disclosure, the transmembrane region can be a transmembrane region derived from any protein as long as it can immobilize the shortened PC1 to the cell membrane. Examples of transmembrane regions include transmembrane regions of PC1 as well as transmembrane regions of T cell receptor (TCR) α and β chains, CD3ε, CD4, CD5, CD7, CD8, CD9, CD16, CD22, CD27, CD28, CD33, CD37, CD45, CD64, CD80, CD86, CD95, CD134, CD137, CD154, PKD2, VSV-G (vesicular stomatitis virus G protein), and PLAP (placental alkaline phosphatase).

In one embodiment, the at least one transmembrane region comprises 1, 2, 3, 4, 5, 6, 7, 8, 9, 10, 11, 12, 13, 14, 15, 16, 17, 18, 19, or 20 transmembrane regions.

PC1 has 11 transmembrane regions (referred to as TM1, TM2, TM3, TM4, TM5, TM6, TM7, TM8, TM9, TM10, and TM11 from the N-terminus). In one embodiment, the at least one transmembrane region comprises at least one transmembrane region of PC1 or a part thereof. In one embodiment, the at least one transmembrane region of PC1 comprises TM1 to TM11, TM2 to TM11, TM3 to TM11, TM4 to TM11, TM5 to TM11, TM6 to TM11, TM7 to TM11, TM8 to TM11, TM9 to TM11, TM10 to TM11, or TM11 of PC1. In one embodiment, the at least one transmembrane region comprises TM1 to TM11 or TM6 to TM11.

As used herein, when the "at least one transmembrane region" includes two or more transmembrane regions, the amino acid sequence of the "at least one transmembrane region" also comprises an amino acid sequence(s) of the extracellular region and/or intracellular region between the transmembrane regions. In one embodiment, the at least one transmembrane region comprises or consists of a sequence having 70%, 75%, 80%, 85%, 90%, 91%, 92%, 93%, 94%, 95%, 96%, 97%, 98%, or 99% or more sequence identity with the sequence at positions 3075 to 4101 of SEQ ID NO: 1. In one embodiment, the at least one transmembrane region comprises or consists of the sequence at positions 3075 to 4101 of SEQ ID NO: 1, or a sequence wherein 1 to 300, 1 to 290, 1 to 280, 1 to 270, 1 to 260, 1 to 250, 1 to 240, 1 to 230, 1 to 220, 1 to 210, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence at positions 3075 to 4101 of SEQ ID NO: 1. In one embodiment, the at least one transmembrane region comprises or consists of the sequence at positions 3075 to 4101 of SEQ ID NO: 1.

In one embodiment, the at least one transmembrane region comprises or consists of a sequence having 70%, 75%, 80%, 85%, 90%, 91%, 92%, 93%, 94%, 95%, 96%, 97%, 98%, or 99% or more sequence identity with the sequence at positions 3602-4101 of SEQ ID NO: 1. In one embodiment, the at least one transmembrane region is the sequence at positions 3602 to 4101 of SEQ ID NO: 1, or a sequence wherein 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence at positions 3602 to 4101 of SEQ ID NO: 1. In one embodiment, the at least one transmembrane region comprises or consists of the sequence at positions 3602 to 4101 of SEQ ID NO: 1.

In one embodiment, the at least one transmembrane region comprises or consists of a sequence having 70%, 75%, 80%, 85%, 90%, 91%, 92%, 93%, 94%, 95%, 96%, 97%, 98%, or 99% or more sequence identity with the sequence at positions 244 to 264 of SEQ ID NO: 21. In one embodiment, the at least one transmembrane region comprises or consists of the sequence at positions 244 to 264 of SEQ ID NO: 21, or a sequence wherein 1 to 5, 1 to 4, or 1, 2, or 3 amino acids are modified in the sequence at positions 244 to 264 of SEQ ID NO: 21. In one embodiment, the at least one transmembrane region comprises or consists of the sequence at positions 244 to 264 of SEQ ID NO: 21.

In one embodiment, the at least one transmembrane region comprises or consists of a sequence having 70%, 75%, 80%, 85%, 90%, 91%, 92%, 93%, 94%, 95%, 96%, 97%, 98%, or 99% or more sequence identity with the sequence at positions 174 to 190 of SEQ ID NO: 24. In one embodiment, the at least one transmembrane region comprises or consists of the sequence at positions 174 to 190 of SEQ ID NO: 24, or a sequence wherein 1 to 5, 1 to 4, or 1, 2, or 3 amino acids are modified in the sequence at positions 174 to 190 of SEQ ID NO: 24. In one embodiment, the at least one transmembrane region comprises or consists of the sequence at positions 174 to 190 of SEQ ID NO: 24.

The sequence of a polynucleotide encoding the transmembrane region is not particularly limited as long as it encodes the transmembrane region described herein. In one embodiment, the sequence encoding the at least one transmembrane region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 3075 to 4101 of SEQ ID NO: 1. In one embodiment, the sequence encoding the at least one transmembrane region comprises or consists of a sequence encoding the sequence at positions 3075 to 4101 of SEQ ID NO: 1, or a sequence wherein 1 to 300, 1 to 290, 1 to 280, 1 to 270, 1 to 260, 1 to 250, 1 to 240, 1 to 230, 1 to 220, 1 to 210, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence at positions 3075 to 4101 of SEQ ID NO: 1. In one embodiment, the sequence encoding the at least one transmembrane region comprises or consists of a sequence encoding the sequence at positions 3075 to 4101 of SEQ ID NO: 1.

In one embodiment, the sequence encoding the at least one transmembrane region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 3602-4101 of SEQ ID NO: 1. In one embodiment, the sequence encoding the at least one transmembrane region comprises or consists of a sequence encoding the sequence at positions 3602 to 4101 of SEQ ID NO: 1, or a sequence wherein 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence at positions 3602 to 4101 of SEQ ID NO: 1. In one embodiment, the sequence encoding the at least one transmembrane region comprises or consists of a sequence encoding the sequence at positions 3602 to 4101 of SEQ ID NO: 1.

In one embodiment, the sequence encoding the at least one transmembrane region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 244 to 264 of SEQ ID NO: 21. In one embodiment, the sequence encoding the at least one transmembrane region comprises or consists of a sequence encoding the sequence at positions 244 to 264 of SEQ ID NO: 21, or a sequence wherein 1 to 5, 1 to 4, or 1, 2, or 3 amino acids are modified in the sequence at positions 244 to 264 of SEQ ID NO: 21. In one embodiment, the sequence encoding the at least one transmembrane region comprises or consists of a sequence encoding the sequence at positions 244 to 264 of SEQ ID NO: 21.

In one embodiment, the sequence encoding the at least one transmembrane region comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 174 to 190 of SEQ ID NO: 24. In one embodiment, the sequence encoding the at least one transmembrane region comprises or consists of a sequence encoding the sequence at positions 174 to 190 of SEQ ID NO: 24, or a sequence wherein 1 to 5, 1 to 4, or 1, 2, or 3 amino acids are modified in the sequence at positions 174 to 190 of SEQ ID NO: 24. In one embodiment, the sequence encoding the at least one transmembrane region comprises or consists of a sequence encoding the sequence at positions 174 to 190 of SEQ ID NO: 24.

In one embodiment, the sequence encoding the at least one transmembrane region comprises or consists of a sequence having 70%, 75%, 80%, 85%, 90%, 91%, 92%, 93%, 94%, 95%, 96%, 97%, 98%, or 99% or more sequence identity with the sequence at positions 9223-12303 of SEQ ID NO: 2. In one embodiment, the sequence encoding the at least one transmembrane region comprises or consists of the sequence at positions 9223 to 12303 of SEQ ID NO: 2, or a sequence wherein 1 to 900, 1 to 800, 1 to 700, 1 to 600, 1 to 500, 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 9223-12303 of SEQ ID NO: 2. In one embodiment, the sequence encoding the at least one transmembrane region comprises or consists of the sequence at positions 9223 to 12303 of SEQ ID NO: 2. The sequence encoding the at least one transmembrane region may have a sequence different from the sequence at positions 9223 to 12303 of SEQ ID NO: 2 due to degeneracy of the genetic code.

In one embodiment, the sequence encoding the at least one transmembrane region comprises or consists of a sequence having 70%, 75%, 80%, 85%, 90%, 91%, 92%, 93%, 94%, 95%, 96%, 97%, 98%, or 99% or more sequence identity with the sequence at positions 10804 to 12303 of SEQ ID NO: 2. In one embodiment, the sequence encoding the at least one transmembrane region comprises or consists of the sequence at positions 10804 to 12303 of SEQ ID NO: 2, or a sequence wherein 1 to 500, 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence at positions 10804 to 12303 of SEQ ID NO: 2. In one embodiment, the sequence encoding the at least one transmembrane region comprises or consists of the sequence at positions 10804 to 12303 of SEQ ID NO: 2. The sequence encoding the at least one transmembrane region may have a sequence different from the sequence at positions 10804 to 12303 of SEQ ID NO: 2 due to degeneracy of the genetic code.

In one embodiment, the sequence encoding the at least one transmembrane region comprises or consists of a sequence having 70%, 75%, 80%, 85%, 90%, 91%, 92%, 93%, 94%, 95%, 96%, 97%, 98%, or 99% or more sequence identity with the sequence at positions 730 to 792 of SEQ ID NO: 22. In one embodiment, the sequence encoding the at least one transmembrane region comprises or consists of the sequence at positions 730 to 792 of SEQ ID NO: 22, or a sequence wherein 1 to 20, 1 to 15, 1 to 10, 1 to 5, 1 to 4, or 1, 2, or 3 nucleotides are modified in the sequence at positions 730 to 792 of SEQ ID NO: 22. In one embodiment, the sequence encoding the at least one transmembrane region comprises or consists of the sequence at positions 730 to 792 of SEQ ID NO: 22. The sequence encoding the at least one transmembrane region may have a sequence different from the sequence at positions 730 to 792 of SEQ ID NO: 22 due to degeneracy of the genetic code.

In one embodiment, the sequence encoding the at least one transmembrane region comprises or consists of a sequence having 70%, 75%, 80%, 85%, 90%, 91%, 92%, 93%, 94%, 95%, 96%, 97%, 98%, or 99% or more sequence identity with the sequence at positions 520 to 570 of SEQ ID NO: 25. In one embodiment, the sequence encoding the at least one transmembrane region comprises or consists of the sequence at positions 520 to 570 of SEQ ID NO: 25, or a sequence wherein 1 to 15, 1 to 10, 1 to 5, 1 to 4, or 1, 2, or 3 nucleotides are modified in the sequence at positions520 to 570 of SEQ ID NO: 25. In one embodiment, the sequence encoding the at least one transmembrane region comprises or consists of the sequence at positions 520 to 570 of SEQ ID NO: 25. The sequence encoding the at least one transmembrane region may have a sequence different from the sequence at positions 520 to 570 of SEQ ID NO: 25 due to degeneracy of the genetic code.

The signal sequence can be a sequence derived from any protein as long as it can localize a shortened PC1 comprising the sequence to the cell membrane. Examples of signal sequences include sequences derived from PC1 as well as sequences derived from Igκ chain, CD16, CD95, CD116, CD137, or PKD2. Also, sequences known to localize proteins to the cell membrane, such as CAAX motifs, may be used (The EMBO Journal vol.10 no. 13 pp.4033-4039, 1991). In one embodiment, the signal sequence comprises at least part of the signal sequence of PC1, Igκ chain, CD16, CD95, CD116, CD137, or PKD2.

In one embodiment, the signal sequence comprises or consists of a sequence having 70%, 75%, 80%, 85%, 90%, 91%, 92%, 93%, 94%, 95%, 96%, 97%, 98%, or 99% or more sequence identity with the sequence at positions 1 to 21 of SEQ ID NO: 19. In one embodiment, the signal sequence comprises or consists of the sequence at positions 1 to 21 of SEQ ID NO: 19, or a sequence wherein 1 to 5, 1 to 4, or 1, 2, or 3 amino acids are modified in the sequence at positions 1 to 21 of SEQ ID NO: 19. In one embodiment, the signal sequence comprises or consists of the sequence at positions 1 to 21 of SEQ ID NO: 19.

In one embodiment, the signal sequence comprises or consists of a sequence having 70%, 75%, 80%, 85%, 90%, 91%, 92%, 93%, 94%, 95%, 96%, 97%, 98%, or 99% or more sequence identity with the sequence at positions 1 to 16 of SEQ ID NO: 21. In one embodiment, the signal sequence comprises or consists of the sequence at positions 1 to 16 of SEQ ID NO: 21, or a sequence wherein 1 to 5, 1 to 4, or 1, 2, or 3 amino acids are modified in the sequence at positions 1 to 16 of SEQ ID NO: 21. In one embodiment, the signal sequence comprises or consists of the sequence at positions 1 to 16 of SEQ ID NO: 21.

In one embodiment, the signal sequence comprises or consists of a sequence having 70%, 75%, 80%, 85%, 90%, 91%, 92%, 93%, 94%, 95%, 96%, 97%, 98%, or 99% or more sequence identity with the sequence at positions 1 to 25 of SEQ ID NO: 24. In one embodiment, the signal sequence comprises or consists of the sequence at positions 1 to 25 of SEQ ID NO: 24, or a sequence wherein 1 to 5, 1 to 4, or 1, 2, or 3 amino acids are modified in the sequence at positions 1 to 25 of SEQ ID NO: 24. In one embodiment, the signal sequence comprises or consists of the sequence at positions 1 to 25 of SEQ ID NO: 24.

The sequence of a polynucleotide encoding the signal sequence is not particularly limited as long as it encodes the signal sequence described herein. In one embodiment, the sequence encoding the signal sequence comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 1 to 21 of SEQ ID NO: 19. In one embodiment, the sequence encoding the signal sequence comprises or consists of a sequence encoding the sequence at positions 1 to 21 of SEQ ID NO: 19, or a sequence wherein 1 to 5, 1 to 4, or 1, 2, or 3 amino acids are modified in the sequence at positions 1 to 21 of SEQ ID NO: 19. In one embodiment, the sequence encoding the signal sequence comprises or consists of a sequence encoding the sequence at positions 1 to 21 of SEQ ID NO: 19.

In one embodiment, the sequence encoding the signal sequence comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 1 to 16 of SEQ ID NO: 21. In one embodiment, the sequence encoding the signal sequence comprises or consists of a sequence encoding the sequence at positions 1 to 16 of SEQ ID NO: 21, or a sequence wherein 1 to 5, 1 to 4, or 1, 2, or 3 amino acids are modified in the sequence at positions 1 to 16 of SEQ ID NO: 21. In one embodiment, the sequence encoding the signal sequence comprises or consists of a sequence encoding the sequence at positions 1 to 16 of SEQ ID NO: 21.

In one embodiment, the sequence encoding the signal sequence comprises or consists of a sequence encoding a sequence having 70%, 75%, 80%, 85%, 90%, 91%, 92%, 93%, 94%, 95%, 96%, 97%, 98%, or 99% or more sequence identity with the sequence at positions 1 to 25 of SEQ ID NO: 24. In one embodiment, the sequence encoding the signal sequence comprises or consists of a sequence encoding the sequence at positions 1 to 25 of SEQ ID NO: 24, or a sequence wherein 1 to 5, 1 to 4, or 1, 2, or 3 amino acids are modified in the sequence at positions 1 to 25 of SEQ ID NO: 24. In one embodiment, the sequence encoding the signal sequence comprises or consists of a sequence encoding the sequence at positions 1 to 25 of SEQ ID NO: 24.

In one embodiment, the sequence encoding the signal sequence comprises or consists of a sequence having 70%, 75%, 80%, 85%, 90%, 91%, 92%, 93%, 94%, 95%, 96%, 97%, 98%, or 99% or more sequence identity with the sequence at positions 1 to 63 of SEQ ID NO: 20. In one embodiment, the sequence encoding the signal sequence comprises or consists of the sequence at positions 1 to 63 of SEQ ID NO: 20, or a sequence wherein 1 to 15, 1 to 10, 1 to 5, 1 to 4, or 1, 2, or 3 nucleotides are modified in the sequence at positions 1 to 63 of SEQ ID NO: 20. In one embodiment, the sequence encoding the signal sequence comprises or consists of the sequence at positions 1 to 63 of SEQ ID NO: 20. The sequence encoding the signal sequence may have a sequence different from the sequence at positions 1 to 63 of SEQ ID NO: 20 due to degeneracy of the genetic code.

In one embodiment, the sequence encoding the signal sequence comprises or consists of a sequence having 70%, 75%, 80%, 85%, 90%, 91%, 92%, 93%, 94%, 95%, 96%, 97%, 98%, or 99% or more sequence identity with the sequence at positions 1 to 48 of SEQ ID NO: 22. In one embodiment, the sequence encoding the signal sequence comprises or consists of the sequence at positions 1 to 48 of SEQ ID NO: 22, or a sequence wherein 1 to 15, 1 to 10, 1 to 5, 1 to 4, or 1, 2, or 3 nucleotides are modified in the sequence at positions 1 to 48 of SEQ ID NO: 22. In one embodiment, the sequence encoding the signal sequence comprises or consists of the sequence at positions 1 to 48 of SEQ ID NO: 22. The sequence encoding the signal sequence may have a sequence different from the sequence at positions 1 to 48 of SEQ ID NO: 22 due to degeneracy of the genetic code.

In one embodiment, the sequence encoding the signal sequence comprises or consists of a sequence having 70%, 75%, 80%, 85%, 90%, 91%, 92%, 93%, 94%, 95%, 96%, 97%, 98%, or 99% or more sequence identity with the sequence at positions 1 to 75 of SEQ ID NO: 25. In one embodiment, the sequence encoding the signal sequence comprises or consists of the sequence at positions 1 to 75 of SEQ ID NO: 25, or a sequence wherein 1 to 15, 1 to 10, 1 to 5, 1 to 4, or 1, 2, or 3 nucleotides are modified in the sequence at positions 1 to 75 of SEQ ID NO: 25. In one embodiment, the sequence encoding the signal sequence comprises or consists of the sequence at positions 1 to 75 of SEQ ID NO: 25. The sequence encoding the signal sequence may have a sequence different from the sequence at positions 1 to 75 of SEQ ID NO: 25 due to degeneracy of the genetic code.

When the polypeptide of the present disclosure comprises two or more portions, for example a signal sequence, at least one transmembrane region, and a CTT region, each portion may be connected by a linker. Examples of linkers include peptide linkers comprising amino acids such as glycine, serine, arginine, alanine, and proline.

When the shortened PC1 comprises two or more portions (and optionally a linker), a polynucleotide encoding the shortened PC1 can be prepared by ligating polynucleotides encoding the portions (and optionally the linker) in-frame.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 5. In one embodiment, the shortened PC1 comprises or consists of the sequence of SEQ ID NO: 5, or a sequence wherein 1 to 500, 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 5. In one embodiment, the shortened PC1 comprises or consists of the sequence of SEQ ID NO: 5.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 5. In one embodiment, the sequence encoding the shortened PC1 comprises or consists of a sequence encoding the sequence of SEQ ID NO: 5, or a sequence wherein 1 to 500, 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 5. In one embodiment, the sequence encoding the shortened PC1 comprises or consists of a sequence encoding the sequence of SEQ ID NO: 5.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 6. In one embodiment, the sequence encoding the shortened PC1 comprises or consists of the sequence of SEQ ID NO: 6, or a sequence wherein 1 to 1200, 1 to 1100, 1 to 1000, 1 to 900, 1 to 800, 1 to 700, 1 to 600, 1 to 500, 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 6. In one embodiment, the sequence encoding the shortened PC1 comprises or consists of the sequence of SEQ ID NO: 6. The sequence encoding the shortened PC1 may have a sequence different from the sequence of SEQ ID NO: 6 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 19. In one embodiment, the shortened PC1 comprises or consists of the sequence of SEQ ID NO: 19, or a sequence wherein 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 19. In one embodiment, the shortened PC1 comprises or consists of the sequence of SEQ ID NO: 19.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 19. In one embodiment, the sequence encoding the shortened PC1 comprises or consists of a sequence encoding the sequence of SEQ ID NO: 19, or a sequence wherein 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 19. In one embodiment, the sequence encoding the shortened PC1 comprises or consists of a sequence encoding the sequence of SEQ ID NO: 19.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 20. In one embodiment, the sequence encoding the shortened PC1 comprises or consists of the sequence of SEQ ID NO: 20, or a sequence wherein 1 to 700, 1 to 600, 1 to 500, 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 20. In one embodiment, the sequence encoding the shortened PC1 comprises or consists of the sequence of SEQ ID NO: 20. The sequence encoding the shortened PC1 may have a sequence different from the sequence of SEQ ID NO: 20 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 21. In one embodiment, the shortened PC1 comprises or consists of the sequence of SEQ ID NO: 21, or a sequence wherein 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 21. In one embodiment, the shortened PC1 comprises or consists of the sequence of SEQ ID NO: 21.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 21. In one embodiment, the sequence encoding the shortened PC1 comprises or consists of a sequence encoding the sequence of SEQ ID NO: 21, or a sequence wherein 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 21. In one embodiment, the sequence encoding the shortened PC1 comprises or consists of a sequence encoding the sequence of SEQ ID NO: 21.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 22. In one embodiment, the sequence encoding the shortened PC1 comprises or consists of the sequence of SEQ ID NO: 22, or a sequence wherein 1 to 700, 1 to 600, 1 to 500, 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 22. In one embodiment, the sequence encoding the shortened PC1 comprises or consists of the sequence of SEQ ID NO: 22. The sequence encoding the shortened PC1 may have a sequence different from the sequence of SEQ ID NO: 22 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 24. In one embodiment, the shortened PC1 comprises or consists of the sequence of SEQ ID NO: 24, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 24. In one embodiment, the shortened PC1 comprises or consists of the sequence of SEQ ID NO: 24.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 24. In one embodiment, the sequence encoding the shortened PC1 comprises or consists of a sequence encoding the sequence of SEQ ID NO: 24, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 24. In one embodiment, the sequence encoding the shortened PC1 comprises or consists of a sequence encoding the sequence of SEQ ID NO: 24.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 25. In one embodiment, the sequence encoding the shortened PC1 comprises or consists of the sequence of SEQ ID NO: 25, or a sequence wherein 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 25. In one embodiment, the sequence encoding the shortened PC1 comprises or consists of the sequence of SEQ ID NO: 25. The sequence encoding the shortened PC1 may have a sequence different from the sequence of SEQ ID NO: 25 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 36. In one embodiment, the shortened PC1 comprises or consists of the sequence of SEQ ID NO: 36, or a sequence wherein 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 36. In one embodiment, the shortened PC1 comprises or consists of the sequence of SEQ ID NO: 36.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 36. In one embodiment, the sequence encoding the shortened PC1 comprises or consists of a sequence encoding the sequence of SEQ ID NO: 36, or a sequence wherein 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 36. In one embodiment, the sequence encoding the shortened PC1 comprises or consists of a sequence encoding the sequence of SEQ ID NO: 36.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 37. In one embodiment, the sequence encoding the shortened PC1 comprises or consists of the sequence of SEQ ID NO: 37, or a sequence wherein 1 to 500, 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 37. In one embodiment, the sequence encoding the shortened PC1 comprises or consists of the sequence of SEQ ID NO: 37. The sequence encoding the shortened PC1 may have a sequence different from the sequence of SEQ ID NO: 37 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 38. In one embodiment, the shortened PC1 comprises or consists of the sequence of SEQ ID NO: 38, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 38. In one embodiment, the shortened PC1 comprises or consists of the sequence of SEQ ID NO: 38.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 38. In one embodiment, the sequence encoding the shortened PC1 comprises or consists of a sequence encoding the sequence of SEQ ID NO: 38, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 38. In one embodiment, the sequence encoding the shortened PC1 comprises or consists of a sequence encoding the sequence of SEQ ID NO: 38.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 39. In one embodiment, the sequence encoding the shortened PC1 comprises or consists of the sequence of SEQ ID NO: 39, or a sequence wherein 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 39. In one embodiment, the sequence encoding the shortened PC1 comprises or consists of the sequence of SEQ ID NO: 39. The sequence encoding the shortened PC1 may have a sequence different from the sequence of SEQ ID NO: 39 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 40. In one embodiment, the shortened PC1 comprises or consists of the sequence of SEQ ID NO: 40, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 40. In one embodiment, the shortened PC1 comprises or consists of the sequence of SEQ ID NO: 40.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 40. In one embodiment, the sequence encoding the shortened PC1 comprises or consists of a sequence encoding the sequence of SEQ ID NO: 40, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 40. In one embodiment, the sequence encoding the shortened PC1 comprises or consists of a sequence encoding the sequence of SEQ ID NO: 40.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 41. In one embodiment, the sequence encoding the shortened PC1 comprises or consists of the sequence of SEQ ID NO: 41, or a sequence wherein 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 41. In one embodiment, the sequence encoding the shortened PC1 comprises or consists of the sequence of SEQ ID NO: 41. The sequence encoding the shortened PC1 may have a sequence different from the sequence of SEQ ID NO: 41 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 42. In one embodiment, the shortened PC1 comprises or consists of the sequence of SEQ ID NO: 42, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 42. In one embodiment, the shortened PC1 comprises or consists of the sequence of SEQ ID NO: 42.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 42. In one embodiment, the sequence encoding the shortened PC1 comprises or consists of a sequence encoding the sequence of SEQ ID NO: 42, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 42. In one embodiment, the sequence encoding the shortened PC1 comprises or consists of a sequence encoding the sequence of SEQ ID NO: 42.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 43. In one embodiment, the sequence encoding the shortened PC1 comprises or consists of the sequence of SEQ ID NO: 43, or a sequence wherein 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 43. In one embodiment, the sequence encoding the shortened PC1 comprises or consists of the sequence of SEQ ID NO: 43. The sequence encoding the shortened PC1 may have a sequence different from the sequence of SEQ ID NO: 43 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 44. In one embodiment, the shortened PC1 comprises or consists of the sequence of SEQ ID NO: 44, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 44. In one embodiment, the shortened PC1 comprises or consists of the sequence of SEQ ID NO: 44.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 44. In one embodiment, the sequence encoding the shortened PC1 comprises or consists of a sequence encoding the sequence of SEQ ID NO: 44, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 44. In one embodiment, the sequence encoding the shortened PC1 comprises or consists of a sequence encoding the sequence of SEQ ID NO: 44.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 45. In one embodiment, the sequence encoding the shortened PC1 comprises or consists of the sequence of SEQ ID NO: 45, or a sequence wherein 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 45. In one embodiment, the sequence encoding the shortened PC1 comprises or consists of the sequence of SEQ ID NO: 45. The sequence encoding the shortened PC1 may have a sequence different from the sequence of SEQ ID NO: 45 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 46. In one embodiment, the shortened PC1 comprises or consists of the sequence of SEQ ID NO: 46, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 46. In one embodiment, the shortened PC1 comprises or consists of the sequence of SEQ ID NO: 46.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 46. In one embodiment, the sequence encoding the shortened PC1 comprises or consists of a sequence encoding the sequence of SEQ ID NO: 46, or a sequence wherein 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 46. In one embodiment, the sequence encoding the shortened PC1 comprises or consists of a sequence encoding the sequence of SEQ ID NO: 46.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 47. In one embodiment, the sequence encoding the shortened PC1 comprises or consists of the sequence of SEQ ID NO: 47, or a sequence wherein 1 to 400, 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 47. In one embodiment, the sequence encoding the shortened PC1 comprises or consists of the sequence of SEQ ID NO: 47. The sequence encoding the shortened PC1 may have a sequence different from the sequence of SEQ ID NO: 47 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 48. In one embodiment, the shortened PC1 comprises or consists of the sequence of SEQ ID NO: 48, or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 48. In one embodiment, the shortened PC1 comprises or consists of the sequence of SEQ ID NO: 48.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 48. In one embodiment, the sequence encoding the shortened PC1 comprises or consists of a sequence encoding the sequence of SEQ ID NO: 48, or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 48. In one embodiment, the sequence encoding the shortened PC1 comprises or consists of a sequence encoding the sequence of SEQ ID NO: 48.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 49. In one embodiment, the sequence encoding the shortened PC1 comprises or consists of the sequence of SEQ ID NO: 49, or a sequence wherein 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 49. In one embodiment, the sequence encoding the shortened PC1 comprises or consists of the sequence of SEQ ID NO: 49. The sequence encoding the shortened PC1 may have a sequence different from the sequence of SEQ ID NO: 49 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 50. In one embodiment, the shortened PC1 comprises or consists of the sequence of SEQ ID NO: 50, or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 50. In one embodiment, the shortened PC1 comprises or consists of the sequence of SEQ ID NO: 50.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 50. In one embodiment, the sequence encoding the shortened PC1 comprises or consists of a sequence encoding the sequence of SEQ ID NO: 50, or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 50. In one embodiment, the sequence encoding the shortened PC1 comprises or consists of a sequence encoding the sequence of SEQ ID NO: 50.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 51. In one embodiment, the sequence encoding the shortened PC1 comprises or consists of the sequence of SEQ ID NO: 51, or a sequence wherein 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 51. In one embodiment, the sequence encoding the shortened PC1 comprises or consists of the sequence of SEQ ID NO: 51. The sequence encoding the shortened PC1 may have a sequence different from the sequence of SEQ ID NO: 51 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 52. In one embodiment, the shortened PC1 comprises or consists of the sequence of SEQ ID NO: 52, or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 52. In one embodiment, the shortened PC1 comprises or consists of the sequence of SEQ ID NO: 52.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 52. In one embodiment, the sequence encoding the shortened PC1 comprises or consists of a sequence encoding the sequence of SEQ ID NO: 52, or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 52. In one embodiment, the sequence encoding the shortened PC1 comprises or consists of a sequence encoding the sequence of SEQ ID NO: 52.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 53. In one embodiment, the sequence encoding the shortened PC1 comprises or consists of the sequence of SEQ ID NO: 53, or a sequence wherein 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 53. In one embodiment, the sequence encoding the shortened PC1 comprises or consists of the sequence of SEQ ID NO: 53. The sequence encoding the shortened PC1 may have a sequence different from the sequence of SEQ ID NO: 53 due to degeneracy of the genetic code.

In one embodiment, the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 54. In one embodiment, the shortened PC1 comprises or consists of the sequence of SEQ ID NO: 54, or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 54. In one embodiment, the shortened PC1 comprises or consists of the sequence of SEQ ID NO: 54.

In one embodiment, the sequence encoding the shortened PC1 comprises or consists of a sequence encoding a sequence having 70%, 75%, 80%, 85%, 90%, 91%, 92%, 93%, 94%, 95%, 96%, 97%, 98%, or 99% or more sequence identity with the sequence of SEQ ID NO: 54. In one embodiment, the sequence encoding the shortened PC1 comprises or consists of a sequence encoding the sequence of SEQ ID NO: 54, or a sequence wherein 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 amino acids are modified in the sequence of SEQ ID NO: 54. In one embodiment, the sequence encoding the shortened PC1 comprises or consists of a sequence encoding the sequence of SEQ ID NO: 54.

In one embodiment, the sequence encoding the shortened PC1 comprises or consists of a sequence having 70%, 75%, 80%, 85%, 90%, 91%, 92%, 93%, 94%, 95%, 96%, 97%, 98%, or 99% or more sequence identity with the sequence of SEQ ID NO: 55. In one embodiment, the sequence encoding the shortened PC1 comprises or consists of the sequence of SEQ ID NO: 55, or a sequence wherein 1 to 300, 1 to 200, 1 to 190, 1 to 180, 1 to 170, 1 to 160, 1 to 150, 1 to 140, 1 to 130, 1 to 120, 1 to 110, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 25, 1 to 20, 1 to 15, 1 to 10, 1 to 5, 1 to 4, or 1, 2, or 3 nucleotides are modified in the sequence of SEQ ID NO: 55. In one embodiment, the sequence encoding the shortened PC1 comprises or consists of the sequence of SEQ ID NO: 55. The sequence encoding the shortened PC1 may have a sequence different from the sequence of SEQ ID NO: 55 due to degeneracy of the genetic code.

As used herein, at least part of a region or sequence can be any portion of the region or sequence, and can be the whole of the region or sequence.

As used herein, a sequence "comprising" a given sequence encompasses a sequence composed of the given sequence and one or more amino acids (in the case of an amino acid sequence) or nucleotides (in the case of a nucleotide sequence) added to the given sequence, and a sequence consisting of the given sequence.

The sequence identity with respect to amino acid or nucleotide sequences refers to the percentage of identical amino acid residues or bases between two sequences that are optimally aligned (aligned to be maximally matched). The sequence identity is calculated by the following formula: sequence identity (%) = [(amino acid residues or bases matched between two sequences)/(alignment length)] × 100. The sequence identity can be calculated using programs such as FASTA and BLAST.

Amino acid or nucleotide modification includes deletion, substitution, insertion and addition of an amino acid(s) or a nucleotide(s). The modification may be deletion, substitution, insertion, addition, or a combination of two or more of them.

The cyst formation inhibitory activity can be confirmed by suitable methods by those skilled in the art. For example, models of cyst formation as described in the Examples or in the literature (Yan B. et al., J Am Soc Nephrol 19: 1300-1310, 2008) can be used. When MDCK (Madin-Darby canine kidney) cells, which are derived from canine kidney renal tubular epithelial cells, are three-dimensionally cultured on collagen gel and stimulated with forskolin, cyst formation is observed. Addition of EGF with forskolin can shorten the time to cyst formation (Molecular Cell 6:1267-1273 2000). If cyst formation is inhibited in MDCK cells expressing a shortened PC1 that are prepared by introducing a polynucleotide encoding the shortened PC1 or a vector comprising the polynucleotide, the shortened PC1 can be determined to have the cyst formation inhibitory activity. The shortened PC1 of the present disclosure can, for example, inhibit cyst formation by at least 20%, 30%, 40%, 50%, 60%, 70%, or 80% compared to control.

It is possible to express a shortened PC1 in a subject by administering a polynucleotide comprising a sequence encoding the shortened PC1 or a vector comprising the polynucleotide to the subject. The polynucleotide may be DNA or RNA, and may comprise one or more non-naturally occurring nucleotides.

A vector is anything that can deliver a polynucleotide of a desired sequence into a cell. The vector can be a viral vector or a non-viral vector. Examples of viral vectors include retrovirus, lentivirus, adenovirus, adeno-associated virus (AAV), Herpes Simplex Virus (HSV), vaccinia virus, Sendai virus, and measles virus vectors. Non-viral vectors include plasmid vectors, liposome vectors, and artificial chromosomes (e.g., YAC, BAC, PAC). Each vector can be prepared according to conventional methods.

The vector may comprise one or more regulatory sequences that regulate expression of a sequence encoding a shortened PC1. Examples of regulatory sequences include promoter, enhancer, polyadenylation signal, and other expression control elements such as post-transcriptional regulatory element (PRE). The promoter can be a constitutive promoter, inducible promoter, or tissue- or cell-specific promoter. Examples of promoters include CMV (Cytomegalovirus) promoter, PGK (Phosphoglycerate kinase) promoter, EF1α (Elongation factor 1-alpha) promoter, CAG promoter, SV40 promoter and MSCV promoter. Examples of polyadenylation signals include SV40 polyadenylation signal, bovine growth hormone gene polyadenylation signal, and globulin polyadenylation signal. Examples of PREs include WPRE, Adenovirus tripartite leader sequence, Cytomegalovirus Intron A, and HSP70 5'UTR (untranslated region).

The vector may comprise an origin of replication (ori) for its replication. It may also comprise a marker for selection of cells comprising the vector. Examples of selection markers include genes that confer resistance to an agent such as neomycin, kanamycin, puromycin, hygromycin, zeocin, or ampicillin.

In one embodiment, the vector is an AAV vector. There are numerous serotypes of AAV, including AAV1, AAV2, AAV3, AAV4, AAV5, AAV6, AAV7, AAV8, AAV9, AAV10, AAV11, AAV12, AAV-rh10, AAV-DJ, and AAVAnc80. The AAV vector may be a vector in a native form, or in a recombinant form, for example with modification of capsid protein. The AAV vector can be prepared, for example, by introducing an AAV vector plasmid comprising a sequence encoding a shortened PC1 (or a sequence encoding a shortened PC1 downstream of a promoter) and inverted terminal repeats (ITRs) at both ends of the sequence into packaging cells together with an AAV helper plasmid comprising Rep gene and Cap gene, which are required for AAV replication and particle formation, and an adenovirus helper plasmid comprising an adenovirus helper gene, which is required for AAV propagation. Examples of packaging cells include 293T cells, 293 cells, HeLa cells, COS1 cells, and COS7 cells. Virus particles released from packaging cells can be recovered from culture supernatant of the packaging cells by purification methods such as centrifugation, filter filtration, and column purification.

In one embodiment, the vector is a lentivirus vector. Examples of lentivirus vectors include HIV (human immunodeficiency virus) (for example, HIV-1 and HIV-2), SIV (simian immunodeficiency virus), FIV (feline immunodeficiency virus), MVV (Maedi-Visna virus), EV1 (Maedi-Visna-like virus), EIAV (equine infectious anemia virus), and CAEV (caprine arthritis encephalitis virus). A lentivirus vector can be prepared, for example, by introducing a viral vector plasmid comprising a sequence encoding a shortened PC1 (or a sequence encoding a shortened PC1 downstream of a promoter) and long terminal repeats (LTRs) (5' LTR and 3' LTR) at both ends of the sequence and a packaging signal, into packaging cells together with one or more plasmid vectors for expressing viral structural proteins such as Gag, Pol, and Env, or into packaging cells expressing these structural proteins. The viral vector may be pseudotyped. For example, one or more plasmid vectors for expressing envelope proteins such as vesicular stomatitis virus G protein (VSV-G) may be further introduced into the packaging cells. Examples of packaging cells include 293T cells, 293 cells, HeLa cells, COS1 cells, and COS7 cells. Virus particles released from packaging cells can be recovered from culture supernatant of the packaging cells by purification methods such as centrifugation, filter filtration, and column purification.

In one embodiment, the polynucleotide comprising a sequence encoding a shortened PC1 is RNA. In this embodiment, the polynucleotide may comprise a 5'cap structure, 5' untranslated region (UTR), a sequence encoding a shortened PC1, 3' UTR, and a poly A sequence. The polynucleotide can be prepared, for example, by *in vitro* transcription using a plasmid DNA as a template. For intracellular delivery, the polynucleotide may be administered in lipid or polymer particles.

A PKD1-related disease can be treated by expressing a shortened PC1 of the present disclosure in a subject, or by administering a shortened PC1 of the present disclosure to a subject. APKD1-related disease means a disease caused by loss of function of PC1 due to mutation of the PKD1 gene. Examples of PKD1-related diseases include cystic disease, hypertension, intracranial aneurysm, colonic diverticula, heart valve regurgitation, and kidney stones. In one embodiment, the PKD1-related disease is a cystic disease. Examples of cystic diseases include polycystic kidney disease (PKD), polycystic liver disease (PLD), nephronophthisis (NPHP), Bardet-Biedl syndrome (BBS), Meckel syndrome (MKS), Joubert syndrome (JBTS), Oral facial digital syndrome, OFD, or pancreatic cysts. In one embodiment, the cystic disease is PKD. PKD includes autosomal dominant polycystic kidney disease (ADPKD) and autosomal recessive polycystic kidney disease (ARPKD).

In one embodiment, the PKD is ADPKD. ADPKD is a hereditary disease whose causal genes are PKD1 and PKD2 genes. It is said that 85% of patients develops the disease due to abnormality of the PKD1 gene. PC1 and PC2 regulate Ca²⁺ influx into cells. It is suggested that the PKD1 or PKD2 gene mutation results in loss of function of PC1 or PC2 and causes cyst formation.

The treatment of a PKD1-related disease includes improvement or suppression of one or more symptoms or findings of the disease. For example, the treatment of ADPKD includes suppression of kidney volume increase or suppression of renal function decline in ADPKD.

The polynucleotide, polypeptide, or vector is administered to a subject in an amount capable of providing a desired effect (for example, treatment of a PKD1-related disease) (as used herein, an effective amount). The dosage is appropriately selected depending on factors such as age, body weight, and health condition of the subject who receives the administration. An AAV vector may be administered, for example, at about 10⁹ to 10¹⁵ vg (vector genome), 10¹⁰ to 10¹⁴ vg, or 10¹⁰ to 10¹³ vg per kg body weight. A lentivirus vector may be administered, for example, at about 10⁴ to 10¹⁰ TU (transducing units) per kg of body weight. A non-viral vector may be administered, for example, at about 0.01 to 100 mg/kg body weight. The administration may be daily, once a day or several days, once a week or several weeks, once a month or several months, or once every few years or in a lifetime. The daily dose may be administered in a single dose or in multiple (e.g., 2 to 4) doses.

Administration can be done with methods appropriately selected depending on factors such as age, body weight, and health condition of the subject who receives the administration. The administration may be oral administration or parenteral administration, and preferably parenteral administration. The parenteral administration includes subcutaneous administration, intradermal administration, intramuscular administration, intraperitoneal administration, intravenous administration, femoral artery-renal artery catheter administration, and retrograde ureteral administration.

A pharmaceutical composition may comprise, in addition to the polynucleotide, polypeptide, or vector, a pharmaceutically acceptable carrier or additive such as sterile water, saline, stabilizer, excipient, antioxidant, buffer, preservative, surfactant, chelating agent, and binding agent. The dosage form may be, but not limited to, an injection. The injection include a solution injection, suspension injection, emulsion injection, and ready-to-use injection (e.g., freeze-dried injection). The pharmaceutical composition may be provided as a kit, and the kit may further comprise an additional element such as a buffer solution to be used for preparation at the time of use or an instruction for use.

The subject may be a mammal, such as a human or non-human mammal. Examples of non-human mammals include mouse, rat, rabbit, cat, dog, sheep, hog, horse, cattle, and monkey. In one embodiment, the subject is human.

Illustrative embodiments of the disclosure are described below.
[1] A polynucleotide comprising a sequence encoding a shortened polycystin-1 (PC1) having cyst formation inhibitory activity, wherein the shortened PC1 comprises a C-terminal tail (CTT) region of PC1 or a part thereof.
[2] The polynucleotide according to item 1, wherein the shortened PC1 comprises the CTT region of PC1.
[3] The polynucleotide according to item 1, wherein the shortened PC1 comprises the part of a CTT region of PC1.
[4] The polynucleotide according to any one of items 1 to 3, wherein the shortened PC1 lacks at least part of an extracellular N-terminal region of PC1.
[5] The polynucleotide according to any one of items 1 to 4, wherein the shortened PC1 lacks the whole of the extracellular N-terminal region of PC1.
[6] The polynucleotide according to any one of items 1 to 5, wherein the shortened PC1 is a membrane-localized polypeptide.
[7] A polynucleotide comprising a sequence encoding a shortened polycystin-1 (PC1), wherein the shortened PC1 is a membrane-localized polypeptide comprising a C-terminal tail (CTT) region of PC1 or a part thereof.
[8] The polynucleotide according to item 7, wherein the shortened PC1 comprises the CTT region of PC1.
[9] The polynucleotide according to item 7, wherein the shortened PC1 comprises the part of a CTT region of PC1.
[10] The polynucleotide according to any one of items 1 to 9, wherein the shortened PC1 further comprises at least part of a stalk of PC1.
[11] The polynucleotide according to item 10, wherein the shortened PC1 further comprises at least one transmembrane region.
[12] The polynucleotide according to any one of items 1 to 9, wherein the shortened PC1 further comprises a signal sequence.
[13] The polynucleotide according to item 12, wherein the shortened PC1 further comprises at least one transmembrane region.
[14] The polynucleotide according to any one of items 1 to 13, wherein the CTT region consists of a sequence having 90% or more sequence identity with the sequence of SEQ ID NO: 3.
[15] The polynucleotide according to any one of items 1 to 14, wherein the CTT region consists of the sequence of SEQ ID NO: 3.
[16] The polynucleotide according to any one of items 1 to 14, wherein the CTT region consists of the sequence at positions 3 to 202 of SEQ ID NO: 3.
[17] The polynucleotide according to any one of items 1 to 16, wherein the part of a CTT region comprises at least part of a sequence having 90% or more sequence identity with the sequence at positions 4154 to 4303 of SEQ ID NO: 1.
[18] The polynucleotide according to any one of items 1 to 17, wherein the part of a CTT region comprises at least part of the sequence at positions 4154 to 43033 of SEQ ID NO: 1.
[19] The polynucleotide according to any one of items 1 to 18, wherein the part of a CTT region comprises a sequence having 90% or more sequence identity with the sequence at positions 4204 to 4243 of SEQ ID NO: 1.
[20] The polynucleotide according to any one of items 1 to 19, wherein the part of a CTT region comprises the sequence at positions 4204 to 4243 of SEQ ID NO: 1.
[21] The polynucleotide according to any one of items 1 to 20, wherein the part of a CTT region comprises a sequence having 90% or more sequence identity with the sequence at positions 4204 to 4253 of SEQ ID NO: 1.
[22] The polynucleotide according to any one of items 1 to 21, wherein the part of a CTT region comprises the sequence at positions 4204 to 4253 of SEQ ID NO: 1.
[23] The polynucleotide according to any one of items 1 to 22, wherein the length of the part of a CTT region is 150 amino acids or less.
[24] The polynucleotide according to any one of items 11 and 13 to 23, wherein the at least one transmembrane region is selected from transmembrane regions of PC1, T cell receptor α or β chain, CD3ε, CD4, CD5, CD7, CD8, CD9, CD16, CD22, CD27, CD28, CD33, CD37, CD45, CD64, CD80, CD86, CD95, CD134, CD137, CD154, PKD2, VSV-G, and PLAP.
[25] The polynucleotide according to any one of items 11 and 13 to 24, wherein the at least one transmembrane region consists of a sequence having 90% or more sequence identity with the sequence at positions 3075 to 4101 of SEQ ID NO: 1.
[26] The polynucleotide according to item 25, wherein at least one transmembrane region consists of the sequence at positions 3075 to 4101 of SEQ ID NO: 1.
[27] The polynucleotide according to any one of items 11 and 13 to 24, wherein the at least one transmembrane region consists of a sequence having 90% or more sequence identity with the sequence at positions 3602 to 4101 of SEQ ID NO: 1.
[28] The polynucleotide according to item 27, wherein the at least one transmembrane region consists of the sequence at positions 3602 to 4101 of SEQ ID NO: 1.
[29] The polynucleotide according to any one of items 11 and 13 to 24, wherein the at least one transmembrane region consists of a sequence having 90% or more sequence identity with the sequence at positions 244 to 264 of SEQ ID NO: 22.
[30] The polynucleotide according to item 29, wherein the at least one transmembrane region consists of the sequence at positions 244 to 264 of SEQ ID NO: 22.
[31] The polynucleotide according to any one of items 11 and 13 to 24, wherein the at least one transmembrane region consists of a sequence having 90% or more sequence identity with the sequence at positions 174 to 190 of SEQ ID NO: 24.
[32] The polynucleotide according to item 31, wherein the at least one transmembrane region consists of the sequence at positions 174 to 190 of SEQ ID NO: 24.
[33] The polynucleotide according to any one of items 12 to 32, wherein the signal sequence comprises at least part of a signal sequence of PC1, Igκ chain, CD16, CD95, CD116, CD137, or PKD2.
[34] The polynucleotide according to any one of items 12 to 33, wherein the signal sequence consists of a sequence having 90% or more sequence identity with the sequence at positions 1 to 21 of SEQ ID NO: 19.
[35] The polynucleotide according to item 34, wherein the signal sequence consists of the sequence at positions 1 to 21 of SEQ ID NO: 19.
[36] The polynucleotide according to any one of items 12 to 33, wherein the signal sequence consists of a sequence having 90% or more sequence identity with the sequence at positions 1 to 16 of SEQ ID NO: 21.
[37] The polynucleotide according to item 36, wherein the signal sequence consists of the sequence at positions 1 to 16 of SEQ ID NO: 21.
[38] The polynucleotide according to any one of items 12 to 33, wherein the signal sequence consists of a sequence having 90% or more sequence identity with the sequence at positions 1 to 25 of SEQ ID NO: 24.
[39] The polynucleotide according to item 38, wherein the signal sequence consists of the sequence at positions 1 to 25 of SEQ ID NO: 24.
[40] The polynucleotide according to any one of items 1 to 39, wherein the shortened PC1 comprises a sequence having 90% or more sequence identity with the sequence of SEQ ID NO: 5, 19, or 21.
[41] The polynucleotide according to item 40, wherein the shortened PC1 comprises the sequence of SEQ ID NO: 5, 19, or 21.
[42] The polynucleotide according to any one of items 1 to 41, comprising a sequence having 90% or more sequence identity with the sequence of SEQ ID NO: 6, 20, or 22.
[43] The polynucleotide according to item 42, comprising the sequence of SEQ ID NO: 6, 20, or 22.
[44] The polynucleotide according to any one of items 1 to 39, wherein the shortened PC1 comprises a sequence having 90% or more sequence identity with the sequence of SEQ ID NO: 24, 36, 38, 40, 42, 44, 46, 48, 50, 52, or 54.
[45] The polynucleotide according to item 44, wherein the shortened PC1 comprises the sequence of SEQ ID NO: 24, 36, 38, 40, 42, 44, 46, 48, 50, 52, or 54.
[46] The polynucleotide according to any one of items 1 to 39, 44, and 45, comprising a sequence having 90% or more sequence identity with the sequence of SEQ ID NO: 25, 37, 39, 41, 43, 45, 47, 49, 51, 53, or 55.
[47] The polynucleotide according to item 46, comprising the sequence of SEQ ID NO: 25, 37, 39, 41, 43, 45, 47, 49, 51, 53, or 55.
[48] A shortened polycystin-1 (PC1) polypeptide encoded by the polynucleotide according to any one of items 1 to 47.
[49] A vector comprising the polynucleotide according to any one of items 1 to 47.
[50] The vector according to item 49, wherein the vector is a viral vector or a non-viral vector.
[51] The vector according to item 50, wherein the viral vector is a retrovirus, lentivirus, adenovirus, adeno-associated virus (AAV), Herpes Simplex Virus (HSV), vaccinia virus, Sendai virus, or measles virus vector.
[52] The vector according to item 50, wherein the non-viral vector is a plasmid vector, a liposome vector, or an artificial chromosome.
[53] A pharmaceutical composition for treating a PKD1-related disease, comprising the polynucleotide according to any one of items 1 to 47, the polypeptide according to item 48, or the vector according to any one of items 49 to 52.
[54] The pharmaceutical composition according to item 53, wherein the PKD1-related disease is a cystic disease.
[55] The pharmaceutical composition according to item 54, wherein the cystic disease is polycystic kidney disease (PKD), polycystic liver disease (PLD), nephronophthisis (NPHP), Bardet-Biedl syndrome (BBS), Meckel syndrome (MKS), Joubert syndrome (JBTS), oral-facial-digital syndrome (OFD) or pancreatic cysts.
[56] The pharmaceutical composition according to item 55, wherein the PKD is autosomal dominant polycystic kidney disease (ADPKD) or autosomal recessive polycystic kidney disease (ARPKD).
[57] The pharmaceutical composition according to item 56, wherein the PKD is ADPKD.
[58] The polynucleotide according to any one of items 1 to 47, the polypeptide according to item 48, or the vector according to any one of items 49 to 52 for use in the treatment of a PKD1-related disease.
[59] A method of treating a PKD1-related disease, comprising administering to a subject in need thereof an effective amount of the polynucleotide according to any one of items 1 to 47, the polypeptide according to item 48, or the vector according to any one of items 49 to 52.
[60] Use of the polynucleotide according to any one of items 1 to 47, the polypeptide according to item 48, or the vector according to any one of items 49 to 52 for the manufacture of a medicament for treating a PKD1-related disease.
[61] A pharmaceutical composition for inhibiting cyst formation in kidney, comprising a polynucleotide encoding a shortened polycystin-1 (PC1) designed to localize a C-terminal tail (CTT) region of PC1 or a part thereof to cell membrane, or a vector comprising the polynucleotide.
[62] A pharmaceutical composition for inhibiting cyst formation in kidney, comprising a polynucleotide encoding a shortened polycystin-1 (PC1) comprising a C-terminal tail (CTT) region of PC1 or a part thereof, or a vector comprising the polynucleotide, wherein the pharmaceutical composition inhibits the cyst formation by localizing the CTT region or the part thereof to cell membrane.
[63] A pharmaceutical composition for inhibiting cyst formation in kidney, comprising a shortened polycystin-1 (PC1) designed to localize a C-terminal tail (CTT) region of PC1 or a part thereof to cell membrane.
[64] A pharmaceutical composition for inhibiting cyst formation in kidney, comprising a shortened polycystin-1 (PC1) comprising a C-terminal tail (CTT) region of PC1 or a part thereof, wherein the pharmaceutical composition inhibits the cyst formation by localizing the CTT region or the part thereof to cell membrane.

The present invention will be further described with reference to the Examples below, but the present invention is not limited to these examples in any sense.

### Examples

### Example 1

### 1. Test methods

### 1.1 Cells

MDCK (Madin-Darby canine kidney) cells, which were derived from canine kidney renal tubular epithelial cells, were used.

### 1.2 Construction of expression vectors

The human PKD1 gene product polycystin-1 (PC1) and its partial regions shown in Table 1 were cloned from a PKD1 expression plasmid (Origene Technologies) and inserted into the multiple cloning site of an episomal vector pEBMulti-Hygb vector (FUJIFILM Wako Pure Chemical Co., Ltd). For the purpose of promoting cell membrane translocation, an Igκ chain leader sequence (pDisplay^{™} Vector (Invitrogen)) or CD16.7 (a fusion protein containing human CD16 extracellular region and human CD7 transmembrane region (Tsiokas 94: 6965-6970 1997 PNAS)) was added at the N-terminus of some of the partial regions. PC1 (Arg1672fs97X) is a mutation found in patients with autosomal dominant polycystic kidney disease. R-CTF, in which a sequence encoding CTF was inserted in the vector in the reverse orientation, was tested as a negative control that did not express CTF.

**Table 1**

| Name | | SEQ ID NO: |
|---|---|---|
| PC1 | human PKD1gene product PC 1 | 1, 2 |
| CTF | C-terminal fragment (CTF) region of PC1 | 5, 6 |
| CTT | C-terminal tail (CTT) region of PC1 | 7, 8 |
| PC1 (Arg1672fs97X) | Deletion of AG at positions 5014-5015 of PKD1 gene | 9, 10 |
| stalk-TM (11) | Stalk sequence and 11-transmembrane region (TM1-TM11) of PC1 | 11, 12 |
| TM (11) | 11-transmembrane region | 13, 14 |
| TM (11)-CTT | 11-transmembrane region and CTT region | 15, 16 |
| TLD (TM (6)-CTT) | 6-transmembrane regions (TM6-TM11) and CTT region | 17, 18 |
| Igκ-TLD | Igκ chain leader sequence + TLD | 19, 20 |
| CD16.7-CTT | Fusion protein of CD16 extracellular region, CD7 transmembrane region, and CTT region | 21, 22 |
| R-CTF | Reverse CTF | 23 |

### 1.3 Transfection of expression vectors into cells and selection of transfected cells

The plasmid vector thus prepared was purified from Escherichia coli and transfected into MDCK cells using Neon Transfection System (ThermoFisher Scientific). To 10 µL of cell suspension of 1×10⁷ cells/mL, 1 µg of the expression vector was suspended and the resulting suspension was put into the Neon Transfection System. Transformation was performed by electroporation under the conditions of voltage: 1150 V, width: 20ms, and number: 1. Then, the cells were added to a medium (DMEM/F12 containing 10% FCS) (1 mL/well, 12 well plate). On the next day, the medium was replaced with the same medium supplemented with 500 µg/mL hygromycin (Life Technologies Japan), and the cells were cultured for 6 to 8 days to select cells to which the expression vector was introduced.

### 1.4 Preparation of collagen gel solution and culture

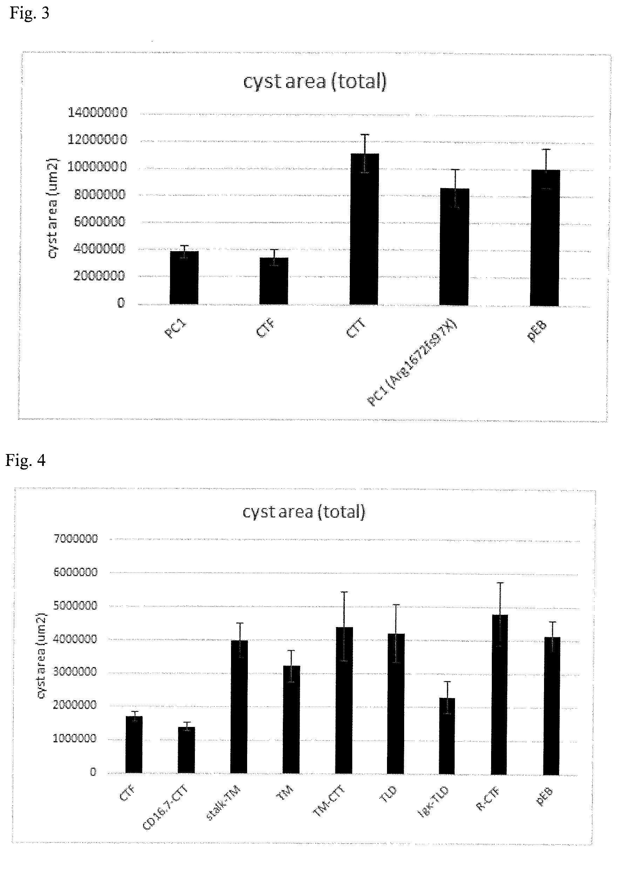

To obtain a collagen gel solution (100 mL), 74.09 mL of 3 mg/mL PureCol (Advanced Cell Matrix Co) (2.25 mg/mL), 10 mL of 10×MEM, 10 mL of inactivated fetal bovine serum (FBS) (10%), 2 mL of 1 M HEPES (20 mM), 2.91 mL of 0.75% NaHCO₃ (26 mM) and 1 mL of sterilized water were mixed and adjusted to pH 7.4 with 1 N NaOH. The collagen gel solution was added at 250 µL per well to a 24-well plate (Corning 353047) and solidified in a CO₂ incubator at 37°C. On the next day, the MDCK cells were mixed in 500 µL of the collagen gel solution and 5,000 cells/well were layered, and the plate was allowed to stand in a CO₂ incubator at 37°C for 4 hours or more so that the collagen gel solution was solidified. Once the solidification was confirmed, forskolin (FUJIFILM Wako Pure Chemical Co., Ltd) and EGF (FUJIFILM Wako Pure Chemical Co., Ltd) was added to 750 µL of 10% FBS-containing DMEM/F12 medium at the final concentrations of 20 mM and 20 µg/mL, respectively (for the test of Fig. 3, hygromycin was added to this medium at the final concentration of 400 µg/mL), and the cells were cultured with the medium in a CO₂ incubator at 37°C. The medium (containing forskolin and EGF, and additionally containing hygromycin in the test of Fig. 3) was changed every 3 to 4 days, and the culture was continued for 8 to 12 days.

### 1.5 Automatic measurement of cyst size and cyst number using Cell Voyager CV6000 or CV8000

From the 24-well plate containing cysts, bright-field images (corresponding to 32 of 4x objective lens areas for each well) were obtained with a Cell Voyager CV6000 or CV8000 (Yokogawa) (15 images at 50 µm intervals). A single image file (MinIP) was obtained by superimposing 15 images at minimum intensity. The cyst area and cyst number were quantified using the analysis support software attached to the CV6000, and the total cyst area was calculated.

### 2. Results

The cyst formation inhibitory effects of PC1, CTF, CTT, and PC1 (Arg1672fs97X) were evaluated using an empty pEBMulti-Hygb vector as a control. The cyst formation was inhibited by PC1 and CTF, but not by CTT and PC1 (Arg1672fs97X) (Fig. 3).

To examine the region required for inhibition of cyst formation, the cyst formation inhibitory effects of CTF, stalk-TM(11), TM(11), TM(11)-CTT, TLD, Igκ-TLD, CD16.7-CTT, and R-CTF were evaluated using an empty pEBMulti-Hygb vector as a control. Among the tested constructs, CTF, Igκ-TLD, and CD16.7-CTT had the cyst formation inhibitory effect (Fig. 4). A comparison of CTF with stalk-TM(11) indicated that the CTT region was required for inhibition of cyst formation. Also, from a comparison of CTF and TM(11)-CTT, and that of Igκ-TLD and CD16.7-CTT, it was suggested that at least part of the stalk or the signal sequence that localized the CTT region to the cell membrane was required for inhibition of cyst formation.

### Example 2

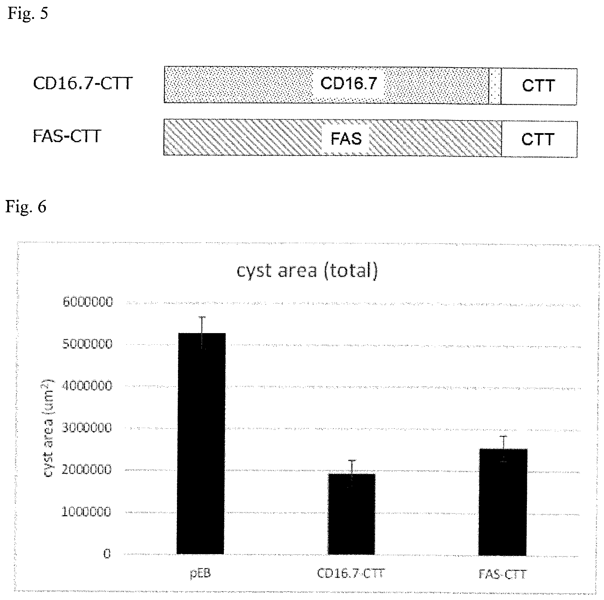

In order to examine whether membrane-translocating shortened PC1 polypeptides other than CD16.7-CTT and Igκ-TLD exhibit the cyst formation inhibitory effect, FAS, which was known to induce cell membrane localization (J. Exp. Med. Vol. 217 No. 12 e20291166 2020), was used to prepare an expression vector for a fusion protein of the FAS and the CTT region (FAS-CTT) (Table 3) in the same manner as in Example 1 (Fig. 5), and its the cyst formation inhibitory effect was evaluated. FAS-CTT showed the cyst formation inhibitory effect as CD16.7-CTT (Fig. 6).

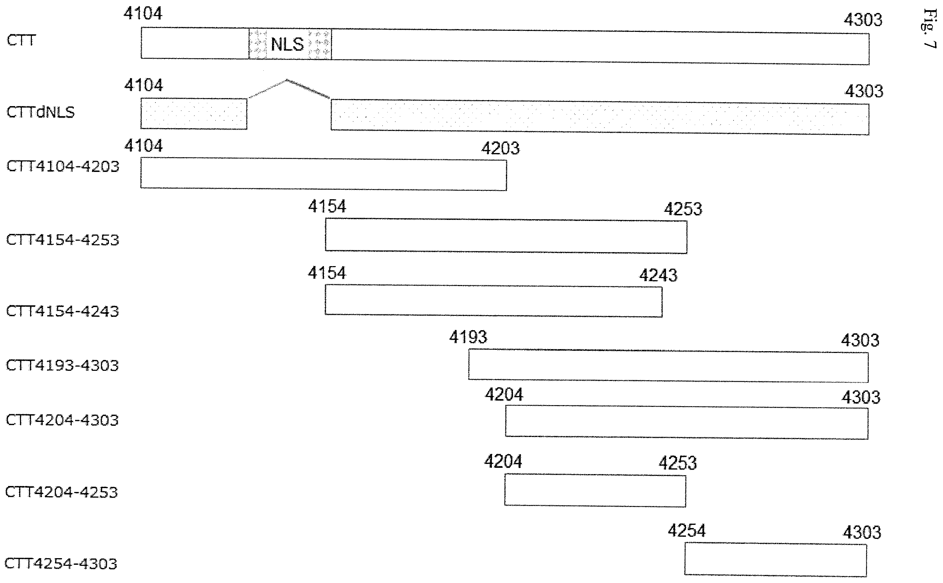

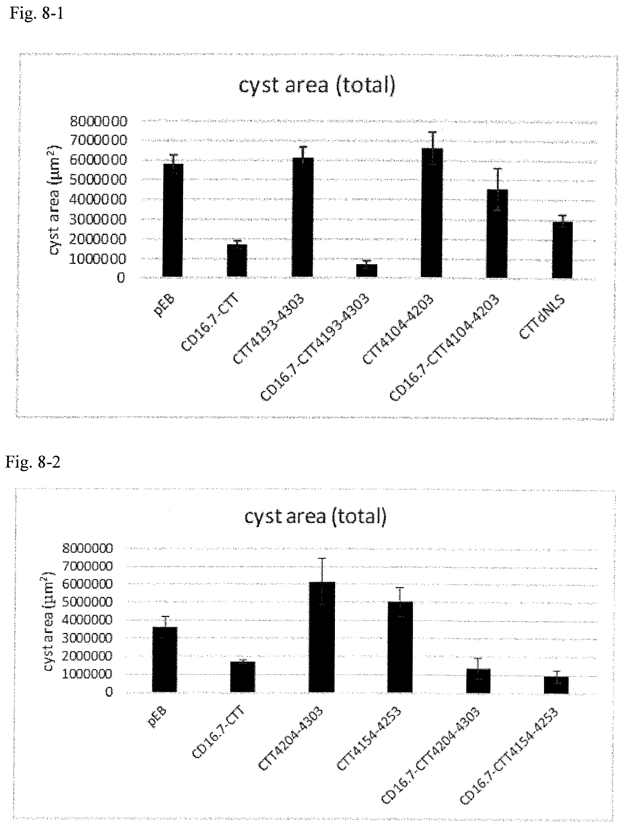

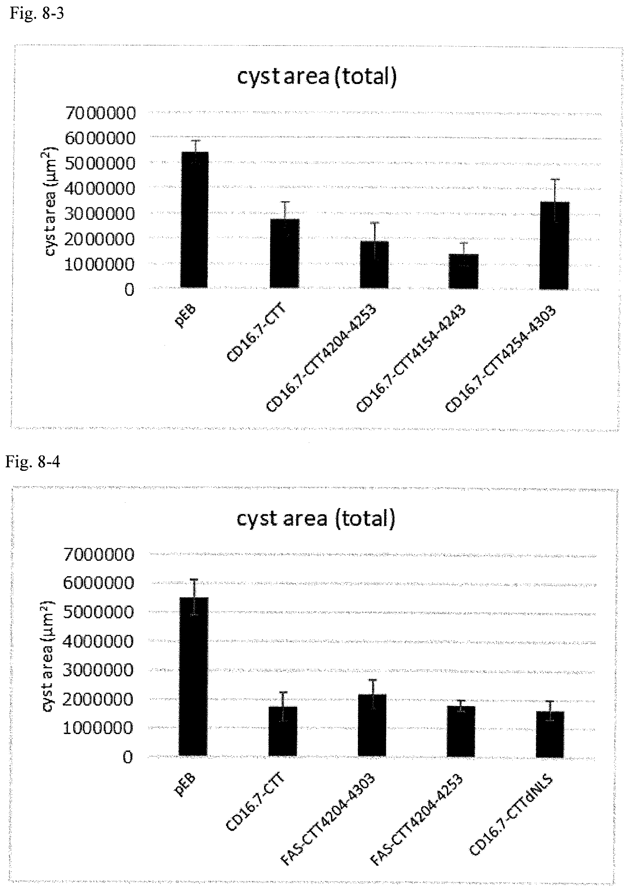

Since the inhibition of cyst formation was thought to require membrane localization of the CTT region, a CTT core region which was important for the inhibition of cyst formation was searched. In the same manner as in Example 1, an expression vector for a partial region of the CTT region shown in Fig. 7 (each consisting of a part of the amino acid sequence at positions 4104-4303 of SEQ ID NO: 1) or a fusion protein of the partial region of the CTT region and CD16.7 or FAS (Table 3) was prepared, and its cyst formation inhibitory effect was evaluated. The results are shown in Figs. 8-1 to 8-4.

**Table 3-1**

| Name | | SEQ ID NO. |
|---|---|---|
| FAS-CTT | fusion protein of FAS extracellular domain, transmembrane region, and CTT region | 24, 25 |
| CTTdNLS | CTT lacking nuclear localization signal (NLS) | 26, 27 |
| CTT4104-4203 | 100 amino acids within CTT region at positions 4104-4203 | 28, 29 |
| CTT4154-4253 | 100 amino acids within CTT region at positions 4154-4253 | 30, 31 |
| CTT4193-4303 | 111 amino acids within CTT region at positions 4193-4303 | 32, 33 |
| CTT4204-4303 | 100 amino acids within CTT region at positions 4204-4303 | 34, 35 |
| CD16.7-CTTdNLS | fusion protein of CD16 extracellular domain, CD7 transmembrane region, and CTTdNLS | 36, 37 |
| CD16.7-CTT4104-4203 | fusion protein of CD16 extracellular domain, CD7 transmembrane region, and 100 amino acids within CTT region at positions 4104-4203 | 38, 39 |
| CD16.7-CTT4154-4253 | fusion protein of CD16 extracellular domain, CD7 transmembrane region, and 100 amino acids within CTT region at positions 4154-4253 | 40, 41 |

**Table 3-2**

| Name | | SEQ ID NO. |
|---|---|---|
| CD 16.7-CTT4154-4243 | fusion protein of CD16 extracellular domain, CD7 transmembrane region, and 90 amino acids within CTT region at positions 4154-4243 | 42, 43 |
| CD16.7-CTT4193-4303 | fusion protein of CD16 extracellular domain, CD7 transmembrane region, and 111 amino acids within CTT region at positions 4193-4303 | 44, 45 |
| CD16.7-CTT4204-4303 | fusion protein of CD16 extracellular domain, CD7 transmembrane region, and 100 amino acids within CTT region at positions 4204-4303 | 46, 47 |
| CD16.7-CTT4204-4253 | fusion protein of CD16 extracellular domain, CD7 transmembrane region, and 50 amino acids within CTT region at positions 4204-4253 | 48, 49 |
| CD16.7-CTT4254-4303 | fusion protein of CD16 extracellular domain, CD7 transmembrane region, and 50 amino acids within CTT region at positions 4254-4303 | 50, 51 |
| FAS-CTT4204-4303 | fusion protein of FAS extracellular domain, transmembrane region, and 100 amino acids within CTT region at positions 4204-4303 | 52, 53 |
| FAS-CTT4204-4253 | fusion protein of FAS extracellular domain, transmembrane region, and 50 amino acids within CTT region at positions 4204-4253 | 54, 55 |

No cyst formation inhibitory effect was observed with only a partial region of the CTT region, except for CTT-dNLS. In contrast, a fusion protein of a partial region of the CTT region and CD16.7 or FAS showed the cyst formation inhibitory effect. Among these, CD16.7-CTT4104-4203 and CD16.7-CTT4254-4203 had weaker cyst formation inhibitory effects than other polypeptides. Polypeptides other than CD16.7-CTT4104-4203 and CD16.7-CTT4254-4203 all had the amino acid sequence at positions 4204 to 4243 of SEQ ID NO:1. These results suggest that the whole CTT region would not be necessary for inhibition of cyst formation, and at least part of the CTT region, such as a partial region of the CTT region corresponding to at least part of positions 4154 to 4303, in particular to positions 4204 to 4243, of SEQ ID NO: 1 should be included.

### Example 3

### 1. Test methods

### 1.1 Transfection of expression vectors into cells and selection of transfected cells

To confirm the intracellular localization of the shortened PC1 polypeptide, an expression vector for CTF, CTT, CD16.7-CTT, or FAS-CTT was transfected into MDCK cells in the same manner as in Example 1. To 10 µL of cell suspension of 1×10⁷ cells/mL, 1 µg of the expression vector was suspended and the resulting suspension was put into the Neon Transfection System. Transformation was performed by electroporation under the conditions of voltage: 1150 V, width: 20ms, and number: 1. Then, the cells were added to a medium (DMEM/F12 containing 10% FCS) (1 mL/well, 12 well plate). On the next day, the medium was replaced with the same medium supplemented with 500 µg/mL hygromycin (Life Technologies Japan), and the cells were cultured for 5 days and passed to 2 × 10⁵cells/well in a 12-well plate.

### 1.2 Cell lysate preparation

The MDCK cells which had been passed into the 12 well plate the day before were washed three times with cold PBS on ice (1mL/well). After washing, 0.25 mg/mL of EZ-link sNHS-SS-biotin (Thermofisher Scientific) solution was added into each well (400 µL/well) and then left for 30 min on ice. After that, EZ-link sNHS-SS-biotin solution was removed and washed three times with DMEM (×1) (Gibco) (1 mL/well). And then 200 µL of 1×RIPA buffer (50 mM Tris-HCl pH 7.5/ 150 mM NaCl/ 0.1% SDS/ 0.5% SDC/ 1% Triton X-100) containing 8M Urea was added into each well and mixed by pipetting to lyse cells twice to obtain total 400µL of cell lysate. The protein concentration of the collected solution was measured with EZQ^{™} Protein Quantitation Kit (# R33200, Thermofisher Scientific) after being well mixed. Two µg (as total protein mass) of each sample was applied for whole cell analysis and the remaining sample was applied for cell surface analysis.

### 1.3 LC-MS measurements

### 1.3.1 Sample preparation for LC-MS analysis

Each sample was mixed with the same amount of 1×RIPA buffer containing 1M Arginine and then biotinylated protein was purified with Pierce^{™} Streptavidin Magnetic Beads (#88817, Thermofisher Scientific) to prepare for cell surface analysis. More specifically, each sample was incubated with mixing for 1 h at room temperature with the beads which were magnetically isolated from 40 µL of the beads solution and washed with mixing for 1 min in 200 µL of wash buffer (1×RIPA containing 8 M Urea and 400 mM Arginine) to make the biotinylated sample bind with the beads. After that, the beads were mixed for 30 sec in 1 mL of wash buffer and then magnetically collected to wash the beads. This procedure was repeated three times. After the washed beads were suspended in 50 mM DTT solution and incubated for 1 h at room temperature, magnetically separated supernatant was collected to isolate the protein sample from the biotin. The protein sample was then alkylated with 250 mM iodoacetamide (shaking incubation for 30 min at room temperature under light shielding). Two µg (as total protein mass) of cell lysate of the sample for whole cell analysis was reduced with 5 mM DTT (incubation for 15 min at 70°C) and alkylated with 50 mM iodoacetamide (shaking incubation for 30 min at room temperature under light shielding). After reduction and alkylation, the samples for both cell surface analysis and whole cell analysis were desalted and then digested with Trypsin/Lys-C Mix, Mass Spec Grade (#V5073, Promega, 0.025 µg/sample) for 18 h at 37°C. The digested peptides were desalted and applied for LC-MS analysis.

### 1.3.2 LC-MS analysis

After trypsin digestion of the proteins, the resulting peptides were analyzed using LC-MS. LC-MS was performed on a nanoHPLC (Ultimate 3000 nanoUHPLC, Thermofisher Scientific) combined with mass spectrometer (Orbitrap Eclipse, Thermofisher Scientific) which equipped with gas phase separation interface (FAIMS Pro, Thermofisher Scientific).In the Nano-HPLC system, a gradient analysis was performed by reversed-phase chromatography using a column which consists of a trap column (C18 Acclaim PepMap, Thermo Scientific) and a separation column (75 µm i.d., 12 cm long, 3 µm C18 particles, Nikkyo Technos) as a stationary phase and 0.1% formic acid-acetonitrile system as a mobile phase. The flow rate was 300 nl/min and peptides were eluted with a linear gradient of increasing acetonitrile concentration from 4% to 35% over 48 minutes. Mass spectrometry was performed in positive ion mode nano-electrospray ionization. In cell surface analysis, full scan MS spectra were obtained in a range of m/z 375-1500, and product ion spectra were acquired using the data-dependent acquisition (DDA) mode. The compensation voltages (CVs) in FAIMS pro were applied at -40V, - 60V and -80V for ion separation in gas phase. The acquired LC-MS data were analyzed with Proteome Discoverer 3.0 (PD 3.0, Thermo Fisher Scientific). The protein identification was performed using SEQUEST search program in PD 3.0 against the UniProt dog reference proteome (P000002254) containing expression genes such as human PKD1 CTT. Label-free quantification was performed with PD 3.0 using precursor ion quantifier nodes. In whole-cell proteome analysis, full scan MS spectra were acquired in the m/z range of 495-745, and product ion spectra were obtained in a data independent acquisition mode in which precursor ions were sequentially isolated with a width of 4 Da. The compensation voltage in FAIMS pro was applied at -45V for gas phase ion separation. MS raw data files were analyzed using DIA-NN software (ver.1.8.1) against uniprot dog reference proteome database (P00002254).

### 2. Results

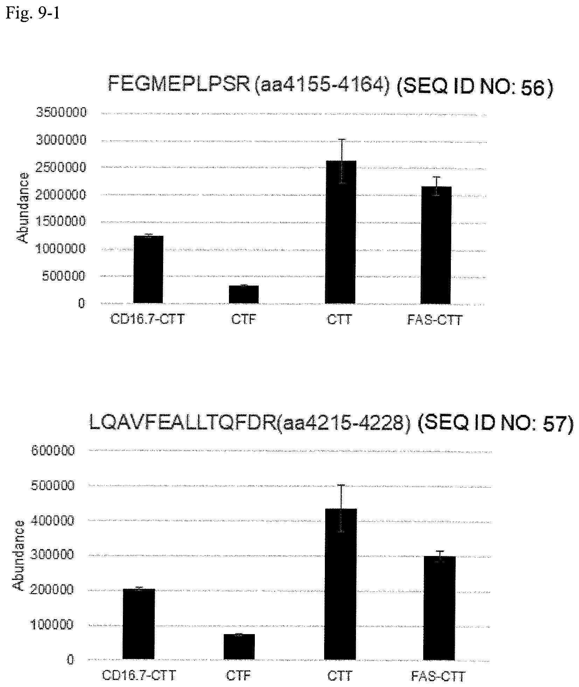

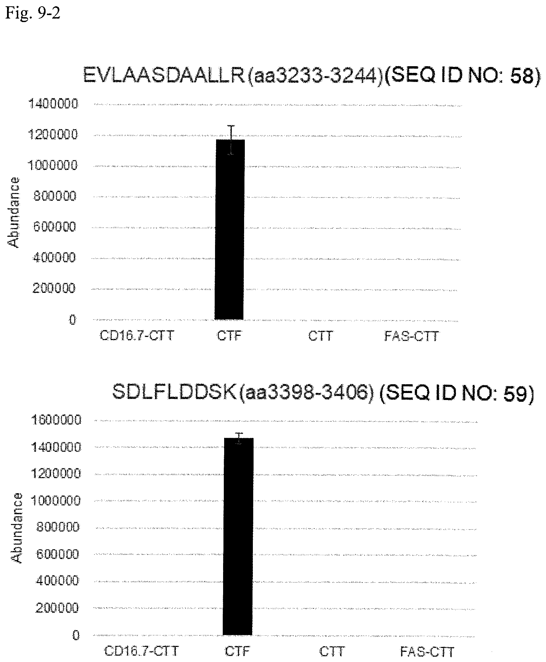

Results of LC-MS expression analysis of the shortened CTT polypeptides in MDCK cells are shown in Figs. 9-1 and 9-2. Using the sample for whole cell analysis, LC-MS analysis was performed for masses corresponding to two peptides within the CTT region (Table 4, No. 1 and No. 2). Expression of all of CD16.7-CTT, CTF, CTT, and FAS-CTT polypeptides was confirmed (Fig. 9-1). In contrast, when LC-MS analysis was performed using the samples for whole-cell analysis for masses corresponding to two peptides within the CTF 11-transmembrane region (Table 4, No. 3 and No. 4), only CTF expression was confirmed (Fig. 9-2). CD16.7-CTT, CTT, and FAS-CTT do not have regions corresponding to the peptides shown in Fig. 9-2 and thus their expression was not confirmed.

**Table 4**

| No. | Amino acid sequence | Amino acid positions | SEQ ID NO. |
|---|---|---|---|
| 1 | FEGMEPLPSR | 4155-4164 | 56 |
| 2 | LQAVFEALLTQFDR | 4215-4228 | 57 |
| 3 | EVLAASDAALLR | 3233-3244 | 58 |
| 4 | SDLFLDDSK | 3398-3406 | 59 |
| 5 | NSLDIFR | 3163-3169 | 60 |

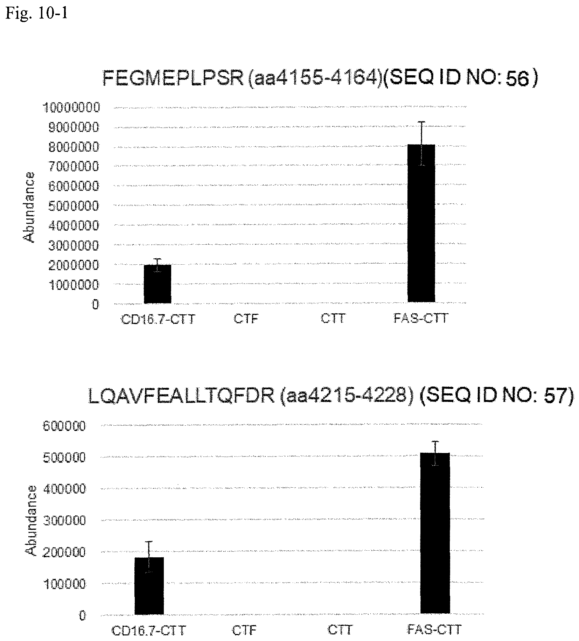

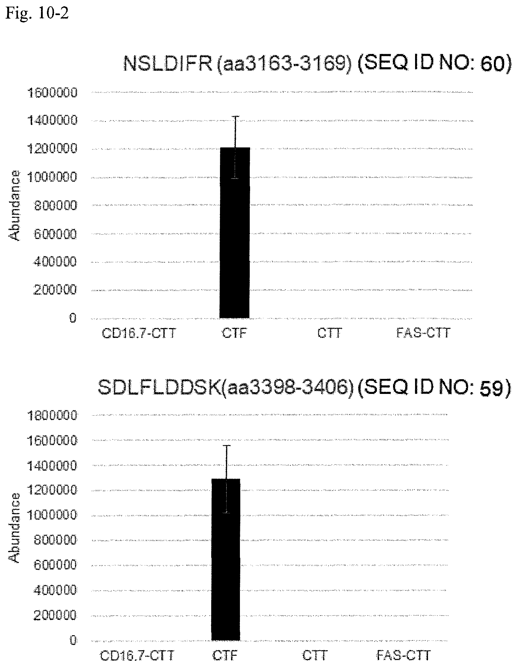

The results of LC-MS analysis of cell membrane localization of the shortened CTT polypeptides in MDCK cells are shown in Figs. 10-1 and 10-2. Using the samples for cell surface analysis, LC-MS analysis was performed for masses corresponding to two peptides within the CTT region (Table 4, No. 1 and No. 2). Then, expression of CD16.7-CTT and FAS-CTT, but not of CTF and CTT, was confirmed. Using the samples for cell surface analysis, LC-MS analysis was performed for masses corresponding to two peptides within the CTF 11-transmembrane region (Table 4, No. 5 and No. 4). Then, CTF was detected, confirming the cell surface localization of CTF.

The results of Figs. 9-1 to 9-2 and 10-1 to 10-2 indicate that shortened polypeptides except for CTT (i.e., CD16.7-CTT, CTF, and FAS-CTT) are localized at the cell membrane.

### Table 2-1

**Table 2-1**

| |
|---|
| SEQ ID NO: 1: Human PC1 |
| MPPAAPARLALALGLGLWLGALAGGPGRGCGPCEPPCLCGPAPGAACRVNCSGRGLRTLGPALRIPADATALDVSHNLLRALDVG LLANLSALAELDISNNKISTLEEGIFANLFNLSEINLSGNPFECDCGLAWLPRWAEEQQVRVVQPEAATCAGPGSLAGQPLLGIP LLDSGCGEEYVACLPDNSSGTVAAVSFSAAHEGLLQPEACSAFCFSTGQGLAALSEQGWCLCGAAQPSSASFACLSLCSGPPPPP APTCRGPTLLQHVFPASPGATLVGPHGPLASGQLAAFHIAAPLPVTATRWDFGDGSAEVDAAGPAASHRYVLPGRYHVTAVLALG AGSALLGTDVQVEAAPAALELVCPSSVQSDESLDLSIQNRGGSGLEAAYSIVALGEEPARAVHPLCPSDTEIFPGNGHCYRLVVE KAAWLQAQEQCQAWAGAALAMVDSPAVQRFLVSRVTRSLDVWIGFSTVQGVEVGPAPQGEAFSLESCQNWLPGEPHPATAEHCVR LGPTGWCNTDLCSAPHSYVCELQPGGPVQDAENLLVGAPSGDLQGPLTPLAQQDGLSAPHEPVEVMVFPGLRLSREAFLTTAEFG TQELRRPAQLRLQVYRLLSTAGTPENGSEPESRSPDNRTQLAPACMPGGRWCPGANICLPLDASCHPQACANGCTSGPGLPGAPY ALWREFLFSVPAGPPAQYSVTLHGQDVLMLPGDLVGLQHDAGPGALLHCSPAPGHPGPQAPYLSANASSWLPHLPAQLEGTWACP ACALRLLAATEQLTVLLGLRPNPGLRLPGRYEVRAEVGNGVSRHNLSCSFDVVSPVAGLRVIYPAPRDGRLYVPTNGSALVLQVD SGANATATARWPGGSVSARFENVCPALVATFVPGCPWETNDTLFSVVALPWLSEGEHVVDVVVENSASRANLSLRVTAEEPICGL RATPSPEARVLQGVLVRYSPVVEAGSDMVFRWTINDKQSLTFQNVVFNVIYQSAAVFKLSLTASNHVSNVTVNYNVTVERMNRMQ GLQVSTVPAVLSPNATLALTAGVLVDSAVEVAFLWTFGDGEQALHQFQPPYNESFPVPDPSVAQVLVEHNVMHTYAAPGEYLLTV LASNAFENLTQQVPVSVRASLPSVAVGVSDGVLVAGRPVTFYPHPLPSPGGVLYTWDFGDGSPVLTQSQPAANHTYASRGTYHVR LEVNNTVSGAAAQADVRVFEELRGLSVDMSLAVEQGAPVVVSAAVQTGDNITWTFDMGDGTVLSGPEATVEHVYLRAQNCTVTVG AASPAGHLARSLHVLVFVLEVLRVEPAACIPTQPDARLTAYVTGNPAHYLFDWTFGDGSSNTTVRGCPTVTHNFTRSGTFPLALV LSSRVNRAHYFTSICVEPEVGNVTLQPERQFVQLGDEAWLVACAWPPFPYRYTWDFGTEEAAPTRARGPEVTFIYRDPGSYLVTV TASNNISAANDSALVEVQEPVLVTSIKVNGSLGLELQQPYLFSAVGRGRPASYLWDLGDGGWLEGPEVTHAYNSTGDFTVRVAGW NEVSRSEAWLNVTVKRRVRGLVVNASRTVVPLNGSVSFSTSLEAGSDVRYSWVLCDRCTPIPGGPTISYTFRSVGTFNIIVTAEN EVGSAQDSIFVYVLQLIEGLQVVGGGRYFPTNHTVQLQAVVRDGTNVSYSWTAWRDRGPALAGSGKGFSLTVLEAGTYHVQLRAT NMLGSAWADCTMDFVEPVGWLMVAASPNPAAVNTSVTLSAELAGGSGVVYTWSLEEGLSWETSEPFTTHSFPTPGLHLVTMTAGN PLGSANATVEVDVQVPVSGLSIRASEPGGSFVAAGSSVPFWGQLATGTNVSWCWAVPGGSSKRGPHVTMVFPDAGTFSIRLNASN AVSWVSATYNLTAEEPIVGLVLWASSKVVAPGQLVHFQILLAAGSAVTFRLQVGGANPEVLPGPRFSHSFPRVGDHVVSVRGKNH VSWAQAQVRIVVLEAVSGLQVPNCCEPGIATGTERNFTARVQRGSRVAYAWYFSLQKVQGDSLVILSGRDVTYTPVAAGLLEIQV RAFNALGSENRTLVLEVQDAVQYVALQSGPCFTNRSAQFEAATSPSPRRVAYHWDFGDGSPGQDTDEPRAEHSYLRPGDYRVQVN ASNLVSFFVAQATVTVQVLACREPEVDVVLPLQVLMRRSQRNYLEAHVDLRDCVTYQTEYRWEVYRTASCQRPGRPARVALPGVD VSRPRLVLPRLALPVGHYCFVFVVSFGDTPLTQSIQANVTVAPERLVPIIEGGSYRVWSDTRDLVLDGSESYDPNLEDGDQTPLS FHWACVASTQREAGGCALNFGPRGSSTVTIPRERLAAGVEYTFSLTVWKAGRKEEATNQTVLIRSGRVPIVSLECVSCKAQAVYE VSRSSYVYLEGRCLNCSSGSKRGRWAARTFSNKTLVLDETTTSTGSAGMRLVLRRGVLRDGEGYTFTLTVLGRSGEEEGCASIRL SPNRPPLGGSCRLFPLGAVHALTTKVHFECTGWHDAEDAGAPLVYALLLRRCRQGHCEEFCVYKGSLSSYGAVLPPGFRPHFEVG LAVVVQDQLGAAVVALNRSLAITLPEPNGSATGLTVWLHGLTASVLPGLLRQADPQHVIEYSLALVTVLNEYERALDVAAEPKHE RQHRAQIRKNITETLVSLRVHTVDDIQQIAAALAQCMGPSRELVCRSCLKQTLHKLEAMMLILQAETTAGTVTPTAIGDSILNIT GDLIHLASSDVRAPQPSELGAESPSRMVASQAYNLTSALMRILMRSRVLNEEPLTLAGEEIVAQGKRSDPRSLLCYGGAPGPGCH FSIPEAFSGALANLSDVVQLIFLVDSNPFPFGYISNYTVSTKVASMAFQTQAGAQIPIERLASERAITVKVPNNSDWAARGHRSS ANSANSVVVQPQASVGAVVTLDSSNPAAGLHLQLNYTLLDGHYLSEEPEPYLAVYLHSEPRPNEHNCSASRRIRPESLQGADHRP YTFFISPGSRDPAGSYHLNLSSHFRWSALQVSVGLYTSLCQYFSEEDMVWRTEGLLPLEETSPRQAVCLTRHLTAFGASLFVPPS HVRFVFPEPTADVNYIVMLTCAVCLVTYMVMAAILHKLDQLDASRGRAIPFCGQRGRFKYEILVKTGWGRGSGTTAHVGIMLYGV DSRSGHRHLDGDRAFHRNSLDIFRIATPHSLGSVWKIRVWHDNKGLSPAWFLQHVIVRDLQTARSAFFLVNDWLSVETEANGGLV EKEVLAASDAALLRFRRLLVAELQRGFFDKHIWLSIWDRPPRSRFTRIQRATCCVLLICLFLGANAVWYGAVGDSAYSTGHVSRL SPLSVDTVAVGLVSSVVVYPVYLAILFLFRMSRSKVAGSPSPTPAGQQVLDIDSCLDSSVLDSSFLTFSGLHAEQAFVGQMKSDL |

### Table 2-2

**Table 2-2**

| |
|---|
| FLDDSKSLVCWPSGEGTLSWPDLLSDPSIVGSNLRQLARGQAGHGLGPEEDGFSLASPYSPAKSFSASDEDLIQQVLAEGVSSPA PTQDTHMETDLLSSLSSTPGEKTETLALQRLGELGPPSPGLNWEQPQAARLSRTGLVEGLRKRLLPAWCASLAHGLSLLLVAVAV AVSGWVGASFPPGVSVAWLLSSSASFLASFLGWEPLKVLLEALYFSLVAKRLHPDEDDTLVESPAVTPVSARVPRVRPPHGFALF LAKEEARKVKRLHGMLRSLLVYMLFLLVTLLASYGDASCHGHAYRLQSAIKQELHSRAFLAITRSEELWPWMAHVLLPYVHGNQS SPELGPPRLRQVRLQEALYPDPPGPRVHTCSAAGGFSTSDYDVGWESPHNGSGTWAYSAPDLLGAWSWGSCAVYDSGGYVQELGL SLEESRDRLRFLQLHNWLDNRSRAVFLELTRYSPAVGLHAAVTLRLEFPAAGRALAALSVRPFALRRLSAGLSLPLLTSVCLLLF AVHFAVAEARTWHREGRWRVLRLGAWARWLLVALTAATALVRLAQLGAADRQWTRFVRGRPRRFTSFDQVAQLSSAARGLAASLL FLLLVKAAQQLRFVRQWSVFGKTLCRALPELLGVTLGLVVLGVAYAQLAILLVSSCVDSLWSVAQALLVLCPGTGLSTLCPAESW HLSPLLCVGLWALRLWGALRLGAVILRWRYHALRGELYRPAWEPQDYEMVELFLRRLRLWMGLSKVKEFRHKVRFEGMEPLPSRS SRGSKVSPDVPPPSAGSDASHPSTSSSQLDGLSVSLGRLGTRCEPEPSRLQAVFEALLTQFDRLNQATEDVYQLEQQLHSLQGRR SSRAPAGSSRGPSPGLRPALPSRLARASRGVDLATGPSRTPLRAKNKVHPSST |
| |
| SEQ ID NO: 2: Human PC1 |
| ATGCCGCCCGCCGCGCCCGCCCGCCTGGCGCTGGCCCTGGGCCTGGGCCTGTGGCTCGGGGCGCTGGCGGGGGGCCCCGGGCGCG GCTGCGGGCCCTGCGAGCCCCCCTGCCTCTGCGGCCCAGCGCCCGGCGCCGCCTGCCGCGTCAACTGCTCGGGCCGCGGGCTGCG GACGCTCGGTCCCGCGCTGCGCATCCCCGCGGACGCCACAGCGCTAGACGTCTCCCACAACCTGCTCCGGGCGCTGGACGTTGGG CTCCTGGCGAACCTCTCGGCGCTGGCAGAGCTGGATATAAGCAACAACAAGATTTCTACGTTAGAAGAAGGAATATTTGCTAATT TATTTAATTTAAGTGAAATAAACCTGAGTGGGAACCCGTTTGAGTGTGACTGTGGCCTGGCGTGGCTGCCGCGATGGGCGGAGGA GCAGCAGGTGCGGGTGGTGCAGCCCGAGGCAGCCACGTGTGCTGGGCCTGGCTCCCTGGCTGGCCAGCCTCTGCTTGGCATCCCC TTGCTGGACAGTGGCTGTGGTGAGGAGTATGTCGCCTGCCTCCCTGACAACAGCTCAGGCACCGTGGCAGCAGTGTCCTTTTCAG CTGCCCACGAAGGCCTGCTTCAGCCAGAGGCCTGCAGCGCCTTCTGCTTCTCCACCGGCCAGGGCCTCGCAGCCCTCTCGGAGCA GGGCTGGTGCCTGTGTGGGGCGGCCCAGCCCTCCAGTGCCTCCTTTGCCTGCCTGTCCCTCTGCTCCGGCCCCCCGCCACCTCCT GCCCCCACCTGTAGGGGCCCCACCCTCCTCCAGCACGTCTTCCCTGCCTCCCCAGGGGCCACCCTGGTGGGGCCCCACGGACCTC TGGCCTCTGGCCAGCTAGCAGCCTTCCACATCGCTGCCCCGCTCCCTGTCACTGCCACACGCTGGGACTTCGGAGACGGCTCCGC CGAGGTGGATGCCGCTGGGCCGGCTGCCTCGCATCGCTATGTGCTGCCTGGGCGCTATCACGTGACGGCCGTGCTGGCCCTGGGG GCCGGCTCAGCCCTGCTGGGGACAGACGTGCAGGTGGAAGCGGCACCTGCCGCCCTGGAGCTCGTGTGCCCGTCCTCGGTGCAGA GTGACGAGAGCCTTGACCTCAGCATCCAGAACCGCGGTGGTTCAGGCCTGGAGGCCGCCTACAGCATCGTGGCCCTGGGCGAGGA GCCGGCCCGAGCGGTGCACCCGCTCTGCCCCTCGGACACGGAGATCTTCCCTGGCAACGGGCACTGCTACCGCCTGGTGGTGGAG AAGGCGGCCTGGCTGCAGGCGCAGGAGCAGTGTCAGGCCTGGGCCGGGGCCGCCCTGGCAATGGTGGACAGTCCCGCCGTGCAGC GCTTCCTGGTCTCCCGGGTCACCAGGAGCCTAGACGTGTGGATCGGCTTCTCGACTGTGCAGGGGGTGGAGGTGGGCCCAGCGCC GCAGGGCGAGGCCTTCAGCCTGGAGAGCTGCCAGAACTGGCTGCCCGGGGAGCCACACCCAGCCACAGCCGAGCACTGCGTCCGG CTCGGGCCCACCGGGTGGTGTAACACCGACCTGTGCTCAGCGCCGCACAGCTACGTCTGCGAGCTGCAGCCCGGAGGCCCAGTGC AGGATGCCGAGAACCTCCTCGTGGGAGCGCCCAGTGGGGACCTGCAGGGACCCCTGACGCCTCTGGCACAGCAGGACGGCCTCTC AGCCCCGCACGAGCCCGTGGAGGTCATGGTATTCCCGGGCCTGCGTCTGAGCCGTGAAGCCTTCCTCACCACGGCCGAATTTGGG ACCCAGGAGCTCCGGCGGCCCGCCCAGCTGCGGCTGCAGGTGTACCGGCTCCTCAGCACAGCAGGGACCCCGGAGAACGGCAGCG AGCCTGAGAGCAGGTCCCCGGACAACAGGACCCAGCTGGCCCCCGCGTGCATGCCAGGGGGACGCTGGTGCCCTGGAGCCAACAT CTGCTTGCCGCTGGACGCCTCCTGCCACCCCCAGGCCTGCGCCAATGGCTGCACGTCAGGGCCAGGGCTACCCGGGGCCCCCTAT GCGCTATGGAGAGAGTTCCTCTTCTCCGTTCCCGCGGGGCCCCCCGCGCAGTACTCGGTCACCCTCCACGGCCAGGATGTCCTCA TGCTCCCTGGTGACCTCGTTGGCTTGCAGCACGACGCTGGCCCTGGCGCCCTCCTGCACTGCTCGCCGGCTCCCGGCCACCCTGG TCCCCAGGCCCCGTACCTCTCCGCCAACGCCTCGTCATGGCTGCCCCACTTGCCAGCCCAGCTGGAGGGCACTTGGGCCTGCCCT GCCTGTGCCCTGCGGCTGCTTGCAGCCACGGAACAGCTCACCGTGCTGCTGGGCTTGAGGCCCAACCCTGGACTGCGGCTGCCTG |

### Table 2-3

**Table 2-3**

| |
|---|
| GGCGCTATGAGGTCCGGGCAGAGGTGGGCAATGGCGTGTCCAGGCACAACCTCTCCTGCAGCTTTGACGTGGTCTCCCCAGTGGC TGGGCTGCGGGTCATCTACCCTGCCCCCCGCGACGGCCGCCTCTACGTGCCCACCAACGGCTCAGCCTTGGTGCTCCAGGTGGAC TCTGGTGCCAACGCCACGGCCACGGCTCGCTGGCCTGGGGGCAGTGTCAGCGCCCGCTTTGAGAATGTCTGCCCTGCCCTGGTGG CCACCTTCGTGCCCGGCTGCCCCTGGGAGACCAACGATACCCTGTTCTCAGTGGTAGCACTGCCGTGGCTCAGTGAGGGGGAGCA CGTGGTGGACGTGGTGGTGGAAAACAGCGCCAGCCGGGCCAACCTCAGCCTGCGGGTGACGGCGGAGGAGCCCATCTGTGGCCTC CGCGCCACGCCCAGCCCCGAGGCCCGTGTACTGCAGGGAGTCCTAGTGAGGTACAGCCCCGTGGTGGAGGCCGGCTCGGACATGG TCTTCCGGTGGACCATCAACGACAAGCAGTCCCTGACCTTCCAGAACGTGGTCTTCAATGTCATTTATCAGAGCGCGGCGGTCTT CAAGCTCTCACTGACGGCCTCCAACCACGTGAGCAACGTCACCGTGAACTACAACGTAACCGTGGAGCGGATGAACAGGATGCAG GGTCTGCAGGTCTCCACAGTGCCGGCCGTGCTGTCCCCCAATGCCACGCTAGCACTGACGGCGGGCGTGCTGGTGGACTCGGCCG TGGAGGTGGCCTTCCTGTGGACCTTTGGGGATGGGGAGCAGGCCCTCCACCAGTTCCAGCCTCCGTACAACGAGTCCTTCCCGGT TCCAGACCCCTCGGTGGCCCAGGTGCTGGTGGAGCACAATGTCATGCACACCTACGCTGCCCCAGGTGAGTACCTCCTGACCGTG CTGGCATCTAATGCCTTCGAGAACCTGACGCAGCAGGTGCCTGTGAGCGTGCGCGCCTCCCTGCCCTCCGTGGCTGTGGGTGTGA GTGACGGCGTCCTGGTGGCCGGCCGGCCCGTCACCTTCTACCCGCACCCGCTGCCCTCGCCTGGGGGTGTTCTTTACACGTGGGA CTTCGGGGACGGCTCCCCTGTCCTGACCCAGAGCCAGCCGGCTGCCAACCACACCTATGCCTCGAGGGGCACCTACCACGTGCGC CTGGAGGTCAACAACACGGTGAGCGGTGCGGCGGCCCAGGCGGATGTGCGCGTCTTTGAGGAGCTCCGCGGACTCAGCGTGGACA TGAGCCTGGCCGTGGAGCAGGGCGCCCCCGTGGTGGTCAGCGCCGCGGTGCAGACGGGCGACAACATCACGTGGACCTTCGACAT GGGGGACGGCACCGTGCTGTCGGGCCCGGAGGCAACAGTGGAGCATGTGTACCTGCGGGCACAGAACTGCACAGTGACCGTGGGT GCGGCCAGCCCCGCCGGCCACCTGGCCCGGAGCCTGCACGTGCTGGTCTTCGTCCTGGAGGTGCTGCGCGTTGAACCCGCCGCCT GCATCCCCACGCAGCCTGACGCGCGGCTCACGGCCTACGTCACCGGGAACCCGGCCCACTACCTCTTCGACTGGACCTTCGGGGA TGGCTCCTCCAACACGACCGTGCGGGGGTGCCCGACGGTGACACACAACTTCACGCGGAGCGGCACGTTCCCCCTGGCGCTGGTG CTGTCCAGCCGCGTGAACAGGGCGCATTACTTCACCAGCATCTGCGTGGAGCCAGAGGTGGGCAACGTCACCCTGCAGCCAGAGA GGCAGTTTGTGCAGCTCGGGGACGAGGCCTGGCTGGTGGCATGTGCCTGGCCCCCGTTCCCCTACCGCTACACCTGGGACTTTGG CACCGAGGAAGCCGCCCCCACCCGTGCCAGGGGCCCTGAGGTGACGTTCATCTACCGAGACCCAGGCTCCTATCTTGTGACAGTC ACCGCGTCCAACAACATCTCTGCTGCCAATGACTCAGCCCTGGTGGAGGTGCAGGAGCCCGTGCTGGTCACCAGCATCAAGGTCA ATGGCTCCCTTGGGCTGGAGCTGCAGCAGCCGTACCTGTTCTCTGCTGTGGGCCGTGGGCGCCCCGCCAGCTACCTGTGGGATCT GGGGGACGGTGGGTGGCTCGAGGGTCCGGAGGTCACCCACGCTTACAACAGCACAGGTGACTTCACCGTTAGGGTGGCCGGCTGG AATGAGGTGAGCCGCAGCGAGGCCTGGCTCAATGTGACGGTGAAGCGGCGCGTGCGGGGGCTCGTCGTCAATGCAAGCCGCACGG TGGTGCCCCTGAATGGGAGCGTGAGCTTCAGCACGTCGCTGGAGGCCGGCAGTGATGTGCGCTATTCCTGGGTGCTCTGTGACCG CTGCACGCCCATCCCTGGGGGTCCTACCATCTCTTACACCTTCCGCTCCGTGGGCACCTTCAATATCATCGTCACGGCTGAGAAC GAGGTGGGCTCCGCCCAGGACAGCATCTTCGTCTATGTCCTGCAGCTCATAGAGGGGCTGCAGGTGGTGGGCGGTGGCCGCTACT TCCCCACCAACCACACGGTACAGCTGCAGGCCGTGGTTAGGGATGGCACCAACGTCTCCTACAGCTGGACTGCCTGGAGGGACAG GGGCCCGGCCCTGGCCGGCAGCGGCAAAGGCTTCTCGCTCACCGTGCTCGAGGCCGGCACCTACCATGTGCAGCTGCGGGCCACC AACATGCTGGGCAGCGCCTGGGCCGACTGCACCATGGACTTCGTGGAGCCTGTGGGGTGGCTGATGGTGGCCGCCTCCCCGAACC CAGCTGCCGTCAACACAAGCGTCACCCTCAGTGCCGAGCTGGCTGGTGGCAGTGGTGTCGTATACACTTGGTCCTTGGAGGAGGG GCTGAGCTGGGAGACCTCCGAGCCATTTACCACCCATAGCTTCCCCACACCCGGCCTGCACTTGGTCACCATGACGGCAGGGAAC CCGCTGGGCTCAGCCAACGCCACCGTGGAAGTGGATGTGCAGGTGCCTGTGAGTGGCCTCAGCATCAGGGCCAGCGAGCCCGGAG GCAGCTTCGTGGCGGCCGGGTCCTCTGTGCCCTTTTGGGGGCAGCTGGCCACGGGCACCAATGTGAGCTGGTGCTGGGCTGTGCC CGGCGGCAGCAGCAAGCGTGGCCCTCATGTCACCATGGTCTTCCCGGATGCTGGCACCTTCTCCATCCGGCTCAATGCCTCCAAC GCAGTCAGCTGGGTCTCAGCCACGTACAACCTCACGGCGGAGGAGCCCATCGTGGGCCTGGTGCTGTGGGCCAGCAGCAAGGTGG TGGCGCCCGGGCAGCTGGTCCATTTTCAGATCCTGCTGGCTGCCGGCTCAGCTGTCACCTTCCGCCTGCAGGTCGGCGGGGCCAA CCCCGAGGTGCTCCCCGGGCCCCGTTTCTCCCACAGCTTCCCCCGCGTCGGAGACCACGTGGTGAGCGTGCGGGGCAAAAACCAC |

### Table 2-4

**Table 2-4**

| |
|---|
| GTGAGCTGGGCCCAGGCGCAGGTGCGCATCGTGGTGCTGGAGGCCGTGAGTGGGCTGCAGGTGCCCAACTGCTGCGAGCCTGGCA TCGCCACGGGCACTGAGAGGAACTTCACAGCCCGCGTGCAGCGCGGCTCTCGGGTCGCCTACGCCTGGTACTTCTCGCTGCAGAA GGTCCAGGGCGACTCGCTGGTCATCCTGTCGGGCCGCGACGTCACCTACACGCCCGTGGCCGCGGGGCTGTTGGAGATCCAGGTG CGCGCCTTCAACGCCCTGGGCAGTGAGAACCGCACGCTGGTGCTGGAGGTTCAGGACGCCGTCCAGTATGTGGCCCTGCAGAGCG GCCCCTGCTTCACCAACCGCTCGGCGCAGTTTGAGGCCGCCACCAGCCCCAGCCCCCGGCGTGTGGCCTACCACTGGGACTTTGG GGATGGGTCGCCAGGGCAGGACACAGATGAGCCCAGGGCCGAGCACTCCTACCTGAGGCCTGGGGACTACCGCGTGCAGGTGAAC GCCTCCAACCTGGTGAGCTTCTTCGTGGCGCAGGCCACGGTGACCGTCCAGGTGCTGGCCTGCCGGGAGCCGGAGGTGGACGTGG TCCTGCCCCTGCAGGTGCTGATGCGGCGATCACAGCGCAACTACTTGGAGGCCCACGTTGACCTGCGCGACTGCGTCACCTACCA GACTGAGTACCGCTGGGAGGTGTATCGCACCGCCAGCTGCCAGCGGCCGGGGCGCCCAGCGCGTGTGGCCCTGCCCGGCGTGGAC GTGAGCCGGCCTCGGCTGGTGCTGCCGCGGCTGGCGCTGCCTGTGGGGCACTACTGCTTTGTGTTTGTCGTGTCATTTGGGGACA CGCCACTGACACAGAGCATCCAGGCCAATGTGACGGTGGCCCCCGAGCGCCTGGTGCCCATCATTGAGGGTGGCTCATACCGCGT GTGGTCAGACACACGGGACCTGGTGCTGGATGGGAGCGAGTCCTACGACCCCAACCTGGAGGACGGCGACCAGACGCCGCTCAGT TTCCACTGGGCCTGTGTGGCTTCGACACAGAGGGAGGCTGGCGGGTGTGCGCTGAACTTTGGGCCCCGCGGGAGCAGCACGGTCA CCATTCCACGGGAGCGGCTGGCGGCTGGCGTGGAGTACACCTTCAGCCTGACCGTGTGGAAGGCCGGCCGCAAGGAGGAGGCCAC CAACCAGACGGTGCTGATCCGGAGTGGCCGGGTGCCCATTGTGTCCTTGGAGTGTGTGTCCTGCAAGGCACAGGCCGTGTACGAA GTGAGCCGCAGCTCCTACGTGTACTTGGAGGGCCGCTGCCTCAATTGCAGCAGCGGCTCCAAGCGAGGGCGGTGGGCTGCACGTA CGTTCAGCAACAAGACGCTGGTGCTGGATGAGACCACCACATCCACGGGCAGTGCAGGCATGCGACTGGTGCTGCGGCGGGGCGT GCTGCGGGACGGCGAGGGATACACCTTCACGCTCACGGTGCTGGGCCGCTCTGGCGAGGAGGAGGGCTGCGCCTCCATCCGCCTG TCCCCCAACCGCCCGCCGCTGGGGGGCTCTTGCCGCCTCTTCCCACTGGGCGCTGTGCACGCCCTCACCACCAAGGTGCACTTCG AATGCACGGGCTGGCATGACGCGGAGGATGCTGGCGCCCCGCTGGTGTACGCCCTGCTGCTGCGGCGCTGTCGCCAGGGCCACTG CGAGGAGTTCTGTGTCTACAAGGGCAGCCTCTCCAGCTACGGAGCCGTGCTGCCCCCGGGTTTCAGGCCACACTTCGAGGTGGGC CTGGCCGTGGTGGTGCAGGACCAGCTGGGAGCCGCTGTGGTCGCCCTCAACAGGTCTTTGGCCATCACCCTCCCAGAGCCCAACG GCAGCGCAACGGGGCTCACAGTCTGGCTGCACGGGCTCACCGCTAGTGTGCTCCCAGGGCTGCTGCGGCAGGCCGATCCCCAGCA CGTCATCGAGTACTCGTTGGCCCTGGTCACCGTGCTGAACGAGTACGAGCGGGCCCTGGACGTGGCGGCAGAGCCCAAGCACGAG CGGCAGCACCGAGCCCAGATACGCAAGAACATCACGGAGACTCTGGTGTCCCTGAGGGTCCACACTGTGGATGACATCCAGCAGA TCGCTGCTGCGCTGGCCCAGTGCATGGGGCCCAGCAGGGAGCTCGTATGCCGCTCGTGCCTGAAGCAGACGCTGCACAAGCTGGA GGCCATGATGCTCATCCTGCAGGCAGAGACCACCGCGGGCACCGTGACGCCCACCGCCATCGGAGACAGCATCCTCAACATCACA GGAGACCTCATCCACCTGGCCAGCTCGGACGTGCGGGCACCACAGCCCTCAGAGCTGGGAGCCGAGTCACCATCTCGGATGGTGG CGTCCCAGGCCTACAACCTGACCTCTGCCCTCATGCGCATCCTCATGCGCTCCCGCGTGCTCAACGAGGAGCCCCTGACGCTGGC GGGCGAGGAGATCGTGGCCCAGGGCAAGCGCTCGGACCCGCGGAGCCTGCTGTGCTATGGCGGCGCCCCAGGGCCTGGCTGCCAC TTCTCCATCCCCGAGGCTTTCAGCGGGGCCCTGGCCAACCTCAGTGACGTGGTGCAGCTCATCTTTCTGGTGGACTCCAATCCCT TTCCCTTTGGCTATATCAGCAACTACACCGTCTCCACCAAGGTGGCCTCGATGGCATTCCAGACACAGGCCGGCGCCCAGATCCC CATCGAGCGGCTGGCCTCAGAGCGCGCCATCACCGTGAAGGTGCCCAACAACTCGGACTGGGCTGCCCGGGGCCACCGCAGCTCC GCCAACTCCGCCAACTCCGTTGTGGTCCAGCCCCAGGCCTCCGTCGGTGCTGTGGTCACCCTGGACAGCAGCAACCCTGCGGCCG GGCTGCATCTGCAGCTCAACTATACGCTGCTGGACGGCCACTACCTGTCTGAGGAACCTGAGCCCTACCTGGCAGTCTACCTACA CTCGGAGCCCCGGCCCAATGAGCACAACTGCTCGGCTAGCAGGAGGATCCGCCCAGAGTCACTCCAGGGTGCTGACCACCGGCCC TACACCTTCTTCATTTCCCCGGGGAGCAGAGACCCAGCGGGGAGTTACCATCTGAACCTCTCCAGCCACTTCCGCTGGTCGGCGC TGCAGGTGTCCGTGGGCCTGTACACGTCCCTGTGCCAGTACTTCAGCGAGGAGGACATGGTGTGGCGGACAGAGGGGCTGCTGCC CCTGGAGGAGACCTCGCCCCGCCAGGCCGTCTGCCTCACCCGCCACCTCACCGCCTTCGGCGCCAGCCTCTTCGTGCCCCCAAGC CATGTCCGCTTTGTGTTTCCTGAGCCGACAGCGGATGTAAACTACATCGTCATGCTGACATGTGCTGTGTGCCTGGTGACCTACA TGGTCATGGCCGCCATCCTGCACAAGCTGGACCAGTTGGATGCCAGCCGGGGCCGCGCCATCCCTTTCTGTGGGCAGCGGGGCCG |

### Table 2-5

**Table 2-5**

| |
|---|
| CTTCAAGTACGAGATCCTCGTCAAGACAGGCTGGGGCCGGGGCTCAGGTACCACGGCCCACGTGGGCATCATGCTGTATGGGGTG GACAGCCGGAGCGGCCACCGGCACCTGGACGGCGACAGAGCCTTCCACCGCAACAGCCTGGACATCTTCCGGATCGCCACCCCGC ACAGCCTGGGTAGCGTGTGGAAGATCCGAGTGTGGCACGACAACAAAGGGCTCAGCCCTGCCTGGTTCCTGCAGCACGTCATCGT CAGGGACCTGCAGACGGCACGCAGCGCCTTCTTCCTGGTCAATGACTGGCTTTCGGTGGAGACGGAGGCCAACGGGGGCCTGGTG GAGAAGGAGGTGCTGGCCGCGAGCGACGCAGCCCTTTTGCGCTTCCGGCGCCTGCTGGTGGCTGAGCTGCAGCGTGGCTTCTTTG ACAAGCACATCTGGCTCTCCATATGGGACCGGCCGCCTCGTAGCCGTTTCACTCGCATCCAGAGGGCCACCTGCTGCGTTCTCCT CATCTGCCTCTTCCTGGGCGCCAACGCCGTGTGGTACGGGGCTGTTGGCGACTCTGCCTACAGCACGGGGCATGTGTCCAGGCTG AGCCCGCTGAGCGTCGACACAGTCGCTGTTGGCCTGGTGTCCAGCGTGGTTGTCTATCCCGTCTACCTGGCCATCCTTTTTCTCT TCCGGATGTCCCGGAGCAAGGTGGCTGGGAGCCCGAGCCCCACACCTGCCGGGCAGCAGGTGCTGGACATCGACAGCTGCCTGGA CTCGTCCGTGCTGGACAGCTCCTTCCTCACGTTCTCAGGCCTCCACGCTGAGCAGGCCTTTGTTGGACAGATGAAGAGTGACTTG TTTCTGGATGATTCTAAGAGTCTGGTGTGCTGGCCCTCCGGCGAGGGAACGCTCAGTTGGCCGGACCTGCTCAGTGACCCGTCCA TTGTGGGTAGCAATCTGCGGCAGCTGGCACGGGGCCAGGCGGGCCATGGGCTGGGCCCAGAGGAGGACGGCTTCTCCCTGGCCAG CCCCTACTCGCCTGCCAAATCCTTCTCAGCATCAGATGAAGACCTGATCCAGCAGGTCCTTGCCGAGGGGGTCAGCAGCCCAGCC CCTACCCAAGACACCCACATGGAAACGGACCTGCTCAGCAGCCTGTCCAGCACTCCTGGGGAGAAGACAGAGACGCTGGCGCTGC AGAGGCTGGGGGAGCTGGGGCCACCCAGCCCAGGCCTGAACTGGGAACAGCCCCAGGCAGCGAGGCTGTCCAGGACAGGACTGGT GGAGGGTCTGCGGAAGCGCCTGCTGCCGGCCTGGTGTGCCTCCCTGGCCCACGGGCTCAGCCTGCTCCTGGTGGCTGTGGCTGTG GCTGTCTCAGGGTGGGTGGGTGCGAGCTTCCCCCCGGGCGTGAGTGTTGCGTGGCTCCTGTCCAGCAGCGCCAGCTTCCTGGCCT CATTCCTCGGCTGGGAGCCACTGAAGGTCTTGCTGGAAGCCCTGTACTTCTCACTGGTGGCCAAGCGGCTGCACCCGGATGAAGA TGACACCCTGGTAGAGAGCCCGGCTGTGACGCCTGTGAGCGCACGTGTGCCCCGCGTACGGCCACCCCACGGCTTTGCACTCTTC CTGGCCAAGGAAGAAGCCCGCAAGGTCAAGAGGCTACATGGCATGCTGCGGAGCCTCCTGGTGTACATGCTTTTTCTGCTGGTGA CCCTGCTGGCCAGCTATGGGGATGCCTCATGCCATGGGCACGCCTACCGTCTGCAAAGCGCCATCAAGCAGGAGCTGCACAGCCG GGCCTTCCTGGCCATCACGCGGTCTGAGGAGCTCTGGCCATGGATGGCCCACGTGCTGCTGCCCTACGTCCACGGGAACCAGTCC AGCCCAGAGCTGGGGCCCCCACGGCTGCGGCAGGTGCGGCTGCAGGAAGCACTCTACCCAGACCCTCCCGGCCCCAGGGTCCACA CGTGCTCGGCCGCAGGAGGCTTCAGCACCAGCGATTACGACGTTGGCTGGGAGAGTCCTCACAATGGCTCGGGGACGTGGGCCTA TTCAGCGCCGGATCTGCTGGGGGCATGGTCCTGGGGCTCCTGTGCCGTGTATGACAGCGGGGGCTACGTGCAGGAGCTGGGCCTG AGCCTGGAGGAGAGCCGCGACCGGCTGCGCTTCCTGCAGCTGCACAACTGGCTGGACAACAGGAGCCGCGCTGTGTTCCTGGAGC TCACGCGCTACAGCCCGGCCGTGGGGCTGCACGCCGCCGTCACGCTGCGCCTCGAGTTCCCGGCGGCCGGCCGCGCCCTGGCCGC CCTCAGCGTCCGCCCCTTTGCGCTGCGCCGCCTCAGCGCGGGCCTCTCGCTGCCTCTGCTCACCTCGGTGTGCCTGCTGCTGTTC GCCGTGCACTTCGCCGTGGCCGAGGCCCGTACTTGGCACAGGGAAGGGCGCTGGCGCGTGCTGCGGCTCGGAGCCTGGGCGCGGT GGCTGCTGGTGGCGCTGACGGCGGCCACGGCACTGGTACGCCTCGCCCAGCTGGGTGCCGCTGACCGCCAGTGGACCCGTTTCGT GCGCGGCCGCCCGCGCCGCTTCACTAGCTTCGACCAGGTGGCGCAGCTGAGCTCCGCAGCCCGTGGCCTGGCGGCCTCGCTGCTC TTCCTGCTTTTGGTCAAGGCTGCCCAGCAGCTACGCTTCGTGCGCCAGTGGTCCGTCTTTGGCAAGACATTATGCCGAGCTCTGC CAGAGCTCCTGGGGGTCACCTTGGGCCTGGTGGTGCTCGGGGTAGCCTACGCCCAGCTGGCCATCCTGCTCGTGTCTTCCTGTGT GGACTCCCTCTGGAGCGTGGCCCAGGCCCTGTTGGTGCTGTGCCCTGGGACTGGGCTCTCTACCCTGTGTCCTGCCGAGTCCTGG CACCTGTCACCCCTGCTGTGTGTGGGGCTCTGGGCACTGCGGCTGTGGGGCGCCCTACGGCTGGGGGCTGTTATTCTCCGCTGGC GCTACCACGCCTTGCGTGGAGAGCTGTACCGGCCGGCCTGGGAGCCCCAGGACTACGAGATGGTGGAGTTGTTCCTGCGCAGGCT GCGCCTCTGGATGGGCCTCAGCAAGGTCAAGGAGTTCCGCCACAAAGTCCGCTTTGAAGGGATGGAGCCGCTGCCCTCTCGCTCC TCCAGGGGCTCCAAGGTATCCCCGGATGTGCCCCCACCCAGCGCTGGCTCCGATGCCTCGCACCCCTCCACCTCCTCCAGCCAGC TGGATGGGCTGAGCGTGAGCCTGGGCCGGCTGGGGACAAGGTGTGAGCCTGAGCCCTCCCGCCTCCAAGCCGTGTTCGAGGCCCT GCTCACCCAGTTTGACCGACTCAACCAGGCCACAGAGGACGTCTACCAGCTGGAGCAGCAGCTGCACAGCCTGCAAGGCCGCAGG AGCAGCCGGGCGCCCGCCGGATCTTCCCGTGGCCCATCCCCGGGCCTGCGGCCAGCACTGCCCAGCCGCCTTGCCCGGGCCAGTC |

### Table 2-6

**Table 2-6**

| |
|---|
| GGGGTGTGGACCTGGCCACTGGCCCCAGCAGGACACCCCTTCGGGCCAAGAACAAGGTCCACCCCAGCAGCACTTAA |
| |
| SEQ ID NO: 3: At positions 4102 to 4303 of SEQ ID NO: 1 |
| GAVILRWRYHALRGELYRPAWEPQDYEMVELFLRRLRLWMGLSKVKEFRHKVRFEGMEPLPSRSSRGSKVSPDVPPPSAGSDASH PSTSSSQLDGLSVSLGRLGTRCEPEPSRLQAVFEALLTQFDRLNQATEDVYQLEQQLHSLQGRRSSRAPAGSSRGPSPGLRPALP SRLARASRGVDLATGPSRTPLRAKNKVHPSST |
| |
| SEQ ID NO: 4: At positions 12304 to 12909 of SEQ ID NO: 2 |
| GGGGCTGTTATTCTCCGCTGGCGCTACCACGCCTTGCGTGGAGAGCTGTACCGGCCGGCCTGGGAGCCCCAGGACTACGAGATGG TGGAGTTGTTCCTGCGCAGGCTGCGCCTCTGGATGGGCCTCAGCAAGGTCAAGGAGTTCCGCCACAAAGTCCGCTTTGAAGGGAT GGAGCCGCTGCCCTCTCGCTCCTCCAGGGGCTCCAAGGTATCCCCGGATGTGCCCCCACCCAGCGCTGGCTCCGATGCCTCGCAC CCCTCCACCTCCTCCAGCCAGCTGGATGGGCTGAGCGTGAGCCTGGGCCGGCTGGGGACAAGGTGTGAGCCTGAGCCCTCCCGCC TCCAAGCCGTGTTCGAGGCCCTGCTCACCCAGTTTGACCGACTCAACCAGGCCACAGAGGACGTCTACCAGCTGGAGCAGCAGCT GCACAGCCTGCAAGGCCGCAGGAGCAGCCGGGCGCCCGCCGGATCTTCCCGTGGCCCATCCCCGGGCCTGCGGCCAGCACTGCCC AGCCGCCTTGCCCGGGCCAGTCGGGGTGTGGACCTGGCCACTGGCCCCAGCAGGACACCCCTTCGGGCCAAGAACAAGGTCCACC CCAGCAGCACT |
| |
| SEQ ID NO: 5: CTF |
| MTAFGASLFVPPSHVRFVFPEPTADVNYIVMLTCAVCLVTYMVMAAILHKLDQLDASRGRAIPFCGQRGRFKYEILVKTGWGRGS GTTAHVGIMLYGVDSRSGHRHLDGDRAFHRNSLDIFRIATPHSLGSVWKIRVWHDNKGLSPAWFLQHVIVRDLQTARSAFFLVND WLSVETEANGGLVEKEVLAASDAALLRFRRLLVAELQRGFFDKHIWLSIWDRPPRSRFTRIQRATCCVLLICLFLGANAVWYGAV GDSAYSTGHVSRLSPLSVDTVAVGLVSSVVVYPVYLAILFLFRMSRSKVAGSPSPTPAGQQVLDIDSCLDSSVLDSSFLTFSGLH AEQAFVGQMKSDLFLDDSKSLVCWPSGEGTLSWPDLLSDPSIVGSNLRQLARGQAGHGLGPEEDGFSLASPYSPAKSFSASDEDL IQQVLAEGVSSPAPTQDTHMETDLLSSLSSTPGEKTETLALQRLGELGPPSPGLNWEQPQAARLSRTGLVEGLRKRLLPAWCASL AHGLSLLLVAVAVAVSGWVGASFPPGVSVAWLLSSSASFLASFLGWEPLKVLLEALYFSLVAKRLHPDEDDTLVESPAVTPVSAR VPRVRPPHGFALFLAKEEARKVKRLHGMLRSLLVYMLFLLVTLLASYGDASCHGHAYRLQSAIKQELHSRAFLAITRSEELWPWM AHVLLPYVHGNQSSPELGPPRLRQVRLQEALYPDPPGPRVHTCSAAGGFSTSDYDVGWESPHNGSGTWAYSAPDLLGAWSWGSCA VYDSGGYVQELGLSLEESRDRLRFLQLHNWLDNRSRAVFLELTRYSPAVGLHAAVTLRLEFPAAGRALAALSVRPFALRRLSAGL SLPLLTSVCLLLFAVHFAVAEARTWHREGRWRVLRLGAWARWLLVALTAATALVRLAQLGAADRQWTRFVRGRPRRFTSFDQVAQ LSSAARGLAASLLFLLLVKAAQQLRFVRQWSVFGKTLCRALPELLGVTLGLVVLGVAYAQLAILLVSSCVDSLWSVAQALLVLCP GTGLSTLCPAESWHLSPLLCVGLWALRLWGALRLGAVILRWRYHALRGELYRPAWEPQDYEMVELFLRRLRLWMGLSKVKEFRHK VRFEGMEPLPSRSSRGSKVSPDVPPPSAGSDASHPSTSSSQLDGLSVSLGRLGTRCEPEPSRLQAVFEALLTQFDRLNQATEDVY QLEQQLHSLQGRRSSRAPAGSSRGPSPGLRPALPSRLARASRGVDLATGPSRTPLRAKNKVHPSST |
| |
| SEQ ID NO: 6: CTF |
| ATGACCGCCTTCGGCGCCAGCCTCTTCGTGCCCCCAAGCCATGTCCGCTTTGTGTTTCCTGAGCCGACAGCGGATGTAAACTACA TCGTCATGCTGACATGTGCTGTGTGCCTGGTGACCTACATGGTCATGGCCGCCATCCTGCACAAGCTGGACCAGTTGGATGCCAG CCGGGGCCGCGCCATCCCTTTCTGTGGGCAGCGGGGCCGCTTCAAGTACGAGATCCTCGTCAAGACAGGCTGGGGCCGGGGCTCA GGTACCACGGCCCACGTGGGCATCATGCTGTATGGGGTGGACAGCCGGAGCGGCCACCGGCACCTGGACGGCGACAGAGCCTTCC ACCGCAACAGCCTGGACATCTTCCGGATCGCCACCCCGCACAGCCTGGGTAGCGTGTGGAAGATCCGAGTGTGGCACGACAACAA AGGGCTCAGCCCTGCCTGGTTCCTGCAGCACGTCATCGTCAGGGACCTGCAGACGGCACGCAGCGCCTTCTTCCTGGTCAATGAC |

### Table 2-7

**Table 2-7**

| |
|---|
| TGGCTTTCGGTGGAGACGGAGGCCAACGGGGGCCTGGTGGAGAAGGAGGTGCTGGCCGCGAGCGACGCAGCCCTTTTGCGCTTCC GGCGCCTGCTGGTGGCTGAGCTGCAGCGTGGCTTCTTTGACAAGCACATCTGGCTCTCCATATGGGACCGGCCGCCTCGTAGCCG TTTCACTCGCATCCAGAGGGCCACCTGCTGCGTTCTCCTCATCTGCCTCTTCCTGGGCGCCAACGCCGTGTGGTACGGGGCTGTT GGCGACTCTGCCTACAGCACGGGGCATGTGTCCAGGCTGAGCCCGCTGAGCGTCGACACAGTCGCTGTTGGCCTGGTGTCCAGCG TGGTTGTCTATCCCGTCTACCTGGCCATCCTTTTTCTCTTCCGGATGTCCCGGAGCAAGGTGGCTGGGAGCCCGAGCCCCACACC TGCCGGGCAGCAGGTGCTGGACATCGACAGCTGCCTGGACTCGTCCGTGCTGGACAGCTCCTTCCTCACGTTCTCAGGCCTCCAC GCTGAGCAGGCCTTTGTTGGACAGATGAAGAGTGACTTGTTTCTGGATGATTCTAAGAGTCTGGTGTGCTGGCCCTCCGGCGAGG GAACGCTCAGTTGGCCGGACCTGCTCAGTGACCCGTCCATTGTGGGTAGCAATCTGCGGCAGCTGGCACGGGGCCAGGCGGGCCA TGGGCTGGGCCCAGAGGAGGACGGCTTCTCCCTGGCCAGCCCCTACTCGCCTGCCAAATCCTTCTCAGCATCAGATGAAGACCTG ATCCAGCAGGTCCTTGCCGAGGGGGTCAGCAGCCCAGCCCCTACCCAAGACACCCACATGGAAACGGACCTGCTCAGCAGCCTGT CCAGCACTCCTGGGGAGAAGACAGAGACGCTGGCGCTGCAGAGGCTGGGGGAGCTGGGGCCACCCAGCCCAGGCCTGAACTGGGA ACAGCCCCAGGCAGCGAGGCTGTCCAGGACAGGACTGGTGGAGGGTCTGCGGAAGCGCCTGCTGCCGGCCTGGTGTGCCTCCCTG GCCCACGGGCTCAGCCTGCTCCTGGTGGCTGTGGCTGTGGCTGTCTCAGGGTGGGTGGGTGCGAGCTTCCCCCCGGGCGTGAGTG TTGCGTGGCTCCTGTCCAGCAGCGCCAGCTTCCTGGCCTCATTCCTCGGCTGGGAGCCACTGAAGGTCTTGCTGGAAGCCCTGTA CTTCTCACTGGTGGCCAAGCGGCTGCACCCGGATGAAGATGACACCCTGGTAGAGAGCCCGGCTGTGACGCCTGTGAGCGCACGT GTGCCCCGCGTACGGCCACCCCACGGCTTTGCACTCTTCCTGGCCAAGGAAGAAGCCCGCAAGGTCAAGAGGCTACATGGCATGC TGCGGAGCCTCCTGGTGTACATGCTTTTTCTGCTGGTGACCCTGCTGGCCAGCTATGGGGATGCCTCATGCCATGGGCACGCCTA CCGTCTGCAAAGCGCCATCAAGCAGGAGCTGCACAGCCGGGCCTTCCTGGCCATCACGCGGTCTGAGGAGCTCTGGCCATGGATG GCCCACGTGCTGCTGCCCTACGTCCACGGGAACCAGTCCAGCCCAGAGCTGGGGCCCCCACGGCTGCGGCAGGTGCGGCTGCAGG AAGCACTCTACCCAGACCCTCCCGGCCCCAGGGTCCACACGTGCTCGGCCGCAGGAGGCTTCAGCACCAGCGATTACGACGTTGG CTGGGAGAGTCCTCACAATGGCTCGGGGACGTGGGCCTATTCAGCGCCGGATCTGCTGGGGGCATGGTCCTGGGGCTCCTGTGCC GTGTATGACAGCGGGGGCTACGTGCAGGAGCTGGGCCTGAGCCTGGAGGAGAGCCGCGACCGGCTGCGCTTCCTGCAGCTGCACA ACTGGCTGGACAACAGGAGCCGCGCTGTGTTCCTGGAGCTCACGCGCTACAGCCCGGCCGTGGGGCTGCACGCCGCCGTCACGCT GCGCCTCGAGTTCCCGGCGGCCGGCCGCGCCCTGGCCGCCCTCAGCGTCCGCCCCTTTGCGCTGCGCCGCCTCAGCGCGGGCCTC TCGCTGCCTCTGCTCACCTCGGTGTGCCTGCTGCTGTTCGCCGTGCACTTCGCCGTGGCCGAGGCCCGTACTTGGCACAGGGAAG GGCGCTGGCGCGTGCTGCGGCTCGGAGCCTGGGCGCGGTGGCTGCTGGTGGCGCTGACGGCGGCCACGGCACTGGTACGCCTCGC CCAGCTGGGTGCCGCTGACCGCCAGTGGACCCGTTTCGTGCGCGGCCGCCCGCGCCGCTTCACTAGCTTCGACCAGGTGGCGCAG CTGAGCTCCGCAGCCCGTGGCCTGGCGGCCTCGCTGCTCTTCCTGCTTTTGGTCAAGGCTGCCCAGCAGCTACGCTTCGTGCGCC AGTGGTCCGTCTTTGGCAAGACATTATGCCGAGCTCTGCCAGAGCTCCTGGGGGTCACCTTGGGCCTGGTGGTGCTCGGGGTAGC CTACGCCCAGCTGGCCATCCTGCTCGTGTCTTCCTGTGTGGACTCCCTCTGGAGCGTGGCCCAGGCCCTGTTGGTGCTGTGCCCT GGGACTGGGCTCTCTACCCTGTGTCCTGCCGAGTCCTGGCACCTGTCACCCCTGCTGTGTGTGGGGCTCTGGGCACTGCGGCTGT GGGGCGCCCTACGGCTGGGGGCTGTTATTCTCCGCTGGCGCTACCACGCCTTGCGTGGAGAGCTGTACCGGCCGGCCTGGGAGCC CCAGGACTACGAGATGGTGGAGTTGTTCCTGCGCAGGCTGCGCCTCTGGATGGGCCTCAGCAAGGTCAAGGAGTTCCGCCACAAA GTCCGCTTTGAAGGGATGGAGCCGCTGCCCTCTCGCTCCTCCAGGGGCTCCAAGGTATCCCCGGATGTGCCCCCACCCAGCGCTG GCTCCGATGCCTCGCACCCCTCCACCTCCTCCAGCCAGCTGGATGGGCTGAGCGTGAGCCTGGGCCGGCTGGGGACAAGGTGTGA GCCTGAGCCCTCCCGCCTCCAAGCCGTGTTCGAGGCCCTGCTCACCCAGTTTGACCGACTCAACCAGGCCACAGAGGACGTCTAC CAGCTGGAGCAGCAGCTGCACAGCCTGCAAGGCCGCAGGAGCAGCCGGGCGCCCGCCGGATCTTCCCGTGGCCCATCCCCGGGCC TGCGGCCAGCACTGCCCAGCCGCCTTGCCCGGGCCAGTCGGGGTGTGGACCTGGCCACTGGCCCCAGCAGGACACCCCTTCGGGC CAAGAACAAGGTCCACCCCAGCAGCACTTAA |
| |
| SEQ ID NO: 7: CTT |

### Table 2-8

**Table 2-8**

| |
|---|
| MGAVILRWRYHALRGELYRPAWEPQDYEMVELFLRRLRLWMGLSKVKEFRHKVRFEGMEPLPSRSSRGSKVSPDVPPPSAGSDAS HPSTSSSQLDGLSVSLGRLGTRCEPEPSRLQAVFEALLTQFDRLNQATEDVYQLEQQLHSLQGRRSSRAPAGSSRGPSPGLRPAL PSRLARASRGVDLATGPSRTPLRAKNKVHPSST |
| |
| SEQ ID NO: 8: CTT |
| ATGGGGGCTGTTATTCTCCGCTGGCGCTACCACGCCTTGCGTGGAGAGCTGTACCGGCCGGCCTGGGAGCCCCAGGACTACGAGA TGGTGGAGTTGTTCCTGCGCAGGCTGCGCCTCTGGATGGGCCTCAGCAAGGTCAAGGAGTTCCGCCACAAAGTCCGCTTTGAAGG GATGGAGCCGCTGCCCTCTCGCTCCTCCAGGGGCTCCAAGGTATCCCCGGATGTGCCCCCACCCAGCGCTGGCTCCGATGCCTCG CACCCCTCCACCTCCTCCAGCCAGCTGGATGGGCTGAGCGTGAGCCTGGGCCGGCTGGGGACAAGGTGTGAGCCTGAGCCCTCCC GCCTCCAAGCCGTGTTCGAGGCCCTGCTCACCCAGTTTGACCGACTCAACCAGGCCACAGAGGACGTCTACCAGCTGGAGCAGCA GCTGCACAGCCTGCAAGGCCGCAGGAGCAGCCGGGCGCCCGCCGGATCTTCCCGTGGCCCATCCCCGGGCCTGCGGCCAGCACTG CCCAGCCGCCTTGCCCGGGCCAGTCGGGGTGTGGACCTGGCCACTGGCCCCAGCAGGACACCCCTTCGGGCCAAGAACAAGGTCC ACCCCAGCAGCACTTAA |
| |
| SEQ ID NO: 9: PC1 (Arg1672fs97X) |
| MPPAAPARLALALGLGLWLGALAGGPGRGCGPCEPPCLCGPAPGAACRVNCSGRGLRTLGPALRIPADATALDVSHNLLRALDVG LLANLSALAELDISNNKISTLEEGIFANLFNLSEINLSGNPFECDCGLAWLPRWAEEQQVRVVQPEAATCAGPGSLAGQPLLGIP LLDSGCGEEYVACLPDNSSGTVAAVSFSAAHEGLLQPEACSAFCFSTGQGLAALSEQGWCLCGAAQPSSASFACLSLCSGPPPPP APTCRGPTLLQHVFPASPGATLVGPHGPLASGQLAAFHIAAPLPVTATRWDFGDGSAEVDAAGPAASHRYVLPGRYHVTAVLALG AGSALLGTDVQVEAAPAALELVCPSSVQSDESLDLSIQNRGGSGLEAAYSIVALGEEPARAVHPLCPSDTEIFPGNGHCYRLVVE KAAWLQAQEQCQAWAGAALAMVDSPAVQRFLVSRVTRSLDVWIGFSTVQGVEVGPAPQGEAFSLESCQNWLPGEPHPATAEHCVR LGPTGWCNTDLCSAPHSYVCELQPGGPVQDAENLLVGAPSGDLQGPLTPLAQQDGLSAPHEPVEVMVFPGLRLSREAFLTTAEFG TQELRRPAQLRLQVYRLLSTAGTPENGSEPESRSPDNRTQLAPACMPGGRWCPGANICLPLDASCHPQACANGCTSGPGLPGAPY ALWREFLFSVPAGPPAQYSVTLHGQDVLMLPGDLVGLQHDAGPGALLHCSPAPGHPGPRAPYLSANASSWLPHLPAQLEGTWACP ACALRLLAATEQLTVLLGLRPNPGLRLPGRYEVRAEVGNGVSRHNLSCSFDVVSPVAGLRVIYPAPRDGRLYVPTNGSALVLQVD SGANATATARWPGGSVSARFENVCPALVATFVPGCPWETNDTLFSVVALPWLSEGEHVVDVVVENSASRANLSLRVTAEEPICGL RATPSPEARVLQGVLVRYSPVVEAGSDMVFRWTINDKQSLTFQNVVFNVIYQSAAVFKLSLTASNHVSNVTVNYNVTVERMNRMQ GLQVSTVPAVLSPNATLALTAGVLVDSAVEVAFLWTFGDGEQALHQFQPPYNESFPVPDPSVAQVLVEHNVMHTYAAPGEYLLTV LASNAFENLTQQVPVSVRASLPSVAVGVSDGVLVAGRPVTFYPHPLPSPGGVLYTWDFGDGSPVLTQSQPAANHTYASRGTYHVR LEVNNTVSGAAAQADVRVFEELRGLSVDMSLAVEQGAPVVVSAAVQTGDNITWTFDMGDGTVLSGPEATVEHVYLRAQNCTVTVG AASPAGHLARSLHVLVFVLEVLRVEPAACIPTQPDARLTAYVTGNPAHYLFDWTFGDGSSNTTVRGCPTVTHNFTRSGTFPLALV LSSRVNRAHYFTSICVEPEVGNVTLQPERQFVQLGDEAWLVACAWPPFPYRYTWDFGTEEAAPTRARGPEVTFIYRDPGSYLVTV TASNNISAANDSALVEVQEPVLVTSIKVNGSLGLELQQPYLFSAVGRGRPASYLWDLGDGGWLEGPEVTHAYNSTGDFTVRVAGW NEVSRSEAWLNVTVKRRVRGLVVNASRTVVPLNGSVSFSTSLEAGSDVRYSWVLCDRCTPIPGGPTISYTFRSVGTFNIIVTAEN EVGSAQDSIFVYVLQLIEGLQVVGGGRYFPTNHTVQLQAVVRDGTNVSYSWTAWRDGPGPGRQRQRLLAHRARGRHLPCAAAGHQ HAGQRLGRLHHGLRGACGVADGGRLPEPSCRQHKRHPQCRAGWWQWCRIHLVLGGGAELGDLRAIYHP |
| |
| SEQ ID NO: 10: PC1 (Arg1672fs97X) |
| ATGCCGCCCGCCGCGCCCGCCCGCCTGGCGCTGGCCCTGGGCCTGGGCCTGTGGCTCGGGGCGCTGGCGGGGGGCCCCGGGCGCG GCTGCGGGCCCTGCGAGCCCCCCTGCCTCTGCGGCCCAGCGCCCGGCGCCGCCTGCCGCGTCAACTGCTCGGGCCGCGGGCTGCG GACGCTCGGTCCCGCGCTGCGCATCCCCGCGGACGCCACAGCGCTAGACGTCTCCCACAACCTGCTCCGGGCGCTGGACGTTGGG |

### Table 2-9

**Table 2-9**

| |
|---|
| CTCCTGGCGAACCTCTCGGCGCTGGCAGAGCTGGATATAAGCAACAACAAGATTTCTACGTTAGAAGAAGGAATATTTGCTAATT TATTTAATTTAAGTGAAATAAACCTGAGTGGGAACCCGTTTGAGTGTGACTGTGGCCTGGCGTGGCTGCCGCGATGGGCGGAGGA GCAGCAGGTGCGGGTGGTGCAGCCCGAGGCAGCCACGTGTGCTGGGCCTGGCTCCCTGGCTGGCCAGCCTCTGCTTGGCATCCCC TTGCTGGACAGTGGCTGTGGTGAGGAGTATGTCGCCTGCCTCCCTGACAACAGCTCAGGCACCGTGGCAGCAGTGTCCTTTTCAG CTGCCCACGAAGGCCTGCTTCAGCCAGAGGCCTGCAGCGCCTTCTGCTTCTCCACCGGCCAGGGCCTCGCAGCCCTCTCGGAGCA GGGCTGGTGCCTGTGTGGGGCGGCCCAGCCCTCCAGTGCCTCCTTTGCCTGCCTGTCCCTCTGCTCCGGCCCCCCGCCACCTCCT GCCCCCACCTGTAGGGGCCCCACCCTCCTCCAGCACGTCTTCCCTGCCTCCCCAGGGGCCACCCTGGTGGGGCCCCACGGACCTC TGGCCTCTGGCCAGCTAGCAGCCTTCCACATCGCTGCCCCGCTCCCTGTCACTGCCACACGCTGGGACTTCGGAGACGGCTCCGC CGAGGTGGATGCCGCTGGGCCGGCTGCCTCGCATCGCTATGTGCTGCCTGGGCGCTATCACGTGACGGCCGTGCTGGCCCTGGGG GCCGGCTCAGCCCTGCTGGGGACAGACGTGCAGGTGGAAGCGGCACCTGCCGCCCTGGAGCTCGTGTGCCCGTCCTCGGTGCAGA GTGACGAGAGCCTCGACCTCAGCATCCAGAACCGCGGTGGTTCAGGCCTGGAGGCCGCCTACAGCATCGTGGCCCTGGGCGAGGA GCCGGCCCGAGCGGTGCACCCGCTCTGCCCCTCGGACACGGAGATCTTCCCTGGCAACGGGCACTGCTACCGCCTGGTGGTGGAG AAGGCGGCCTGGCTGCAGGCGCAGGAGCAGTGTCAGGCCTGGGCCGGGGCCGCCCTGGCAATGGTGGACAGTCCCGCCGTGCAGC GCTTCCTGGTCTCCCGGGTCACCAGGAGCCTAGACGTGTGGATCGGCTTCTCGACTGTGCAGGGGGTGGAGGTGGGCCCAGCGCC GCAGGGCGAGGCCTTCAGCCTGGAGAGCTGCCAGAACTGGCTGCCCGGGGAGCCACACCCAGCCACAGCCGAGCACTGCGTCCGG CTCGGGCCCACCGGGTGGTGTAACACCGACCTGTGCTCAGCGCCGCACAGCTACGTCTGCGAGCTGCAGCCCGGAGGCCCAGTGC AGGATGCCGAGAACCTCCTCGTGGGAGCGCCCAGTGGGGACCTGCAGGGACCCCTGACGCCTCTGGCACAGCAGGACGGCCTCTC AGCCCCGCACGAGCCCGTGGAGGTCATGGTATTCCCGGGCCTGCGTCTGAGCCGTGAAGCCTTCCTCACCACGGCCGAATTTGGG ACCCAGGAGCTCCGGCGGCCCGCCCAGCTGCGGCTGCAGGTGTACCGGCTCCTCAGCACAGCAGGGACCCCGGAGAACGGCAGCG AGCCTGAGAGCAGGTCCCCGGACAACAGGACCCAGCTGGCCCCCGCGTGCATGCCAGGGGGACGCTGGTGCCCTGGAGCCAACAT CTGCTTGCCGCTGGACGCCTCCTGCCACCCCCAGGCCTGCGCCAATGGCTGCACGTCAGGGCCAGGGCTACCCGGGGCCCCCTAT GCGCTATGGAGAGAGTTCCTCTTCTCCGTTCCCGCGGGGCCCCCCGCGCAGTACTCGGTCACCCTCCACGGCCAGGATGTCCTCA TGCTCCCTGGTGACCTCGTTGGCTTGCAGCACGACGCTGGCCCTGGCGCCCTCCTGCACTGCTCGCCGGCTCCCGGCCACCCTGG TCCCCGGGCCCCGTACCTCTCCGCCAACGCCTCGTCATGGCTGCCCCACTTGCCAGCCCAGCTGGAGGGCACTTGGGCCTGCCCT GCCTGTGCCCTGCGGCTGCTTGCAGCCACGGAACAGCTCACCGTGCTGCTGGGCTTGAGGCCCAACCCTGGACTGCGGCTGCCTG GGCGCTATGAGGTCCGGGCAGAGGTGGGCAATGGCGTGTCCAGGCACAACCTCTCCTGCAGCTTTGACGTGGTCTCCCCAGTGGC TGGGCTGCGGGTCATCTACCCTGCCCCCCGCGACGGCCGCCTCTACGTGCCCACCAACGGCTCAGCCTTGGTGCTCCAGGTGGAC TCTGGTGCCAACGCCACGGCCACGGCTCGCTGGCCTGGGGGCAGTGTCAGCGCCCGCTTTGAGAATGTCTGCCCTGCCCTGGTGG CCACCTTCGTGCCCGGCTGCCCCTGGGAGACCAACGATACCCTGTTCTCAGTGGTAGCACTGCCGTGGCTCAGTGAGGGGGAGCA CGTGGTGGACGTGGTGGTGGAAAACAGCGCCAGCCGGGCCAACCTCAGCCTGCGGGTGACGGCGGAGGAGCCCATCTGTGGCCTC CGCGCCACGCCCAGCCCCGAGGCCCGTGTACTGCAGGGAGTCCTAGTGAGGTACAGCCCCGTGGTGGAGGCCGGCTCGGACATGG TCTTCCGGTGGACCATCAACGACAAGCAGTCCCTGACCTTCCAGAACGTGGTCTTCAATGTCATTTATCAGAGCGCGGCGGTCTT CAAGCTCTCACTGACGGCCTCCAACCACGTGAGCAACGTCACCGTGAACTACAACGTAACCGTGGAGCGGATGAACAGGATGCAG GGTCTGCAGGTCTCCACAGTGCCGGCCGTGCTGTCCCCCAATGCCACGCTAGCACTGACGGCGGGCGTGCTGGTGGACTCGGCCG TGGAGGTGGCCTTCCTGTGGACCTTTGGGGATGGGGAGCAGGCCCTCCACCAGTTCCAGCCTCCGTACAACGAGTCCTTCCCGGT TCCAGACCCCTCGGTGGCCCAGGTGCTGGTGGAGCACAATGTCATGCACACCTACGCTGCCCCAGGTGAGTACCTCCTGACCGTG CTGGCATCTAATGCCTTCGAGAACCTGACGCAGCAGGTGCCTGTGAGCGTGCGCGCCTCCCTGCCCTCCGTGGCTGTGGGTGTGA GTGACGGCGTCCTGGTGGCCGGCCGGCCCGTCACCTTCTACCCGCACCCGCTGCCCTCGCCTGGGGGTGTTCTTTACACGTGGGA CTTCGGGGACGGCTCCCCTGTCCTGACCCAGAGCCAGCCGGCTGCCAACCACACCTATGCCTCGAGGGGCACCTACCACGTGCGC CTGGAGGTCAACAACACGGTGAGCGGTGCGGCGGCCCAGGCGGATGTGCGCGTCTTTGAGGAGCTCCGCGGACTCAGCGTGGACA TGAGCCTGGCCGTGGAGCAGGGCGCCCCCGTGGTGGTCAGCGCCGCGGTGCAGACGGGCGACAACATCACGTGGACCTTCGACAT |

### Table 2-10

**Table 2-10**

| |
|---|
| GGGGGACGGCACCGTGCTGTCGGGCCCGGAGGCAACAGTGGAGCATGTGTACCTGCGGGCACAGAACTGCACAGTGACCGTGGGT GCGGCCAGCCCCGCCGGCCACCTGGCCCGGAGCCTGCACGTGCTGGTCTTCGTCCTGGAGGTGCTGCGCGTTGAACCCGCCGCCT GCATCCCCACGCAGCCTGACGCGCGGCTCACGGCCTACGTCACCGGGAACCCGGCCCACTACCTCTTCGACTGGACCTTCGGGGA TGGCTCCTCCAACACGACCGTGCGGGGGTGCCCGACGGTGACACACAACTTCACGCGGAGCGGCACGTTCCCCCTGGCGCTGGTG CTGTCCAGCCGCGTGAACAGGGCGCATTACTTCACCAGCATCTGCGTGGAGCCAGAGGTGGGCAACGTCACCCTGCAGCCAGAGA GGCAGTTTGTGCAGCTCGGGGACGAGGCCTGGCTGGTGGCATGTGCCTGGCCCCCGTTCCCCTACCGCTACACCTGGGACTTTGG CACCGAGGAAGCCGCCCCCACCCGTGCCAGGGGCCCTGAGGTGACGTTCATCTACCGAGACCCAGGCTCCTATCTTGTGACAGTC ACCGCGTCCAACAACATCTCTGCTGCCAATGACTCAGCCCTGGTGGAGGTGCAGGAGCCCGTGCTGGTCACCAGCATCAAGGTCA ATGGCTCCCTTGGGCTGGAGCTGCAGCAGCCGTACCTGTTCTCTGCTGTGGGCCGTGGGCGCCCCGCCAGCTACCTGTGGGATCT GGGGGACGGTGGGTGGCTCGAGGGTCCGGAGGTCACCCACGCTTACAACAGCACAGGTGACTTCACCGTTAGGGTGGCCGGCTGG AATGAGGTGAGCCGCAGCGAGGCCTGGCTCAATGTGACGGTGAAGCGGCGCGTGCGGGGGCTCGTCGTCAATGCAAGCCGCACGG TGGTGCCCCTGAATGGGAGCGTGAGCTTCAGCACGTCGCTGGAGGCCGGCAGTGATGTGCGCTATTCCTGGGTGCTCTGTGACCG CTGCACGCCCATCCCTGGGGGTCCTACCATCTCTTACACCTTCCGCTCCGTGGGCACCTTCAATATCATCGTCACGGCTGAGAAC GAGGTGGGCTCCGCCCAGGACAGCATCTTCGTCTATGTCCTGCAGCTCATAGAGGGGCTGCAGGTGGTGGGCGGTGGCCGCTACT TCCCCACCAACCACACGGTACAGCTGCAGGCCGTGGTTAGGGATGGCACCAACGTCTCCTACAGCTGGACTGCCTGGAGGGACGG GCCCGGCCCTGGCCGGCAGCGGCAAAGGCTTCTCGCTCACCGTGCTCGAGGCCGGCACCTACCATGTGCAGCTGCGGGCCACCAA CATGCTGGGCAGCGCCTGGGCCGACTGCACCATGGACTTCGTGGAGCCTGTGGGGTGGCTGATGGTGGCCGCCTCCCCGAACCCA GCTGCCGTCAACACAAGCGTCACCCTCAGTGCCGAGCTGGCTGGTGGCAGTGGTGTCGTATACACTTGGTCCTTGGAGGAGGGGC TGAGCTGGGAGACCTCCGAGCCATTTACCACCCATAGCTTCCCCACACCCGGCCTGCACTTGGTCACCATGACGGCAGGGAACCC GCTGGGCTCAGCCAACGCCACCGTGGAAGTGGATGTGCAGGTGCCTGTGAGTGGCCTCAGCATCAGGGCCAGCGAGCCCGGAGGC AGCTTCGTGGCGGCCGGGTCCTCTGTGCCCTTTTGGGGGCAGCTGGCCACGGGCACCAATGTGAGCTGGTGCTGGGCTGTGCCCG GCGGCAGCAGCAAGCGTGGCCCTCATGTCACCATGGTCTTCCCGGATGCTGGCACCTTCTCCATCCGGCTCAATGCCTCCAACGC AGTCAGCTGGGTCTCAGCCACGTACAACCTCACGGCGGAGGAGCCCATCGTGGGCCTGGTGCTGTGGGCCAGCAGCAAGGTGGTG GCGCCCGGGCAGCTGGTCCATTTTCAGATCCTGCTGGCTGCCGGCTCAGCTGTCACCTTCCGCCTGCAGGTCGGCGGGGCCAACC CCGAGGTGCTCCCCGGGCCCCGTTTCTCCCACAGCTTCCCCCGCGTCGGAGACCACGTGGTGAGCGTGCGGGGCAAAAACCACGT GAGCTGGGCCCAGGCGCAGGTGCGCATCGTGGTGCTGGAGGCCGTGAGTGGGCTGCAGGTGCCCAACTGCTGCGAGCCTGGCATC GCCACGGGCACTGAGAGGAACTTCACAGCCCGCGTGCAGCGCGGCTCTCGGGTCGCCTACGCCTGGTACTTCTCGCTGCAGAAGG TCCAGGGCGACTCGCTGGTCATCCTGTCGGGCCGCGACGTCACCTACACGCCCGTGGCCGCGGGGCTGTTGGAGATCCAGGTGCG CGCCTTCAACGCCCTGGGCAGTGAGAACCGCACGCTGGTGCTGGAGGTTCAGGACGCCGTCCAGTATGTGGCCCTGCAGAGCGGC CCCTGCTTCACCAACCGCTCGGCGCAGTTTGAGGCCGCCACCAGCCCCAGCCCCCGGCGTGTGGCCTACCACTGGGACTTTGGGG ATGGGTCGCCAGGGCAGGACACAGATGAGCCCAGGGCCGAGCACTCCTACCTGAGGCCTGGGGACTACCGCGTGCAGGTGAACGC CTCCAACCTGGTGAGCTTCTTCGTGGCGCAGGCCACGGTGACCGTCCAGGTGCTGGCCTGCCGGGAGCCGGAGGTGGACGTGGTC CTGCCCCTGCAGGTGCTGATGCGGCGATCACAGCGCAACTACTTGGAGGCCCACGTTGACCTGCGCGACTGCGTCACCTACCAGA CTGAGTACCGCTGGGAGGTGTATCGCACCGCCAGCTGCCAGCGGCCGGGGCGCCCAGCGCGTGTGGCCCTGCCCGGCGTGGACGT GAGCCGGCCTCGGCTGGTGCTGCCGCGGCTGGCGCTGCCTGTGGGGCACTACTGCTTTGTGTTTGTCGTGTCATTTGGGGACACG CCACTGACACAGAGCATCCAGGCCAATGTGACGGTGGCCCCCGAGCGCCTGGTGCCCATCATTGAGGGTGGCTCATACCGCGTGT GGTCAGACACACGGGACCTGGTGCTGGATGGGAGCGAGTCCTACGACCCCAACCTGGAGGACGGCGACCAGACGCCGCTCAGTTT CCACTGGGCCTGTGTGGCTTCGACACAGAGGGAGGCTGGCGGGTGTGCGCTGAACTTTGGGCCCCGCGGGAGCAGCACGGTCACC ATTCCACGGGAGCGGCTGGCGGCTGGCGTGGAGTACACCTTCAGCCTGACCGTGTGGAAGGCCGGCCGCAAGGAGGAGGCCACCA ACCAGACGGTGCTGATCCGGAGTGGCCGGGTGCCCATTGTGTCCTTGGAGTGTGTGTCCTGCAAGGCACAGGCCGTGTACGAAGT GAGCCGCAGCTCCTACGTGTACTTGGAGGGCCGCTGCCTCAATTGCAGCAGCGGCTCCAAGCGAGGGCGGTGGGCTGCACGTACG |

### Table 2-11

**Table 2-11**

| |
|---|
| TTCAGCAACAAGACGCTGGTGCTGGATGAGACCACCACATCCACGGGCAGTGCAGGCATGCGACTGGTGCTGCGGCGGGGCGTGC TGCGGGACGGCGAGGGATACACCTTCACGCTCACGGTGCTGGGCCGCTCTGGCGAGGAGGAGGGCTGCGCCTCCATCCGCCTGTC CCCCAACCGCCCGCCGCTGGGGGGCTCTTGCCGCCTCTTCCCACTGGGCGCTGTGCACGCCCTCACCACCAAGGTGCACTTCGAA TGCACGGGCTGGCATGACGCGGAGGATGCTGGCGCCCCGCTGGTGTACGCCCTGCTGCTGCGGCGCTGTCGCCAGGGCCACTGCG AGGAGTTCTGTGTCTACAAGGGCAGCCTCTCCAGCTACGGAGCCGTGCTGCCCCCGGGTTTCAGGCCACACTTCGAGGTGGGCCT GGCCGTGGTGGTGCAGGACCAGCTGGGAGCCGCTGTGGTCGCCCTCAACAGGTCTTTGGCCATCACCCTCCCAGAGCCCAACGGC AGCGCAACGGGGCTCACAGTCTGGCTGCACGGGCTCACCGCTAGTGTGCTCCCAGGGCTGCTGCGGCAGGCCGATCCCCAGCACG TCATCGAGTACTCGTTGGCCCTGGTCACCGTGCTGAACGAGTACGAGCGGGCCCTGGACGTGGCGGCAGAGCCCAAGCACGAGCG GCAGCACCGAGCCCAGATACGCAAGAACATCACGGAGACTCTGGTGTCCCTGAGGGTCCACACTGTGGATGACATCCAGCAGATC GCTGCTGCGCTGGCCCAGTGCATGGGGCCCAGCAGGGAGCTCGTATGCCGCTCGTGCCTGAAGCAGACGCTGCACAAGCTGGAGG CCATGATGCTCATCCTGCAGGCAGAGACCACCGCGGGCACCGTGACGCCCACCGCCATCGGAGACAGCATCCTCAACATCACAGG AGACCTCATCCACCTGGCCAGCTCGGACGTGCGGGCACCACAGCCCTCAGAGCTGGGAGCCGAGTCACCATCTCGGATGGTGGCG TCCCAGGCCTACAACCTGACCTCTGCCCTCATGCGCATCCTCATGCGCTCCCGCGTGCTCAACGAGGAGCCCCTGACGCTGGCGG GCGAGGAGATCGTGGCCCAGGGCAAGCGCTCGGACCCGCGGAGCCTGCTGTGCTATGGCGGCGCCCCAGGGCCTGGCTGCCACTT CTCCATCCCCGAGGCTTTCAGCGGGGCCCTGGCCAACCTCAGTGACGTGGTGCAGCTCATCTTTCTGGTGGACTCCAATCCCTTT CCCTTTGGCTATATCAGCAACTACACCGTCTCCACCAAGGTGGCCTCGATGGCATTCCAGACACAGGCCGGCGCCCAGATCCCCA TCGAGCGGCTGGCCTCAGAGCGCGCCATCACCGTGAAGGTGCCCAACAACTCGGACTGGGCTGCCCGGGGCCACCGCAGCTCCGC CAACTCCGCCAACTCCGTTGTGGTCCAGCCCCAGGCCTCCGTCGGTGCTGTGGTCACCCTGGACAGCAGCAACCCTGCGGCCGGG CTGCATCTGCAGCTCAACTATACGCTGCTGGACGGCCACTACCTGTCTGAGGAACCTGAGCCCTACCTGGCAGTCTACCTACACT CGGAGCCCCGGCCCAATGAGCACAACTGCTCGGCTAGCAGGAGGATCCGCCCAGAGTCACTCCAGGGTGCTGACCACCGGCCCTA CACCTTCTTCATTTCCCCGGGGAGCAGAGACCCAGCGGGGAGTTACCATCTGAACCTCTCCAGCCACTTCCGCTGGTCGGCGCTG CAGGTGTCCGTGGGCCTGTACACGTCCCTGTGCCAGTACTTCAGCGAGGAGGACATGGTGTGGCGGACAGAGGGGCTGCTGCCCC TGGAGGAGACCTCGCCCCGCCAGGCCGTCTGCCTCACCCGCCACCTCACCGCCTTCGGCGCCAGCCTCTTCGTGCCCCCAAGCCA TGTCCGCTTTGTGTTTCCTGAGCCGACAGCGGATGTAAACTACATCGTCATGCTGACATGTGCTGTGTGCCTGGTGACCTACATG GTCATGGCCGCCATCCTGCACAAGCTGGACCAGTTGGATGCCAGCCGGGGCCGCGCCATCCCTTTCTGTGGGCAGCGGGGCCGCT TCAAGTACGAGATCCTCGTCAAGACAGGCTGGGGCCGGGGCTCAGGTACCACGGCCCACGTGGGCATCATGCTGTATGGGGTGGA CAGCCGGAGCGGCCACCGGCACCTGGACGGCGACAGAGCCTTCCACCGCAACAGCCTGGACATCTTCCGGATCGCCACCCCGCAC AGCCTGGGTAGCGTGTGGAAGATCCGAGTGTGGCACGACAACAAAGGGCTCAGCCCTGCCTGGTTCCTGCAGCACGTCATCGTCA GGGACCTGCAGACGGCACGCAGCGCCTTCTTCCTGGTCAATGACTGGCTTTCGGTGGAGACGGAGGCCAACGGGGGCCTGGTGGA GAAGGAGGTGCTGGCCGCGAGCGACGCAGCCCTTTTGCGCTTCCGGCGCCTGCTGGTGGCTGAGCTGCAGCGTGGCTTCTTTGAC AAGCACATCTGGCTCTCCATATGGGACCGGCCGCCTCGTAGCCGTTTCACTCGCATCCAGAGGGCCACCTGCTGCGTTCTCCTCA TCTGCCTCTTCCTGGGCGCCAACGCCGTGTGGTACGGGGCTGTTGGCGACTCTGCCTACAGCACGGGGCATGTGTCCAGGCTGAG CCCGCTGAGCGTCGACACAGTCGCTGTTGGCCTGGTGTCCAGCGTGGTTGTCTATCCCGTCTACCTGGCCATCCTTTTTCTCTTC CGGATGTCCCGGAGCAAGGTGGCTGGGAGCCCGAGCCCCACACCTGCCGGGCAGCAGGTGCTGGACATCGACAGCTGCCTGGACT CGTCCGTGCTGGACAGCTCCTTCCTCACGTTCTCAGGCCTCCACGCTGAGCAGGCCTTTGTTGGACAGATGAAGAGTGACTTGTT TCTGGATGATTCTAAGAGTCTGGTGTGCTGGCCCTCCGGCGAGGGAACGCTCAGTTGGCCGGACCTGCTCAGTGACCCGTCCATT GTGGGTAGCAATCTGCGGCAGCTGGCACGGGGCCAGGCGGGCCATGGGCTGGGCCCAGAGGAGGACGGCTTCTCCCTGGCCAGCC CCTACTCGCCTGCCAAATCCTTCTCAGCATCAGATGAAGACCTGATCCAGCAGGTCCTTGCCGAGGGGGTCAGCAGCCCAGCCCC TACCCAAGACACCCACATGGAAACGGACCTGCTCAGCAGCCTGTCCAGCACTCCTGGGGAGAAGACAGAGACGCTGGCGCTGCAG AGGCTGGGGGAGCTGGGGCCACCCAGCCCAGGCCTGAACTGGGAACAGCCCCAGGCAGCGAGGCTGTCCAGGACAGGACTGGTGG AGGGTCTGCGGAAGCGCCTGCTGCCGGCCTGGTGTGCCTCCCTGGCCCACGGGCTCAGCCTGCTCCTGGTGGCTGTGGCTGTGGC |

### Table 2-12

**Table 2-12**

| |
|---|
| TGTCTCAGGGTGGGTGGGTGCGAGCTTCCCCCCGGGCGTGAGTGTTGCGTGGCTCCTGTCCAGCAGCGCCAGCTTCCTGGCCTCA TTCCTCGGCTGGGAGCCACTGAAGGTCTTGCTGGAAGCCCTGTACTTCTCACTGGTGGCCAAGCGGCTGCACCCGGATGAAGATG ACACCCTGGTAGAGAGCCCGGCTGTGACGCCTGTGAGCGCACGTGTGCCCCGCGTACGGCCACCCCACGGCTTTGCACTCTTCCT GGCCAAGGAAGAAGCCCGCAAGGTCAAGAGGCTACATGGCATGCTGCGGAGCCTCCTGGTGTACATGCTTTTTCTGCTGGTGACC CTGCTGGCCAGCTATGGGGATGCCTCATGCCATGGGCACGCCTACCGTCTGCAAAGCGCCATCAAGCAGGAGCTGCACAGCCGGG CCTTCCTGGCCATCACGCGGTCTGAGGAGCTCTGGCCATGGATGGCCCACGTGCTGCTGCCCTACGTCCACGGGAACCAGTCCAG CCCAGAGCTGGGGCCCCCACGGCTGCGGCAGGTGCGGCTGCAGGAAGCACTCTACCCAGACCCTCCCGGCCCCAGGGTCCACACG TGCTCGGCCGCAGGAGGCTTCAGCACCAGCGATTACGACGTTGGCTGGGAGAGTCCTCACAATGGCTCGGGGACGTGGGCCTATT CAGCGCCGGATCTGCTGGGGGCATGGTCCTGGGGCTCCTGTGCCGTGTATGACAGCGGGGGCTACGTGCAGGAGCTGGGCCTGAG CCTGGAGGAGAGCCGCGACCGGCTGCGCTTCCTGCAGCTGCACAACTGGCTGGACAACAGGAGCCGCGCTGTGTTCCTGGAGCTC ACGCGCTACAGCCCGGCCGTGGGGCTGCACGCCGCCGTCACGCTGCGCCTCGAGTTCCCGGCGGCCGGCCGCGCCCTGGCCGCCC TCAGCGTCCGCCCCTTTGCGCTGCGCCGCCTCAGCGCGGGCCTCTCGCTGCCTCTGCTCACCTCGGTGTGCCTGCTGCTGTTCGC CGTGCACTTCGCCGTGGCCGAGGCCCGTACTTGGCACAGGGAAGGGCGCTGGCGCGTGCTGCGGCTCGGAGCCTGGGCGCGGTGG CTGCTGGTGGCGCTGACGGCGGCCACGGCACTGGTACGCCTCGCCCAGCTGGGTGCCGCTGACCGCCAGTGGACCCGTTTCGTGC GCGGCCGCCCGCGCCGCTTCACTAGCTTCGACCAGGTGGCGCAGCTGAGCTCCGCAGCCCGTGGCCTGGCGGCCTCGCTGCTCTT CCTGCTTTTGGTCAAGGCTGCCCAGCAGCTACGCTTCGTGCGCCAGTGGTCCGTCTTTGGCAAGACATTATGCCGAGCTCTGCCA GAGCTCCTGGGGGTCACCTTGGGCCTGGTGGTGCTCGGGGTAGCCTACGCCCAGCTGGCCATCCTGCTCGTGTCTTCCTGTGTGG ACTCCCTCTGGAGCGTGGCCCAGGCCCTGTTGGTGCTGTGCCCTGGGACTGGGCTCTCTACCCTGTGTCCTGCCGAGTCCTGGCA CCTGTCACCCCTGCTGTGTGTGGGGCTCTGGGCACTGCGGCTGTGGGGCGCCCTACGGCTGGGGGCTGTTATTCTCCGCTGGCGC TACCACGCCTTGCGTGGAGAGCTGTACCGGCCGGCCTGGGAGCCCCAGGACTACGAGATGGTGGAGTTGTTCCTGCGCAGGCTGC GCCTCTGGATGGGCCTCAGCAAGGTCAAGGAGTTCCGCCACAAAGTCCGCTTTGAAGGGATGGAGCCGCTGCCCTCTCGCTCCTC CAGGGGCTCCAAGGTATCCCCGGATGTGCCCCCACCCAGCGCTGGCTCCGATGCCTCGCACCCCTCCACCTCCTCCAGCCAGCTG GATGGGCTGAGCGTGAGCCTGGGCCGGCTGGGGACAAGGTGTGAGCCTGAGCCCTCCCGCCTCCAAGCCGTGTTCGAGGCCCTGC TCACCCAGTTTGACCGACTCAACCAGGCCACAGAGGACGTCTACCAGCTGGAGCAGCAGCTGCACAGCCTGCAAGGCCGCAGGAG CAGCCGGGCGCCCGCCGGATCTTCCCGTGGCCCATCCCCGGGCCTGCGGCCAGCACTGCCCAGCCGCCTTGCCCGGGCCAGTCGG GGTGTGGACCTGGCCACTGGCCCCAGCAGGACACCCCTTCGGGCCAAGAACAAGGTCCACCCCAGCAGCACTTAA |
| |
| SEQ ID NO: 11: stalk-TM(11) |
| MTAFGASLFVPPSHVRFVFPEPTADVNYIVMLTCAVCLVTYMVMAAILHKLDQLDASRGRAIPFCGQRGRFKYEILVKTGWGRGS GTTAHVGIMLYGVDSRSGHRHLDGDRAFHRNSLDIFRIATPHSLGSVWKIRVWHDNKGLSPAWFLQHVIVRDLQTARSAFFLVND WLSVETEANGGLVEKEVLAASDAALLRFRRLLVAELQRGFFDKHIWLSIWDRPPRSRFTRIQRATCCVLLICLFLGANAVWYGAV GDSAYSTGHVSRLSPLSVDTVAVGLVSSVVVYPVYLAILFLFRMSRSKVAGSPSPTPAGQQVLDIDSCLDSSVLDSSFLTFSGLH AEQAFVGQMKSDLFLDDSKSLVCWPSGEGTLSWPDLLSDPSIVGSNLRQLARGQAGHGLGPEEDGFSLASPYSPAKSFSASDEDL IQQVLAEGVSSPAPTQDTHMETDLLSSLSSTPGEKTETLALQRLGELGPPSPGLNWEQPQAARLSRTGLVEGLRKRLLPAWCASL AHGLSLLLVAVAVAVSGWVGASFPPGVSVAWLLSSSASFLASFLGWEPLKVLLEALYFSLVAKRLHPDEDDTLVESPAVTPVSAR VPRVRPPHGFALFLAKEEARKVKRLHGMLRSLLVYMLFLLVTLLASYGDASCHGHAYRLQSAIKQELHSRAFLAITRSEELWPWM AHVLLPYVHGNQSSPELGPPRLRQVRLQEALYPDPPGPRVHTCSAAGGFSTSDYDVGWESPHNGSGTWAYSAPDLLGAWSWGSCA VYDSGGYVQELGLSLEESRDRLRFLQLHNWLDNRSRAVFLELTRYSPAVGLHAAVTLRLEFPAAGRALAALSVRPFALRRLSAGL SLPLLTSVCLLLFAVHFAVAEARTWHREGRWRVLRLGAWARWLLVALTAATALVRLAQLGAADRQWTRFVRGRPRRFTSFDQVAQ LSSAARGLAASLLFLLLVKAAQQLRFVRQWSVFGKTLCRALPELLGVTLGLVVLGVAYAQLAILLVSSCVDSLWSVAQALLVLCP GTGLSTLCPA |

### Table 2-13

**Table 2-13**

| |
|---|
| |
| SEQ ID NO: 12: stalk-TM(11) |
| ATGACCGCCTTCGGCGCCAGCCTCTTCGTGCCCCCAAGCCATGTCCGCTTTGTGTTTCCTGAGCCGACAGCGGATGTAAACTACA TCGTCATGCTGACATGTGCTGTGTGCCTGGTGACCTACATGGTCATGGCCGCCATCCTGCACAAGCTGGACCAGTTGGATGCCAG CCGGGGCCGCGCCATCCCTTTCTGTGGGCAGCGGGGCCGCTTCAAGTACGAGATCCTCGTCAAGACAGGCTGGGGCCGGGGCTCA GGTACCACGGCCCACGTGGGCATCATGCTGTATGGGGTGGACAGCCGGAGCGGCCACCGGCACCTGGACGGCGACAGAGCCTTCC ACCGCAACAGCCTGGACATCTTCCGGATCGCCACCCCGCACAGCCTGGGTAGCGTGTGGAAGATCCGAGTGTGGCACGACAACAA AGGGCTCAGCCCTGCCTGGTTCCTGCAGCACGTCATCGTCAGGGACCTGCAGACGGCACGCAGCGCCTTCTTCCTGGTCAATGAC TGGCTTTCGGTGGAGACGGAGGCCAACGGGGGCCTGGTGGAGAAGGAGGTGCTGGCCGCGAGCGACGCAGCCCTTTTGCGCTTCC GGCGCCTGCTGGTGGCTGAGCTGCAGCGTGGCTTCTTTGACAAGCACATCTGGCTCTCCATATGGGACCGGCCGCCTCGTAGCCG TTTCACTCGCATCCAGAGGGCCACCTGCTGCGTTCTCCTCATCTGCCTCTTCCTGGGCGCCAACGCCGTGTGGTACGGGGCTGTT GGCGACTCTGCCTACAGCACGGGGCATGTGTCCAGGCTGAGCCCGCTGAGCGTCGACACAGTCGCTGTTGGCCTGGTGTCCAGCG TGGTTGTCTATCCCGTCTACCTGGCCATCCTTTTTCTCTTCCGGATGTCCCGGAGCAAGGTGGCTGGGAGCCCGAGCCCCACACC TGCCGGGCAGCAGGTGCTGGACATCGACAGCTGCCTGGACTCGTCCGTGCTGGACAGCTCCTTCCTCACGTTCTCAGGCCTCCAC GCTGAGCAGGCCTTTGTTGGACAGATGAAGAGTGACTTGTTTCTGGATGATTCTAAGAGTCTGGTGTGCTGGCCCTCCGGCGAGG GAACGCTCAGTTGGCCGGACCTGCTCAGTGACCCGTCCATTGTGGGTAGCAATCTGCGGCAGCTGGCACGGGGCCAGGCGGGCCA TGGGCTGGGCCCAGAGGAGGACGGCTTCTCCCTGGCCAGCCCCTACTCGCCTGCCAAATCCTTCTCAGCATCAGATGAAGACCTG ATCCAGCAGGTCCTTGCCGAGGGGGTCAGCAGCCCAGCCCCTACCCAAGACACCCACATGGAAACGGACCTGCTCAGCAGCCTGT CCAGCACTCCTGGGGAGAAGACAGAGACGCTGGCGCTGCAGAGGCTGGGGGAGCTGGGGCCACCCAGCCCAGGCCTGAACTGGGA ACAGCCCCAGGCAGCGAGGCTGTCCAGGACAGGACTGGTGGAGGGTCTGCGGAAGCGCCTGCTGCCGGCCTGGTGTGCCTCCCTG GCCCACGGGCTCAGCCTGCTCCTGGTGGCTGTGGCTGTGGCTGTCTCAGGGTGGGTGGGTGCGAGCTTCCCCCCGGGCGTGAGTG TTGCGTGGCTCCTGTCCAGCAGCGCCAGCTTCCTGGCCTCATTCCTCGGCTGGGAGCCACTGAAGGTCTTGCTGGAAGCCCTGTA CTTCTCACTGGTGGCCAAGCGGCTGCACCCGGATGAAGATGACACCCTGGTAGAGAGCCCGGCTGTGACGCCTGTGAGCGCACGT GTGCCCCGCGTACGGCCACCCCACGGCTTTGCACTCTTCCTGGCCAAGGAAGAAGCCCGCAAGGTCAAGAGGCTACATGGCATGC TGCGGAGCCTCCTGGTGTACATGCTTTTTCTGCTGGTGACCCTGCTGGCCAGCTATGGGGATGCCTCATGCCATGGGCACGCCTA CCGTCTGCAAAGCGCCATCAAGCAGGAGCTGCACAGCCGGGCCTTCCTGGCCATCACGCGGTCTGAGGAGCTCTGGCCATGGATG GCCCACGTGCTGCTGCCCTACGTCCACGGGAACCAGTCCAGCCCAGAGCTGGGGCCCCCACGGCTGCGGCAGGTGCGGCTGCAGG AAGCACTCTACCCAGACCCTCCCGGCCCCAGGGTCCACACGTGCTCGGCCGCAGGAGGCTTCAGCACCAGCGATTACGACGTTGG CTGGGAGAGTCCTCACAATGGCTCGGGGACGTGGGCCTATTCAGCGCCGGATCTGCTGGGGGCATGGTCCTGGGGCTCCTGTGCC GTGTATGACAGCGGGGGCTACGTGCAGGAGCTGGGCCTGAGCCTGGAGGAGAGCCGCGACCGGCTGCGCTTCCTGCAGCTGCACA ACTGGCTGGACAACAGGAGCCGCGCTGTGTTCCTGGAGCTCACGCGCTACAGCCCGGCCGTGGGGCTGCACGCCGCCGTCACGCT GCGCCTCGAGTTCCCGGCGGCCGGCCGCGCCCTGGCCGCCCTCAGCGTCCGCCCCTTTGCGCTGCGCCGCCTCAGCGCGGGCCTC TCGCTGCCTCTGCTCACCTCGGTGTGCCTGCTGCTGTTCGCCGTGCACTTCGCCGTGGCCGAGGCCCGTACTTGGCACAGGGAAG GGCGCTGGCGCGTGCTGCGGCTCGGAGCCTGGGCGCGGTGGCTGCTGGTGGCGCTGACGGCGGCCACGGCACTGGTACGCCTCGC CCAGCTGGGTGCCGCTGACCGCCAGTGGACCCGTTTCGTGCGCGGCCGCCCGCGCCGCTTCACTAGCTTCGACCAGGTGGCGCAG CTGAGCTCCGCAGCCCGTGGCCTGGCGGCCTCGCTGCTCTTCCTGCTTTTGGTCAAGGCTGCCCAGCAGCTACGCTTCGTGCGCC AGTGGTCCGTCTTTGGCAAGACATTATGCCGAGCTCTGCCAGAGCTCCTGGGGGTCACCTTGGGCCTGGTGGTGCTCGGGGTAGC CTACGCCCAGCTGGCCATCCTGCTCGTGTCTTCCTGTGTGGACTCCCTCTGGAGCGTGGCCCAGGCCCTGTTGGTGCTGTGCCCT GGGACTGGGCTCTCTACCCTGTGTCCTGCCTAA |
| |
| SEQ ID NO: 13: TM(11) |

### Table 2-14

**Table 2-14**

| |
|---|
| MYIVMLTCAVCLVTYMVMAAILHKLDQLDASRGRAIPFCGQRGRFKYEILVKTGWGRGSGTTAHVGIMLYGVDSRSGHRHLDGDR AFHRNSLDIFRIATPHSLGSVWKIRVWHDNKGLSPAWFLQHVIVRDLQTARSAFFLVNDWLSVETEANGGLVEKEVLAASDAALL RFRRLLVAELQRGFFDKHIWLSIWDRPPRSRFTRIQRATCCVLLICLFLGANAVWYGAVGDSAYSTGHVSRLSPLSVDTVAVGLV SSVVVYPVYLAILFLFRMSRSKVAGSPSPTPAGQQVLDIDSCLDSSVLDSSFLTFSGLHAEQAFVGQMKSDLFLDDSKSLVCWPS GEGTLSWPDLLSDPSIVGSNLRQLARGQAGHGLGPEEDGFSLASPYSPAKSFSASDEDLIQQVLAEGVSSPAPTQDTHMETDLLS SLSSTPGEKTETLALQRLGELGPPSPGLNWEQPQAARLSRTGLVEGLRKRLLPAWCASLAHGLSLLLVAVAVAVSGWVGASFPPG VSVAWLLSSSASFLASFLGWEPLKVLLEALYFSLVAKRLHPDEDDTLVESPAVTPVSARVPRVRPPHGFALFLAKEEARKVKRLH GMLRSLLVYMLFLLVTLLASYGDASCHGHAYRLQSAIKQELHSRAFLAITRSEELWPWMAHVLLPYVHGNQSSPELGPPRLRQVR LQEALYPDPPGPRVHTCSAAGGFSTSDYDVGWESPHNGSGTWAYSAPDLLGAWSWGSCAVYDSGGYVQELGLSLEESRDRLRFLQ LHNWLDNRSRAVFLELTRYSPAVGLHAAVTLRLEFPAAGRALAALSVRPFALRRLSAGLSLPLLTSVCLLLFAVHFAVAEARTWH REGRWRVLRLGAWARWLLVALTAATALVRLAQLGAADRQWTRFVRGRPRRFTSFDQVAQLSSAARGLAASLLFLLLVKAAQQLRF VRQWSVFGKTLCRALPELLGVTLGLVVLGVAYAQLAILLVSSCVDSLWSVAQALLVLCPGTGLSTLCPA |
| |
| SEQ ID NO: 14: TM(11) |
| ATGTACATCGTCATGCTGACATGTGCTGTGTGCCTGGTGACCTACATGGTCATGGCCGCCATCCTGCACAAGCTGGACCAGTTGG ATGCCAGCCGGGGCCGCGCCATCCCTTTCTGTGGGCAGCGGGGCCGCTTCAAGTACGAGATCCTCGTCAAGACAGGCTGGGGCCG GGGCTCAGGTACCACGGCCCACGTGGGCATCATGCTGTATGGGGTGGACAGCCGGAGCGGCCACCGGCACCTGGACGGCGACAGA GCCTTCCACCGCAACAGCCTGGACATCTTCCGGATCGCCACCCCGCACAGCCTGGGTAGCGTGTGGAAGATCCGAGTGTGGCACG ACAACAAAGGGCTCAGCCCTGCCTGGTTCCTGCAGCACGTCATCGTCAGGGACCTGCAGACGGCACGCAGCGCCTTCTTCCTGGT CAATGACTGGCTTTCGGTGGAGACGGAGGCCAACGGGGGCCTGGTGGAGAAGGAGGTGCTGGCCGCGAGCGACGCAGCCCTTTTG CGCTTCCGGCGCCTGCTGGTGGCTGAGCTGCAGCGTGGCTTCTTTGACAAGCACATCTGGCTCTCCATATGGGACCGGCCGCCTC GTAGCCGTTTCACTCGCATCCAGAGGGCCACCTGCTGCGTTCTCCTCATCTGCCTCTTCCTGGGCGCCAACGCCGTGTGGTACGG GGCTGTTGGCGACTCTGCCTACAGCACGGGGCATGTGTCCAGGCTGAGCCCGCTGAGCGTCGACACAGTCGCTGTTGGCCTGGTG TCCAGCGTGGTTGTCTATCCCGTCTACCTGGCCATCCTTTTTCTCTTCCGGATGTCCCGGAGCAAGGTGGCTGGGAGCCCGAGCC CCACACCTGCCGGGCAGCAGGTGCTGGACATCGACAGCTGCCTGGACTCGTCCGTGCTGGACAGCTCCTTCCTCACGTTCTCAGG CCTCCACGCTGAGCAGGCCTTTGTTGGACAGATGAAGAGTGACTTGTTTCTGGATGATTCTAAGAGTCTGGTGTGCTGGCCCTCC GGCGAGGGAACGCTCAGTTGGCCGGACCTGCTCAGTGACCCGTCCATTGTGGGTAGCAATCTGCGGCAGCTGGCACGGGGCCAGG CGGGCCATGGGCTGGGCCCAGAGGAGGACGGCTTCTCCCTGGCCAGCCCCTACTCGCCTGCCAAATCCTTCTCAGCATCAGATGA AGACCTGATCCAGCAGGTCCTTGCCGAGGGGGTCAGCAGCCCAGCCCCTACCCAAGACACCCACATGGAAACGGACCTGCTCAGC AGCCTGTCCAGCACTCCTGGGGAGAAGACAGAGACGCTGGCGCTGCAGAGGCTGGGGGAGCTGGGGCCACCCAGCCCAGGCCTGA ACTGGGAACAGCCCCAGGCAGCGAGGCTGTCCAGGACAGGACTGGTGGAGGGTCTGCGGAAGCGCCTGCTGCCGGCCTGGTGTGC CTCCCTGGCCCACGGGCTCAGCCTGCTCCTGGTGGCTGTGGCTGTGGCTGTCTCAGGGTGGGTGGGTGCGAGCTTCCCCCCGGGC GTGAGTGTTGCGTGGCTCCTGTCCAGCAGCGCCAGCTTCCTGGCCTCATTCCTCGGCTGGGAGCCACTGAAGGTCTTGCTGGAAG CCCTGTACTTCTCACTGGTGGCCAAGCGGCTGCACCCGGATGAAGATGACACCCTGGTAGAGAGCCCGGCTGTGACGCCTGTGAG CGCACGTGTGCCCCGCGTACGGCCACCCCACGGCTTTGCACTCTTCCTGGCCAAGGAAGAAGCCCGCAAGGTCAAGAGGCTACAT GGCATGCTGCGGAGCCTCCTGGTGTACATGCTTTTTCTGCTGGTGACCCTGCTGGCCAGCTATGGGGATGCCTCATGCCATGGGC ACGCCTACCGTCTGCAAAGCGCCATCAAGCAGGAGCTGCACAGCCGGGCCTTCCTGGCCATCACGCGGTCTGAGGAGCTCTGGCC ATGGATGGCCCACGTGCTGCTGCCCTACGTCCACGGGAACCAGTCCAGCCCAGAGCTGGGGCCCCCACGGCTGCGGCAGGTGCGG CTGCAGGAAGCACTCTACCCAGACCCTCCCGGCCCCAGGGTCCACACGTGCTCGGCCGCAGGAGGCTTCAGCACCAGCGATTACG ACGTTGGCTGGGAGAGTCCTCACAATGGCTCGGGGACGTGGGCCTATTCAGCGCCGGATCTGCTGGGGGCATGGTCCTGGGGCTC CTGTGCCGTGTATGACAGCGGGGGCTACGTGCAGGAGCTGGGCCTGAGCCTGGAGGAGAGCCGCGACCGGCTGCGCTTCCTGCAG |

### Table 2-15

**Table 2-15**

| |
|---|
| CTGCACAACTGGCTGGACAACAGGAGCCGCGCTGTGTTCCTGGAGCTCACGCGCTACAGCCCGGCCGTGGGGCTGCACGCCGCCG TCACGCTGCGCCTCGAGTTCCCGGCGGCCGGCCGCGCCCTGGCCGCCCTCAGCGTCCGCCCCTTTGCGCTGCGCCGCCTCAGCGC GGGCCTCTCGCTGCCTCTGCTCACCTCGGTGTGCCTGCTGCTGTTCGCCGTGCACTTCGCCGTGGCCGAGGCCCGTACTTGGCAC AGGGAAGGGCGCTGGCGCGTGCTGCGGCTCGGAGCCTGGGCGCGGTGGCTGCTGGTGGCGCTGACGGCGGCCACGGCACTGGTAC GCCTCGCCCAGCTGGGTGCCGCTGACCGCCAGTGGACCCGTTTCGTGCGCGGCCGCCCGCGCCGCTTCACTAGCTTCGACCAGGT GGCGCAGCTGAGCTCCGCAGCCCGTGGCCTGGCGGCCTCGCTGCTCTTCCTGCTTTTGGTCAAGGCTGCCCAGCAGCTACGCTTC GTGCGCCAGTGGTCCGTCTTTGGCAAGACATTATGCCGAGCTCTGCCAGAGCTCCTGGGGGTCACCTTGGGCCTGGTGGTGCTCG GGGTAGCCTACGCCCAGCTGGCCATCCTGCTCGTGTCTTCCTGTGTGGACTCCCTCTGGAGCGTGGCCCAGGCCCTGTTGGTGCT GTGCCCTGGGACTGGGCTCTCTACCCTGTGTCCTGCCTAA |
| |
| SEQ ID NO: 15: TM(11)-CTT |
| MYIVMLTCAVCLVTYMVMAAILHKLDQLDASRGRAIPFCGQRGRFKYEILVKTGWGRGSGTTAHVGIMLYGVDSRSGHRHLDGDR AFHRNSLDIFRIATPHSLGSVWKIRVWHDNKGLSPAWFLQHVIVRDLQTARSAFFLVNDWLSVETEANGGLVEKEVLAASDAALL RFRRLLVAELQRGFFDKHIWLSIWDRPPRSRFTRIQRATCCVLLICLFLGANAVWYGAVGDSAYSTGHVSRLSPLSVDTVAVGLV SSVVVYPVYLAILFLFRMSRSKVAGSPSPTPAGQQVLDIDSCLDSSVLDSSFLTFSGLHAEQAFVGQMKSDLFLDDSKSLVCWPS GEGTLSWPDLLSDPSIVGSNLRQLARGQAGHGLGPEEDGFSLASPYSPAKSFSASDEDLIQQVLAEGVSSPAPTQDTHMETDLLS SLSSTPGEKTETLALQRLGELGPPSPGLNWEQPQAARLSRTGLVEGLRKRLLPAWCASLAHGLSLLLVAVAVAVSGWVGASFPPG VSVAWLLSSSASFLASFLGWEPLKVLLEALYFSLVAKRLHPDEDDTLVESPAVTPVSARVPRVRPPHGFALFLAKEEARKVKRLH GMLRSLLVYMLFLLVTLLASYGDASCHGHAYRLQSAIKQELHSRAFLAITRSEELWPWMAHVLLPYVHGNQSSPELGPPRLRQVR LQEALYPDPPGPRVHTCSAAGGFSTSDYDVGWESPHNGSGTWAYSAPDLLGAWSWGSCAVYDSGGYVQELGLSLEESRDRLRFLQ LHNWLDNRSRAVFLELTRYSPAVGLHAAVTLRLEFPAAGRALAALSVRPFALRRLSAGLSLPLLTSVCLLLFAVHFAVAEARTWH REGRWRVLRLGAWARWLLVALTAATALVRLAQLGAADRQWTRFVRGRPRRFTSFDQVAQLSSAARGLAASLLFLLLVKAAQQLRF VRQWSVFGKTLCRALPELLGVTLGLVVLGVAYAQLAILLVSSCVDSLWSVAQALLVLCPGTGLSTLCPAESWHLSPLLCVGLWAL RLWGALRLGAVILRWRYHALRGELYRPAWEPQDYEMVELFLRRLRLWMGLSKVKEFRHKVRFEGMEPLPSRSSRGSKVSPDVPPP SAGSDASHPSTSSSQLDGLSVSLGRLGTRCEPEPSRLQAVFEALLTQFDRLNQATEDVYQLEQQLHSLQGRRSSRAPAGSSRGPS PGLRPALPSRLARASRGVDLATGPSRTPLRAKNKVHPSST |
| |
| SEQ ID NO: 16: TM(11)-CTT |
| ATGTACATCGTCATGCTGACATGTGCTGTGTGCCTGGTGACCTACATGGTCATGGCCGCCATCCTGCACAAGCTGGACCAGTTGG ATGCCAGCCGGGGCCGCGCCATCCCTTTCTGTGGGCAGCGGGGCCGCTTCAAGTACGAGATCCTCGTCAAGACAGGCTGGGGCCG GGGCTCAGGTACCACGGCCCACGTGGGCATCATGCTGTATGGGGTGGACAGCCGGAGCGGCCACCGGCACCTGGACGGCGACAGA GCCTTCCACCGCAACAGCCTGGACATCTTCCGGATCGCCACCCCGCACAGCCTGGGTAGCGTGTGGAAGATCCGAGTGTGGCACG ACAACAAAGGGCTCAGCCCTGCCTGGTTCCTGCAGCACGTCATCGTCAGGGACCTGCAGACGGCACGCAGCGCCTTCTTCCTGGT CAATGACTGGCTTTCGGTGGAGACGGAGGCCAACGGGGGCCTGGTGGAGAAGGAGGTGCTGGCCGCGAGCGACGCAGCCCTTTTG CGCTTCCGGCGCCTGCTGGTGGCTGAGCTGCAGCGTGGCTTCTTTGACAAGCACATCTGGCTCTCCATATGGGACCGGCCGCCTC GTAGCCGTTTCACTCGCATCCAGAGGGCCACCTGCTGCGTTCTCCTCATCTGCCTCTTCCTGGGCGCCAACGCCGTGTGGTACGG GGCTGTTGGCGACTCTGCCTACAGCACGGGGCATGTGTCCAGGCTGAGCCCGCTGAGCGTCGACACAGTCGCTGTTGGCCTGGTG TCCAGCGTGGTTGTCTATCCCGTCTACCTGGCCATCCTTTTTCTCTTCCGGATGTCCCGGAGCAAGGTGGCTGGGAGCCCGAGCC CCACACCTGCCGGGCAGCAGGTGCTGGACATCGACAGCTGCCTGGACTCGTCCGTGCTGGACAGCTCCTTCCTCACGTTCTCAGG CCTCCACGCTGAGCAGGCCTTTGTTGGACAGATGAAGAGTGACTTGTTTCTGGATGATTCTAAGAGTCTGGTGTGCTGGCCCTCC GGCGAGGGAACGCTCAGTTGGCCGGACCTGCTCAGTGACCCGTCCATTGTGGGTAGCAATCTGCGGCAGCTGGCACGGGGCCAGG |

### Table 2-16

**Table 2-16**

| |
|---|
| CGGGCCATGGGCTGGGCCCAGAGGAGGACGGCTTCTCCCTGGCCAGCCCCTACTCGCCTGCCAAATCCTTCTCAGCATCAGATGA AGACCTGATCCAGCAGGTCCTTGCCGAGGGGGTCAGCAGCCCAGCCCCTACCCAAGACACCCACATGGAAACGGACCTGCTCAGC AGCCTGTCCAGCACTCCTGGGGAGAAGACAGAGACGCTGGCGCTGCAGAGGCTGGGGGAGCTGGGGCCACCCAGCCCAGGCCTGA ACTGGGAACAGCCCCAGGCAGCGAGGCTGTCCAGGACAGGACTGGTGGAGGGTCTGCGGAAGCGCCTGCTGCCGGCCTGGTGTGC CTCCCTGGCCCACGGGCTCAGCCTGCTCCTGGTGGCTGTGGCTGTGGCTGTCTCAGGGTGGGTGGGTGCGAGCTTCCCCCCGGGC GTGAGTGTTGCGTGGCTCCTGTCCAGCAGCGCCAGCTTCCTGGCCTCATTCCTCGGCTGGGAGCCACTGAAGGTCTTGCTGGAAG CCCTGTACTTCTCACTGGTGGCCAAGCGGCTGCACCCGGATGAAGATGACACCCTGGTAGAGAGCCCGGCTGTGACGCCTGTGAG CGCACGTGTGCCCCGCGTACGGCCACCCCACGGCTTTGCACTCTTCCTGGCCAAGGAAGAAGCCCGCAAGGTCAAGAGGCTACAT GGCATGCTGCGGAGCCTCCTGGTGTACATGCTTTTTCTGCTGGTGACCCTGCTGGCCAGCTATGGGGATGCCTCATGCCATGGGC ACGCCTACCGTCTGCAAAGCGCCATCAAGCAGGAGCTGCACAGCCGGGCCTTCCTGGCCATCACGCGGTCTGAGGAGCTCTGGCC ATGGATGGCCCACGTGCTGCTGCCCTACGTCCACGGGAACCAGTCCAGCCCAGAGCTGGGGCCCCCACGGCTGCGGCAGGTGCGG CTGCAGGAAGCACTCTACCCAGACCCTCCCGGCCCCAGGGTCCACACGTGCTCGGCCGCAGGAGGCTTCAGCACCAGCGATTACG ACGTTGGCTGGGAGAGTCCTCACAATGGCTCGGGGACGTGGGCCTATTCAGCGCCGGATCTGCTGGGGGCATGGTCCTGGGGCTC CTGTGCCGTGTATGACAGCGGGGGCTACGTGCAGGAGCTGGGCCTGAGCCTGGAGGAGAGCCGCGACCGGCTGCGCTTCCTGCAG CTGCACAACTGGCTGGACAACAGGAGCCGCGCTGTGTTCCTGGAGCTCACGCGCTACAGCCCGGCCGTGGGGCTGCACGCCGCCG TCACGCTGCGCCTCGAGTTCCCGGCGGCCGGCCGCGCCCTGGCCGCCCTCAGCGTCCGCCCCTTTGCGCTGCGCCGCCTCAGCGC GGGCCTCTCGCTGCCTCTGCTCACCTCGGTGTGCCTGCTGCTGTTCGCCGTGCACTTCGCCGTGGCCGAGGCCCGTACTTGGCAC AGGGAAGGGCGCTGGCGCGTGCTGCGGCTCGGAGCCTGGGCGCGGTGGCTGCTGGTGGCGCTGACGGCGGCCACGGCACTGGTAC GCCTCGCCCAGCTGGGTGCCGCTGACCGCCAGTGGACCCGTTTCGTGCGCGGCCGCCCGCGCCGCTTCACTAGCTTCGACCAGGT GGCGCAGCTGAGCTCCGCAGCCCGTGGCCTGGCGGCCTCGCTGCTCTTCCTGCTTTTGGTCAAGGCTGCCCAGCAGCTACGCTTC GTGCGCCAGTGGTCCGTCTTTGGCAAGACATTATGCCGAGCTCTGCCAGAGCTCCTGGGGGTCACCTTGGGCCTGGTGGTGCTCG GGGTAGCCTACGCCCAGCTGGCCATCCTGCTCGTGTCTTCCTGTGTGGACTCCCTCTGGAGCGTGGCCCAGGCCCTGTTGGTGCT GTGCCCTGGGACTGGGCTCTCTACCCTGTGTCCTGCCGAGTCCTGGCACCTGTCACCCCTGCTGTGTGTGGGGCTCTGGGCACTG CGGCTGTGGGGCGCCCTACGGCTGGGGGCTGTTATTCTCCGCTGGCGCTACCACGCCTTGCGTGGAGAGCTGTACCGGCCGGCCT GGGAGCCCCAGGACTACGAGATGGTGGAGTTGTTCCTGCGCAGGCTGCGCCTCTGGATGGGCCTCAGCAAGGTCAAGGAGTTCCG CCACAAAGTCCGCTTTGAAGGGATGGAGCCGCTGCCCTCTCGCTCCTCCAGGGGCTCCAAGGTATCCCCGGATGTGCCCCCACCC AGCGCTGGCTCCGATGCCTCGCACCCCTCCACCTCCTCCAGCCAGCTGGATGGGCTGAGCGTGAGCCTGGGCCGGCTGGGGACAA GGTGTGAGCCTGAGCCCTCCCGCCTCCAAGCCGTGTTCGAGGCCCTGCTCACCCAGTTTGACCGACTCAACCAGGCCACAGAGGA CGTCTACCAGCTGGAGCAGCAGCTGCACAGCCTGCAAGGCCGCAGGAGCAGCCGGGCGCCCGCCGGATCTTCCCGTGGCCCATCC CCGGGCCTGCGGCCAGCACTGCCCAGCCGCCTTGCCCGGGCCAGTCGGGGTGTGGACCTGGCCACTGGCCCCAGCAGGACACCCC TTCGGGCCAAGAACAAGGTCCACCCCAGCAGCACTTAA |
| |
| SEQ ID NO: 17: TLD |
| MGWEPLKVLLEALYFSLVAKRLHPDEDDTLVESPAVTPVSARVPRVRPPHGFALFLAKEEARKVKRLHGMLRSLLVYMLFLLVTL LASYGDASCHGHAYRLQSAIKQELHSRAFLAITRSEELWPWMAHVLLPYVHGNQSSPELGPPRLRQVRLQEALYPDPPGPRVHTC SAAGGFSTSDYDVGWESPHNGSGTWAYSAPDLLGAWSWGSCAVYDSGGYVQELGLSLEESRDRLRFLQLHNWLDNRSRAVFLELT RYSPAVGLHAAVTLRLEFPAAGRALAALSVRPFALRRLSAGLSLPLLTSVCLLLFAVHFAVAEARTWHREGRWRVLRLGAWARWL LVALTAATALVRLAQLGAADRQWTRFVRGRPRRFTSFDQVAQLSSAARGLAASLLFLLLVKAAQQLRFVRQWSVFGKTLCRALPE LLGVTLGLVVLGVAYAQLAILLVSSCVDSLWSVAQALLVLCPGTGLSTLCPAESWHLSPLLCVGLWALRLWGALRLGAVILRWRY HALRGELYRPAWEPQDYEMVELFLRRLRLWMGLSKVKEFRHKVRFEGMEPLPSRSSRGSKVSPDVPPPSAGSDASHPSTSSSQLD GLSVSLGRLGTRCEPEPSRLQAVFEALLTQFDRLNQATEDVYQLEQQLHSLQGRRSSRAPAGSSRGPSPGLRPALPSRLARASRG |

### Table 2-17

**Table 2-17**

| |
|---|
| VDLATGPSRTPLRAKNKVHPSST |
| |
| SEQ ID NO: 18: TLD |
| ATGGGCTGGGAGCCACTGAAGGTCTTGCTGGAAGCCCTGTACTTCTCACTGGTGGCCAAGCGGCTGCACCCGGATGAAGATGACA CCCTGGTAGAGAGCCCGGCTGTGACGCCTGTGAGCGCACGTGTGCCCCGCGTACGGCCACCCCACGGCTTTGCACTCTTCCTGGC CAAGGAAGAAGCCCGCAAGGTCAAGAGGCTACATGGCATGCTGCGGAGCCTCCTGGTGTACATGCTTTTTCTGCTGGTGACCCTG CTGGCCAGCTATGGGGATGCCTCATGCCATGGGCACGCCTACCGTCTGCAAAGCGCCATCAAGCAGGAGCTGCACAGCCGGGCCT TCCTGGCCATCACGCGGTCTGAGGAGCTCTGGCCATGGATGGCCCACGTGCTGCTGCCCTACGTCCACGGGAACCAGTCCAGCCC AGAGCTGGGGCCCCCACGGCTGCGGCAGGTGCGGCTGCAGGAAGCACTCTACCCAGACCCTCCCGGCCCCAGGGTCCACACGTGC TCGGCCGCAGGAGGCTTCAGCACCAGCGATTACGACGTTGGCTGGGAGAGTCCTCACAATGGCTCGGGGACGTGGGCCTATTCAG CGCCGGATCTGCTGGGGGCATGGTCCTGGGGCTCCTGTGCCGTGTATGACAGCGGGGGCTACGTGCAGGAGCTGGGCCTGAGCCT GGAGGAGAGCCGCGACCGGCTGCGCTTCCTGCAGCTGCACAACTGGCTGGACAACAGGAGCCGCGCTGTGTTCCTGGAGCTCACG CGCTACAGCCCGGCCGTGGGGCTGCACGCCGCCGTCACGCTGCGCCTCGAGTTCCCGGCGGCCGGCCGCGCCCTGGCCGCCCTCA GCGTCCGCCCCTTTGCGCTGCGCCGCCTCAGCGCGGGCCTCTCGCTGCCTCTGCTCACCTCGGTGTGCCTGCTGCTGTTCGCCGT GCACTTCGCCGTGGCCGAGGCCCGTACTTGGCACAGGGAAGGGCGCTGGCGCGTGCTGCGGCTCGGAGCCTGGGCGCGGTGGCTG CTGGTGGCGCTGACGGCGGCCACGGCACTGGTACGCCTCGCCCAGCTGGGTGCCGCTGACCGCCAGTGGACCCGTTTCGTGCGCG GCCGCCCGCGCCGCTTCACTAGCTTCGACCAGGTGGCGCAGCTGAGCTCCGCAGCCCGTGGCCTGGCGGCCTCGCTGCTCTTCCT GCTTTTGGTCAAGGCTGCCCAGCAGCTACGCTTCGTGCGCCAGTGGTCCGTCTTTGGCAAGACATTATGCCGAGCTCTGCCAGAG CTCCTGGGGGTCACCTTGGGCCTGGTGGTGCTCGGGGTAGCCTACGCCCAGCTGGCCATCCTGCTCGTGTCTTCCTGTGTGGACT CCCTCTGGAGCGTGGCCCAGGCCCTGTTGGTGCTGTGCCCTGGGACTGGGCTCTCTACCCTGTGTCCTGCCGAGTCCTGGCACCT GTCACCCCTGCTGTGTGTGGGGCTCTGGGCACTGCGGCTGTGGGGCGCCCTACGGCTGGGGGCTGTTATTCTCCGCTGGCGCTAC CACGCCTTGCGTGGAGAGCTGTACCGGCCGGCCTGGGAGCCCCAGGACTACGAGATGGTGGAGTTGTTCCTGCGCAGGCTGCGCC TCTGGATGGGCCTCAGCAAGGTCAAGGAGTTCCGCCACAAAGTCCGCTTTGAAGGGATGGAGCCGCTGCCCTCTCGCTCCTCCAG GGGCTCCAAGGTATCCCCGGATGTGCCCCCACCCAGCGCTGGCTCCGATGCCTCGCACCCCTCCACCTCCTCCAGCCAGCTGGAT GGGCTGAGCGTGAGCCTGGGCCGGCTGGGGACAAGGTGTGAGCCTGAGCCCTCCCGCCTCCAAGCCGTGTTCGAGGCCCTGCTCA CCCAGTTTGACCGACTCAACCAGGCCACAGAGGACGTCTACCAGCTGGAGCAGCAGCTGCACAGCCTGCAAGGCCGCAGGAGCAG CCGGGCGCCCGCCGGATCTTCCCGTGGCCCATCCCCGGGCCTGCGGCCAGCACTGCCCAGCCGCCTTGCCCGGGCCAGTCGGGGT GTGGACCTGGCCACTGGCCCCAGCAGGACACCCCTTCGGGCCAAGAACAAGGTCCACCCCAGCAGCACTTAA |
| |
| SEQ ID NO: 19: Ig*κ*-TLD |
| METDTLLLWVLLLWVPGSTGDGWEPLKVLLEALYFSLVAKRLHPDEDDTLVESPAVTPVSARVPRVRPPHGFALFLAKEEARKVK RLHGMLRSLLVYMLFLLVTLLASYGDASCHGHAYRLQSAIKQELHSRAFLAITRSEELWPWMAHVLLPYVHGNQSSPELGPPRLR QVRLQEALYPDPPGPRVHTCSAAGGFSTSDYDVGWESPHNGSGTWAYSAPDLLGAWSWGSCAVYDSGGYVQELGLSLEESRDRLR FLQLHNWLDNRSRAVFLELTRYSPAVGLHAAVTLRLEFPAAGRALAALSVRPFALRRLSAGLSLPLLTSVCLLLFAVHFAVAEAR TWHREGRWRVLRLGAWARWLLVALTAATALVRLAQLGAADRQWTRFVRGRPRRFTSFDQVAQLSSAARGLAASLLFLLLVKAAQQ LRFVRQWSVFGKTLCRALPELLGVTLGLVVLGVAYAQLAILLVSSCVDSLWSVAQALLVLCPGTGLSTLCPAESWHLSPLLCVGL WALRLWGALRLGAVILRWRYHALRGELYRPAWEPQDYEMVELFLRRLRLWMGLSKVKEFRHKVRFEGMEPLPSRSSRGSKVSPDV PPPSAGSDASHPSTSSSQLDGLSVSLGRLGTRCEPEPSRLQAVFEALLTQFDRLNQATEDVYQLEQQLHSLQGRRSSRAPAGSSR GPSPGLRPALPSRLARASRGVDLATGPSRTPLRAKNKVHPSST |
| |
| SEQ ID NO: 20: Ig*κ*-TLD |

### Table 2-18

**Table 2-18**

| |
|---|
| ATGGAGACAGACACACTCCTGCTATGGGTACTGCTGCTCTGGGTTCCAGGTTCCACTGGTGACGGCTGGGAGCCACTGAAGGTCT TGCTGGAAGCCCTGTACTTCTCACTGGTGGCCAAGCGGCTGCACCCGGATGAAGATGACACCCTGGTAGAGAGCCCGGCTGTGAC GCCTGTGAGCGCACGTGTGCCCCGCGTACGGCCACCCCACGGCTTTGCACTCTTCCTGGCCAAGGAAGAAGCCCGCAAGGTCAAG AGGCTACATGGCATGCTGCGGAGCCTCCTGGTGTACATGCTTTTTCTGCTGGTGACCCTGCTGGCCAGCTATGGGGATGCCTCAT GCCATGGGCACGCCTACCGTCTGCAAAGCGCCATCAAGCAGGAGCTGCACAGCCGGGCCTTCCTGGCCATCACGCGGTCTGAGGA GCTCTGGCCATGGATGGCCCACGTGCTGCTGCCCTACGTCCACGGGAACCAGTCCAGCCCAGAGCTGGGGCCCCCACGGCTGCGG CAGGTGCGGCTGCAGGAAGCACTCTACCCAGACCCTCCCGGCCCCAGGGTCCACACGTGCTCGGCCGCAGGAGGCTTCAGCACCA GCGATTACGACGTTGGCTGGGAGAGTCCTCACAATGGCTCGGGGACGTGGGCCTATTCAGCGCCGGATCTGCTGGGGGCATGGTC CTGGGGCTCCTGTGCCGTGTATGACAGCGGGGGCTACGTGCAGGAGCTGGGCCTGAGCCTGGAGGAGAGCCGCGACCGGCTGCGC TTCCTGCAGCTGCACAACTGGCTGGACAACAGGAGCCGCGCTGTGTTCCTGGAGCTCACGCGCTACAGCCCGGCCGTGGGGCTGC ACGCCGCCGTCACGCTGCGCCTCGAGTTCCCGGCGGCCGGCCGCGCCCTGGCCGCCCTCAGCGTCCGCCCCTTTGCGCTGCGCCG CCTCAGCGCGGGCCTCTCGCTGCCTCTGCTCACCTCGGTGTGCCTGCTGCTGTTCGCCGTGCACTTCGCCGTGGCCGAGGCCCGT ACTTGGCACAGGGAAGGGCGCTGGCGCGTGCTGCGGCTCGGAGCCTGGGCGCGGTGGCTGCTGGTGGCGCTGACGGCGGCCACGG CACTGGTACGCCTCGCCCAGCTGGGTGCCGCTGACCGCCAGTGGACCCGTTTCGTGCGCGGCCGCCCGCGCCGCTTCACTAGCTT CGACCAGGTGGCGCAGCTGAGCTCCGCAGCCCGTGGCCTGGCGGCCTCGCTGCTCTTCCTGCTTTTGGTCAAGGCTGCCCAGCAG CTACGCTTCGTGCGCCAGTGGTCCGTCTTTGGCAAGACATTATGCCGAGCTCTGCCAGAGCTCCTGGGGGTCACCTTGGGCCTGG TGGTGCTCGGGGTAGCCTACGCCCAGCTGGCCATCCTGCTCGTGTCTTCCTGTGTGGACTCCCTCTGGAGCGTGGCCCAGGCCCT GTTGGTGCTGTGCCCTGGGACTGGGCTCTCTACCCTGTGTCCTGCCGAGTCCTGGCACCTGTCACCCCTGCTGTGTGTGGGGCTC TGGGCACTGCGGCTGTGGGGCGCCCTACGGCTGGGGGCTGTTATTCTCCGCTGGCGCTACCACGCCTTGCGTGGAGAGCTGTACC GGCCGGCCTGGGAGCCCCAGGACTACGAGATGGTGGAGTTGTTCCTGCGCAGGCTGCGCCTCTGGATGGGCCTCAGCAAGGTCAA GGAGTTCCGCCACAAAGTCCGCTTTGAAGGGATGGAGCCGCTGCCCTCTCGCTCCTCCAGGGGCTCCAAGGTATCCCCGGATGTG CCCCCACCCAGCGCTGGCTCCGATGCCTCGCACCCCTCCACCTCCTCCAGCCAGCTGGATGGGCTGAGCGTGAGCCTGGGCCGGC TGGGGACAAGGTGTGAGCCTGAGCCCTCCCGCCTCCAAGCCGTGTTCGAGGCCCTGCTCACCCAGTTTGACCGACTCAACCAGGC CACAGAGGACGTCTACCAGCTGGAGCAGCAGCTGCACAGCCTGCAAGGCCGCAGGAGCAGCCGGGCGCCCGCCGGATCTTCCCGT GGCCCATCCCCGGGCCTGCGGCCAGCACTGCCCAGCCGCCTTGCCCGGGCCAGTCGGGGTGTGGACCTGGCCACTGGCCCCAGCA GGACACCCCTTCGGGCCAAGAACAAGGTCCACCCCAGCAGCACTTAA |
| |
| SEQ ID NO: 21: CD16.7-CTT |
| MWQLLLPTALLLLVSAGMRTEDLPKAVVFLEPQWYSVLEKDSVTLKCQGAYSPEDNSTQWFHNESLISSQASSYFIDAATVNDSG EYRCQTNLSTLSDPVQLEVHIGWLLLQAPRWVFKEEDPIHLRCHSWKNTALHKVTYLQNGKDRKYFHHNSDFHIPKATLKDSGSY FCRGLVGSKNVSSETVNITITQGLAVSTISSFSPPGADPPRASALPAPPTGSALPDPQTASALPDPPAASALPAALAVISFLLGL GLGVACVLARTRESWHLSPLLCVGLWALRLWGALRLGAVILRWRYHALRGELYRPAWEPQDYEMVELFLRRLRLWMGLSKVKEFR HKVRFEGMEPLPSRSSRGSKVSPDVPPPSAGSDASHPSTSSSQLDGLSVSLGRLGTRCEPEPSRLQAVFEALLTQFDRLNQATED VYQLEQQLHSLQGRRSSRAPAGSSRGPSPGLRPALPSRLARASRGVDLATGPSRTPLRAKNKVHPSST |
| |
| SEQ ID NO: 22: CD16.7-CTT |
| ATGTGGCAGCTGCTCCTCCCAACTGCTCTGCTACTTCTAGTTTCAGCTGGCATGCGGACTGAAGATCTCCCAAAGGCTGTGGTGT TCCTGGAGCCTCAATGGTACAGCGTGCTTGAGAAGGACAGTGTGACTCTGAAGTGCCAGGGAGCCTACTCCCCTGAGGACAATTC CACACAGTGGTTTCACAATGAGAGCCTCATCTCAAGCCAGGCCTCGAGCTACTTCATTGACGCTGCCACAGTCAACGACAGTGGA GAGTACAGGTGCCAGACAAACCTCTCCACCCTCAGTGACCCGGTGCAGCTAGAAGTCCATATCGGCTGGCTGTTGCTCCAGGCCC CTCGGTGGGTGTTCAAGGAGGAAGACCCTATTCACCTGAGGTGTCACAGCTGGAAGAACACTGCTCTGCATAAGGTCACATATTT |

### Table 2-19

**Table 2-19**

| |
|---|
| ACAGAATGGCAAAGACAGGAAGTATTTTCATCATAATTCTGACTTCCACATTCCAAAAGCCACACTCAAAGATAGCGGCTCCTAC TTCTGCAGGGGGCTTGTTGGGAGTAAAAATGTGTCTTCAGAGACTGTGAACATCACCATCACTCAAGGTTTGGCAGTGTCAACCA TCTCATCATTCTCTCCACCTGGCGCGGATCCACCAAGGGCCTCTGCCCTCCCTGCCCCACCGACAGGCTCCGCCCTCCCTGACCC GCAGACAGCCTCTGCCCTCCCTGACCCGCCAGCAGCCTCTGCCCTCCCTGCGGCCCTGGCGGTGATCTCCTTCCTCCTCGGGCTG GGCCTGGGGGTGGCGTGTGTGCTGGCGAGGACGCGTGAGTCCTGGCACTTGTCACCCCTGCTGTGTGTGGGGCTCTGGGCACTGC GGCTGTGGGGCGCCCTACGGCTGGGGGCTGTTATTCTCCGCTGGCGCTACCACGCCTTGCGTGGAGAGCTGTACCGGCCGGCCTG GGAGCCCCAGGACTACGAGATGGTGGAGTTGTTCCTGCGCAGGCTGCGCCTCTGGATGGGCCTCAGCAAGGTCAAGGAGTTCCGC CACAAAGTCCGCTTTGAAGGGATGGAGCCGCTGCCCTCTCGCTCCTCCAGGGGCTCCAAGGTATCCCCGGATGTGCCCCCACCCA GCGCTGGCTCCGATGCCTCGCACCCCTCCACCTCCTCCAGCCAGCTGGATGGGCTGAGCGTGAGCCTGGGCCGGCTGGGGACAAG GTGTGAGCCTGAGCCCTCCCGCCTCCAAGCCGTGTTCGAGGCCCTGCTCACCCAGTTTGACCGACTCAACCAGGCCACAGAGGAC GTCTACCAGCTGGAGCAGCAGCTGCACAGCCTGCAAGGCCGCAGGAGCAGCCGGGCGCCCGCCGGATCTTCCCGTGGCCCATCCC CGGGCCTGCGGCCAGCACTGCCCAGCCGCCTTGCCCGGGCCAGTCGGGGTGTGGACCTGGCCACTGGCCCCAGCAGGACACCCCT TCGGGCCAAGAACAAGGTCCACCCCAGCAGCACTTAG |
| |
| SEQ ID NO: 23: R-CTF |
| ATGAGTGCTGCTGGGGTGGACCTTGTTCTTGGCCCGAAGGGGTGTCCTGCTGGGGCCAGTGGCCAGGTCCACACCCCGACTGGCC CGGGCAAGGCGGCTGGGCAGTGCTGGCCGCAGGCCCGGGGATGGGCCACGGGAAGATCCGGCGGGCGCCCGGCTGCTCCTGCGGC CTTGCAGGCTGTGCAGCTGCTGCTCCAGCTGGTAGACGTCCTCTGTGGCCTGGTTGAGTCGGTCAAACTGGGTGAGCAGGGCCTC GAACACGGCTTGGAGGCGGGAGGGCTCAGGCTCACACCTTGTCCCCAGCCGGCCCAGGCTCACGCTCAGCCCATCCAGCTGGCTG GAGGAGGTGGAGGGGTGCGAGGCATCGGAGCCAGCGCTGGGTGGGGGCACATCCGGGGATACCTTGGAGCCCCTGGAGGAGCGAG AGGGCAGCGGCTCCATCCCTTCAAAGCGGACTTTGTGGCGGAACTCCTTGACCTTGCTGAGGCCCATCCAGAGGCGCAGCCTGCG CAGGAACAACTCCACCATCTCGTAGTCCTGGGGCTCCCAGGCCGGCCGGTACAGCTCTCCACGCAAGGCGTGGTAGCGCCAGCGG AGAATAACAGCCCCCAGCCGTAGGGCGCCCCACAGCCGCAGTGCCCAGAGCCCCACACACAGCAGGGGTGACAGGTGCCAGGACT CGGCAGGACACAGGGTAGAGAGCCCAGTCCCAGGGCACAGCACCAACAGGGCCTGGGCCACGCTCCAGAGGGAGTCCACACAGGA AGACACGAGCAGGATGGCCAGCTGGGCGTAGGCTACCCCGAGCACCACCAGGCCCAAGGTGACCCCCAGGAGCTCTGGCAGAGCT CGGCATAATGTCTTGCCAAAGACGGACCACTGGCGCACGAAGCGTAGCTGCTGGGCAGCCTTGACCAAAAGCAGGAAGAGCAGCG AGGCCGCCAGGCCACGGGCTGCGGAGCTCAGCTGCGCCACCTGGTCGAAGCTAGTGAAGCGGCGCGGGCGGCCGCGCACGAAACG GGTCCACTGGCGGTCAGCGGCACCCAGCTGGGCGAGGCGTACCAGTGCCGTGGCCGCCGTCAGCGCCACCAGCAGCCACCGCGCC CAGGCTCCGAGCCGCAGCACGCGCCAGCGCCCTTCCCTGTGCCAAGTACGGGCCTCGGCCACGGCGAAGTGCACGGCGAACAGCA GCAGGCACACCGAGGTGAGCAGAGGCAGCGAGAGGCCCGCGCTGAGGCGGCGCAGCGCAAAGGGGCGGACGCTGAGGGCGGCCAG GGCGCGGCCGGCCGCCGGGAACTCGAGGCGCAGCGTGACGGCGGCGTGCAGCCCCACGGCCGGGCTGTAGCGCGTGAGCTCCAGG AACACAGCGCGGCTCCTGTTGTCCAGCCAGTTGTGCAGCTGCAGGAAGCGCAGCCGGTCGCGGCTCTCCTCCAGGCTCAGGCCCA GCTCCTGCACGTAGCCCCCGCTGTCATACACGGCACAGGAGCCCCAGGACCATGCCCCCAGCAGATCCGGCGCTGAATAGGCCCA CGTCCCCGAGCCATTGTGAGGACTCTCCCAGCCAACGTCGTAATCGCTGGTGCTGAAGCCTCCTGCGGCCGAGCACGTGTGGACC CTGGGGCCGGGAGGGTCTGGGTAGAGTGCTTCCTGCAGCCGCACCTGCCGCAGCCGTGGGGGCCCCAGCTCTGGGCTGGACTGGT TCCCGTGGACGTAGGGCAGCAGCACGTGGGCCATCCATGGCCAGAGCTCCTCAGACCGCGTGATGGCCAGGAAGGCCCGGCTGTG CAGCTCCTGCTTGATGGCGCTTTGCAGACGGTAGGCGTGCCCATGGCATGAGGCATCCCCATAGCTGGCCAGCAGGGTCACCAGC AGAAAAAGCATGTACACCAGGAGGCTCCGCAGCATGCCATGTAGCCTCTTGACCTTGCGGGCTTCTTCCTTGGCCAGGAAGAGTG CAAAGCCGTGGGGTGGCCGTACGCGGGGCACACGTGCGCTCACAGGCGTCACAGCCGGGCTCTCTACCAGGGTGTCATCTTCATC CGGGTGCAGCCGCTTGGCCACCAGTGAGAAGTACAGGGCTTCCAGCAAGACCTTCAGTGGCTCCCAGCCGAGGAATGAGGCCAGG AAGCTGGCGCTGCTGGACAGGAGCCACGCAACACTCACGCCCGGGGGGAAGCTCGCACCCACCCACCCTGAGACAGCCACAGCCA |

### Table 2-20

**Table 2-20**

| |
|---|
| CAGCCACCAGGAGCAGGCTGAGCCCGTGGGCCAGGGAGGCACACCAGGCCGGCAGCAGGCGCTTCCGCAGACCCTCCACCAGTCC TGTCCTGGACAGCCTCGCTGCCTGGGGCTGTTCCCAGTTCAGGCCTGGGCTGGGTGGCCCCAGCTCCCCCAGCCTCTGCAGCGCC AGCGTCTCTGTCTTCTCCCCAGGAGTGCTGGACAGGCTGCTGAGCAGGTCCGTTTCCATGTGGGTGTCTTGGGTAGGGGCTGGGC TGCTGACCCCCTCGGCAAGGACCTGCTGGATCAGGTCTTCATCTGATGCTGAGAAGGATTTGGCAGGCGAGTAGGGGCTGGCCAG GGAGAAGCCGTCCTCCTCTGGGCCCAGCCCATGGCCCGCCTGGCCCCGTGCCAGCTGCCGCAGATTGCTACCCACAATGGACGGG TCACTGAGCAGGTCCGGCCAACTGAGCGTTCCCTCGCCGGAGGGCCAGCACACCAGACTCTTAGAATCATGCAGAAACAAGTCAC TCTTCATCTGTCCAACAAAGGCCTGCTCAGCGTGGAGGCCTGAGAACGTGAGGAAGGAGCTGTCCAGCACGGACGAGTCCAGGCA GCTGTCGATGTCCAGCACCTGCTGCCCGGCAGGTGTGGGGCTCGGGCTCCCAGCCACCTTGCTCCGGGACATCCGGAAGAGAAAA AGGATGGCCAGGTAGACGGGATAGACAACCACGCTGGACACCAGGCCAACAGCGACTGTGTCGACGCTCAGCGGGCTCAGCCTGG ACACATGCCCCGTGCTGTAGGCAGAGTCGCCAACAGCCCCGTACCACACGGCGTTGGCGCCCAGGAAGAGGCAGATGAGGAGAAC GCAGCAGGTGGCCCTCTGGATGCGAGTGAAACGGCTACGAGGCGGCCGGTCCCATATGGAGAGCCAGATGTGCTTGTCAAAGAAG CCACGCTGCAGCTCAGCCACCAGCAGGCGCCGGAAGCGCAAAAGGGCTGCGTCGCTCGCGGCCAGCACCTCCTTCTCCACCAGGC CCCCGTTGGCCTCCGTCTCCACCGAAAGCCAGTCATTGACCAGGAAGAAGGCGCTGCGTGCCGTCTGCAGGTCCCTGACGATGAC GTGCTGCAGGAACCAGGCAGGGCTGAGCCCTTTGTTGTCGTGCCACACTCGGATCTTCCACACGCTACCCAGGCTGTGCGGGGTG GCGATCCGGAAGATGTCCAGGCTGTTGCGGTGGAAGGCTCTGTCGCCGTCCAGGTGCCGGTGGCCGCTCCGGCTGTCCACCCCAT ACAGCATGATGCCCACGTGGGCCGTGGTACCTGAGCCCCGGCCCCAGCCTGTCTTGACGAGGATCTCGTACTTGAAGCGGCCCCG CTGCCCACAGAAAGGGATGGCGCGGCCCCGGCTGGCATCCAACTGGTCCAGCTTGTGCAGGATGGCGGCCATGACCATGTAGGTC ACCAGGCACACAGCACATGTCAGCATGACGATGTAGTTTACATCCGCTGTCGGCTCAGGAAACACAAAGCGGACATGGCTTGGGG GCACGAAGAGGCTGGCGCCGAAGGCGGTTAA |

### Table 5-1

**Table 5-1**

| |
|---|
| SEQ ID NO: 24: FAS-CTT |
| MLGIWTLLPLVLTSVARLSSKSVNAQVTDINSKGLELRKTVTTVETQNLEGLHHDGQFCHKPCPPGERKARDCTVNGDEPDCVPC QEGKEYTDKAHFSSKCRRCRLCDEGHGLEVEINCTRTQNTKCRCKPNFFCNSTVCEHCDPCTKCEHGIIKECTLTSNTKCKEEGS RSNLGWLCLLLLPIPLIVWVVILRWRYHALRGELYRPAWEPQDYEMVELFLRRLRLWMGLSKVKEFRHKVRFEGMEPLPSRSSRG SKVSPDVPPPSAGSDASHPSTSSSQLDGLSVSLGRLGTRCEPEPSRLQAVFEALLTQFDRLNQATEDVYQLEQQLHSLQGRRSSR APAGSSRGPSPGLRPALPSRLARASRGVDLATGPSRTPLRAKNKVHPSST |
| |
| SEQ ID NO: 25: FAS-CTT |
| ATGCTGGGCATCTGGACCCTCCTACCTCTGGTTCTTACGTCTGTTGCTAGATTATCGTCCAAAAGTGTTAATGCCCAAGTGACTG ACATCAACTCCAAGGGATTGGAATTGAGGAAGACTGTTACTACAGTTGAGACTCAGAACTTGGAAGGCCTGCATCATGATGGCCA ATTCTGCCATAAGCCCTGTCCTCCAGGTGAAAGGAAAGCTAGGGACTGCACAGTCAATGGGGATGAACCAGACTGCGTGCCCTGC CAAGAAGGGAAGGAGTACACAGACAAAGCCCATTTTTCTTCCAAATGCAGAAGATGTAGATTGTGTGATGAAGGACATGGCTTAG AAGTGGAAATAAACTGCACCCGGACCCAGAATACCAAGTGCAGATGTAAACCAAACTTTTTTTGTAACTCTACTGTATGTGAACA CTGTGACCCTTGCACCAAATGTGAACATGGAATCATCAAGGAATGCACACTCACCAGCAACACCAAGTGCAAAGAGGAAGGATCC AGATCTAACTTGGGGTGGCTTTGTCTTCTTCTTTTGCCAATTCCACTAATTGTTTGGGTGGTTATTCTCCGCTGGCGCTACCACG CCTTGCGTGGAGAGCTGTACCGGCCGGCCTGGGAGCCCCAGGACTACGAGATGGTGGAGTTGTTCCTGCGCAGGCTGCGCCTCTG GATGGGCCTCAGCAAGGTCAAGGAGTTCCGCCACAAAGTCCGCTTTGAAGGGATGGAGCCGCTGCCCTCTCGCTCCTCCAGGGGC TCCAAGGTATCCCCGGATGTGCCCCCACCCAGCGCTGGCTCCGATGCCTCGCACCCCTCCACCTCCTCCAGCCAGCTGGATGGGC TGAGCGTGAGCCTGGGCCGGCTGGGGACAAGGTGTGAGCCTGAGCCCTCCCGCCTCCAAGCCGTGTTCGAGGCCCTGCTCACCCA GTTTGACCGACTCAACCAGGCCACAGAGGACGTCTACCAGCTGGAGCAGCAGCTGCACAGCCTGCAAGGCCGCAGGAGCAGCCGG GCGCCCGCCGGATCTTCCCGTGGCCCATCCCCGGGCCTGCGGCCAGCACTGCCCAGCCGCCTTGCCCGGGCCAGTCGGGGTGTGG ACCTGGCCACTGGCCCCAGCAGGACACCCCTTCGGGCCAAGAACAAGGTCCACCCCAGCAGCACTTAG |
| |
| SEQ ID NO: 26: CTTdNLS |
| MVILRWRYHALRGELYRPAWEPQDYEMVELFFEGMEPLPSRSSRGSKVSPDVPPPSAGSDASHPSTSSSQLDGLSVSLGRLGTRC EPEPSRLQAVFEALLTQFDRLNQATEDVYQLEQQLHSLQGRRSSRAPAGSSRGPSPGLRPALPSRLARASRGVDLATGPSRTPLR AKNKVHPSST |
| |
| SEQ ID NO: 27: CTTdNLS |
| ATGGTTATTCTCCGCTGGCGCTACCACGCCTTGCGTGGAGAGCTGTACCGGCCGGCCTGGGAGCCCCAGGACTACGAGATGGTGG AGTTGTTCTTTGAAGGGATGGAGCCGCTGCCCTCTCGCTCCTCCAGGGGCTCCAAGGTATCCCCGGATGTGCCCCCACCCAGCGC TGGCTCCGATGCCTCGCACCCCTCCACCTCCTCCAGCCAGCTGGATGGGCTGAGCGTGAGCCTGGGCCGGCTGGGGACAAGGTGT GAGCCTGAGCCCTCCCGCCTCCAAGCCGTGTTCGAGGCCCTGCTCACCCAGTTTGACCGACTCAACCAGGCCACAGAGGACGTCT ACCAGCTGGAGCAGCAGCTGCACAGCCTGCAAGGCCGCAGGAGCAGCCGGGCGCCCGCCGGATCTTCCCGTGGCCCATCCCCGGG CCTGCGGCCAGCACTGCCCAGCCGCCTTGCCCGGGCCAGTCGGGGTGTGGACCTGGCCACTGGCCCCAGCAGGACACCCCTTCGG GCCAAGAACAAGGTCCACCCCAGCAGCACTTAG |
| |
| SEQ ID NO: 28: CTT4104-4203 |
| MVILRWRYHALRGELYRPAWEPQDYEMVELFLRRLRLWMGLSKVKEFRHKVRFEGMEPLPSRSSRGSKVSPDVPPPSAGSDASHP |

### Table 5-2

**Table 5-2**

| |
|---|
| STSSSQLDGLSVSLGR |
| |
| SEQ ID NO: 29: CTT4104-4203 |
| ATGGTTATTCTCCGCTGGCGCTACCACGCCTTGCGTGGAGAGCTGTACCGGCCGGCCTGGGAGCCCCAGGACTACGAGATGGTGG AGTTGTTCCTGCGCAGGCTGCGCCTCTGGATGGGCCTCAGCAAGGTCAAGGAGTTCCGCCACAAAGTCCGCTTTGAAGGGATGGA GCCGCTGCCCTCTCGCTCCTCCAGGGGCTCCAAGGTATCCCCGGATGTGCCCCCACCCAGCGCTGGCTCCGATGCCTCGCACCCC TCCACCTCCTCCAGCCAGCTGGATGGGCTGAGCGTGAGCCTGGGCCGGTAG |
| |
| SEQ ID NO: 30: CTT4154-4253 |
| MRFEGMEPLPSRSSRGSKVSPDVPPPSAGSDASHPSTSSSQLDGLSVSLGRLGTRCEPEPSRLQAVFEALLTQFDRLNQATEDVY QLEQQLHSLQGRRSSR |
| |
| SEQ ID NO: 31: CTT4154-4253 |
| ATGCGCTTTGAAGGGATGGAGCCGCTGCCCTCTCGCTCCTCCAGGGGCTCCAAGGTATCCCCGGATGTGCCCCCACCCAGCGCTG GCTCCGATGCCTCGCACCCCTCCACCTCCTCCAGCCAGCTGGATGGGCTGAGCGTGAGCCTGGGCCGGCTGGGGACAAGGTGTGA GCCTGAGCCCTCCCGCCTCCAAGCCGTGTTCGAGGCCCTGCTCACCCAGTTTGACCGACTCAACCAGGCCACAGAGGACGTCTAC CAGCTGGAGCAGCAGCTGCACAGCCTGCAAGGCCGCAGGAGCAGCCGGTAG |
| |
| SEQ ID NO: 32: CTT4193-4303 |
| MQLDGLSVSLGRLGTRCEPEPSRLQAVFEALLTQFDRLNQATEDVYQLEQQLHSLQGRRSSRAPAGSSRGPSPGLRPALPSRLAR ASRGVDLATGPSRTPLRAKNKVHPSST |
| |
| SEQ ID NO: 33: CTT4193-4303 |
| ATGCAGCTGGATGGGCTGAGCGTGAGCCTGGGCCGGCTGGGGACAAGGTGTGAGCCTGAGCCCTCCCGCCTCCAAGCCGTGTTCG AGGCCCTGCTCACCCAGTTTGACCGACTCAACCAGGCCACAGAGGACGTCTACCAGCTGGAGCAGCAGCTGCACAGCCTGCAAGG CCGCAGGAGCAGCCGGGCGCCCGCCGGATCTTCCCGTGGCCCATCCCCGGGCCTGCGGCCAGCACTGCCCAGCCGCCTTGCCCGG GCCAGTCGGGGTGTGGACCTGGCCACTGGCCCCAGCAGGACACCCCTTCGGGCCAAGAACAAGGTCCACCCCAGCAGCACTTAG |
| |
| SEQ ID NO: 34: CTT4204-4303 |
| MLGTRCEPEPSRLQAVFEALLTQFDRLNQATEDVYQLEQQLHSLQGRRSSRAPAGSSRGPSPGLRPALPSRLARASRGVDLATGP SRTPLRAKNKVHPSST |
| |
| SEQ ID NO: 35: CTT4204-4303 |
| ATGCTGGGGACAAGGTGTGAGCCTGAGCCCTCCCGCCTCCAAGCCGTGTTCGAGGCCCTGCTCACCCAGTTTGACCGACTCAACC AGGCCACAGAGGACGTCTACCAGCTGGAGCAGCAGCTGCACAGCCTGCAAGGCCGCAGGAGCAGCCGGGCGCCCGCCGGATCTTC CCGTGGCCCATCCCCGGGCCTGCGGCCAGCACTGCCCAGCCGCCTTGCCCGGGCCAGTCGGGGTGTGGACCTGGCCACTGGCCCC AGCAGGACACCCCTTCGGGCCAAGAACAAGGTCCACCCCAGCAGCACTTAG |
| |
| SEQ ID NO: 36: CD16.7-CTTdNLS |

### Table 5-3

**Table 5-3**

| |
|---|
| MWQLLLPTALLLLVSAGMRTEDLPKAVVFLEPQWYSVLEKDSVTLKCQGAYSPEDNSTQWFHNESLISSQASSYFIDAATVNDSG EYRCQTNLSTLSDPVQLEVHIGWLLLQAPRWVFKEEDPIHLRCHSWKNTALHKVTYLQNGKDRKYFHHNSDFHIPKATLKDSGSY FCRGLVGSKNVSSETVNITITQGLAVSTISSFSPPGADPPRASALPAPPTGSALPDPQTASALPDPPAASALPAALAVISFLLGL GLGVACVLARTRVILRWRYHALRGELYRPAWEPQDYEMVELFFEGMEPLPSRSSRGSKVSPDVPPPSAGSDASHPSTSSSQLDGL SVSLGRLGTRCEPEPSRLQAVFEALLTQFDRLNQATEDVYQLEQQLHSLQGRRSSRAPAGSSRGPSPGLRPALPSRLARASRGVD LATGPSRTPLRAKNKVHPSST |
| |
| SEQ ID NO: 37: CD16. 7-CTTdNLS |
| ATGTGGCAGCTGCTCCTCCCAACTGCTCTGCTACTTCTAGTTTCAGCTGGCATGCGGACTGAAGATCTCCCAAAGGCTGTGGTGT TCCTGGAGCCTCAATGGTACAGCGTGCTTGAGAAGGACAGTGTGACTCTGAAGTGCCAGGGAGCCTACTCCCCTGAGGACAATTC CACACAGTGGTTTCACAATGAGAGCCTCATCTCAAGCCAGGCCTCGAGCTACTTCATTGACGCTGCCACAGTCAACGACAGTGGA GAGTACAGGTGCCAGACAAACCTCTCCACCCTCAGTGACCCGGTGCAGCTAGAAGTCCATATCGGCTGGCTGTTGCTCCAGGCCC CTCGGTGGGTGTTCAAGGAGGAAGACCCTATTCACCTGAGGTGTCACAGCTGGAAGAACACTGCTCTGCATAAGGTCACATATTT ACAGAATGGCAAAGACAGGAAGTATTTTCATCATAATTCTGACTTCCACATTCCAAAAGCCACACTCAAAGATAGCGGCTCCTAC TTCTGCAGGGGGCTTGTTGGGAGTAAAAATGTGTCTTCAGAGACTGTGAACATCACCATCACTCAAGGTTTGGCAGTGTCAACCA TCTCATCATTCTCTCCACCTGGCGCGGATCCACCAAGGGCCTCTGCCCTCCCTGCCCCACCGACAGGCTCCGCCCTCCCTGACCC GCAGACAGCCTCTGCCCTCCCTGACCCGCCAGCAGCCTCTGCCCTCCCTGCGGCCCTGGCGGTGATCTCCTTCCTCCTCGGGCTG GGCCTGGGGGTGGCGTGTGTGCTGGCGAGGACGCGTgttattctccgctggcgctaccacgccttgcgtggagagctgtaccggc cggcctgggagccccaggactacgagatggtggagttgttctttgaagggatggagccgctgccctctcgctcctccaggggctc caaggtatccccggatgtgcccccacccagcgctggctccgatgcctcgcacccctccacctcctccagccagctggatgggctg agcgtgagcctgggccggctggggacaaggtgtgagcctgagccctcccgcctccaagccgtgttcgaggccctgctcacccagt ttgaccgactcaaccaggccacagaggacgtctaccagctggagcagcagctgcacagcctgcaaggccgcaggagcagccgggc gcccgccggatcttcccgtggcccatccccgggcctgcggccagcactgcccagccgccttgcccgggccagtcggggtgtggac ctggccactggccccagcaggacaccccttcgggccaagaacaaggtccaccccagcagcacttaa |
| |
| SEQ ID NO: 38: CD16. 7-CTT4104-4203 |
| MWQLLLPTALLLLVSAGMRTEDLPKAVVFLEPQWYSVLEKDSVTLKCQGAYSPEDNSTQWFHNESLISSQASSYFIDAATVNDSG EYRCQTNLSTLSDPVQLEVHIGWLLLQAPRWVFKEEDPIHLRCHSWKNTALHKVTYLQNGKDRKYFHHNSDFHIPKATLKDSGSY FCRGLVGSKNVSSETVNITITQGLAVSTISSFSPPGADPPRASALPAPPTGSALPDPQTASALPDPPAASALPAALAVISFLLGL GLGVACVLARTRVILRWRYHALRGELYRPAWEPQDYEMVELFLRRLRLWMGLSKVKEFRHKVRFEGMEPLPSRSSRGSKVSPDVP PPSAGSDASHPSTSSSQLDGLSVSLGR |
| |
| SEQ ID NO: 39: CD16. 7-CTT4104-4203 |
| ATGTGGCAGCTGCTCCTCCCAACTGCTCTGCTACTTCTAGTTTCAGCTGGCATGCGGACTGAAGATCTCCCAAAGGCTGTGGTGT TCCTGGAGCCTCAATGGTACAGCGTGCTTGAGAAGGACAGTGTGACTCTGAAGTGCCAGGGAGCCTACTCCCCTGAGGACAATTC CACACAGTGGTTTCACAATGAGAGCCTCATCTCAAGCCAGGCCTCGAGCTACTTCATTGACGCTGCCACAGTCAACGACAGTGGA GAGTACAGGTGCCAGACAAACCTCTCCACCCTCAGTGACCCGGTGCAGCTAGAAGTCCATATCGGCTGGCTGTTGCTCCAGGCCC CTCGGTGGGTGTTCAAGGAGGAAGACCCTATTCACCTGAGGTGTCACAGCTGGAAGAACACTGCTCTGCATAAGGTCACATATTT ACAGAATGGCAAAGACAGGAAGTATTTTCATCATAATTCTGACTTCCACATTCCAAAAGCCACACTCAAAGATAGCGGCTCCTAC |

### Table 5-4

**Table 5-4**

| |
|---|
| TTCTGCAGGGGGCTTGTTGGGAGTAAAAATGTGTCTTCAGAGACTGTGAACATCACCATCACTCAAGGTTTGGCAGTGTCAACCA TCTCATCATTCTCTCCACCTGGCGCGGATCCACCAAGGGCCTCTGCCCTCCCTGCCCCACCGACAGGCTCCGCCCTCCCTGACCC GCAGACAGCCTCTGCCCTCCCTGACCCGCCAGCAGCCTCTGCCCTCCCTGCGGCCCTGGCGGTGATCTCCTTCCTCCTCGGGCTG GGCCTGGGGGTGGCGTGTGTGCTGGCGAGGACGCGTgttattctccgctggcgctaccacgccttgcgtggagagctgtaccggc cggcctgggagccccaggactacgagatggtggagttgttcctgcgcaggctgcgcctctggatgggcctcagcaaggtcaagga gttccgccacaaagtccgctttgaagggatggagccgctgccctctcgctcctccaggggctccaaggtatccccggatgtgccc ccacccagcgctggctccgatgcctcgcacccctccacctcctccagccagctggatgggctgagcgtgagcctgggccggtag |
| |
| SEQ ID NO: 40: CD16. 7-CTT4154-4253 |
| MWQLLLPTALLLLVSAGMRTEDLPKAVVFLEPQWYSVLEKDSVTLKCQGAYSPEDNSTQWFHNESLISSQASSYFIDAATVNDSG EYRCQTNLSTLSDPVQLEVHIGWLLLQAPRWVFKEEDPIHLRCHSWKNTALHKVTYLQNGKDRKYFHHNSDFHIPKATLKDSGSY FCRGLVGSKNVSSETVNITITQGLAVSTISSFSPPGADPPRASALPAPPTGSALPDPQTASALPDPPAASALPAALAVISFLLGL GLGVACVLARTRRFEGMEPLPSRSSRGSKVSPDVPPPSAGSDASHPSTSSSQLDGLSVSLGRLGTRCEPEPSRLQAVFEALLTQF DRLNQATEDVYQLEQQLHSLQGRRSSR |
| |
| SEQ ID NO: 41: CD16. 7-CTT4154-4253 |
| ATGTGGCAGCTGCTCCTCCCAACTGCTCTGCTACTTCTAGTTTCAGCTGGCATGCGGACTGAAGATCTCCCAAAGGCTGTGGTGT TCCTGGAGCCTCAATGGTACAGCGTGCTTGAGAAGGACAGTGTGACTCTGAAGTGCCAGGGAGCCTACTCCCCTGAGGACAATTC CACACAGTGGTTTCACAATGAGAGCCTCATCTCAAGCCAGGCCTCGAGCTACTTCATTGACGCTGCCACAGTCAACGACAGTGGA GAGTACAGGTGCCAGACAAACCTCTCCACCCTCAGTGACCCGGTGCAGCTAGAAGTCCATATCGGCTGGCTGTTGCTCCAGGCCC CTCGGTGGGTGTTCAAGGAGGAAGACCCTATTCACCTGAGGTGTCACAGCTGGAAGAACACTGCTCTGCATAAGGTCACATATTT ACAGAATGGCAAAGACAGGAAGTATTTTCATCATAATTCTGACTTCCACATTCCAAAAGCCACACTCAAAGATAGCGGCTCCTAC TTCTGCAGGGGGCTTGTTGGGAGTAAAAATGTGTCTTCAGAGACTGTGAACATCACCATCACTCAAGGTTTGGCAGTGTCAACCA TCTCATCATTCTCTCCACCTGGCGCGGATCCACCAAGGGCCTCTGCCCTCCCTGCCCCACCGACAGGCTCCGCCCTCCCTGACCC GCAGACAGCCTCTGCCCTCCCTGACCCGCCAGCAGCCTCTGCCCTCCCTGCGGCCCTGGCGGTGATCTCCTTCCTCCTCGGGCTG GGCCTGGGGGTGGCGTGTGTGCTGGCGAGGACGCGTcgctttgaagggatggagccgctgccctctcgctcctccaggggctcca aggtatccccggatgtgcccccacccagcgctggctccgatgcctcgcacccctccacctcctccagccagctggatgggctgag cgtgagcctgggccggctggggacaaggtgtgagcctgagccctcccgcctccaagccgtgttcgaggccctgctcacccagttt gaccgactcaaccaggccacagaggacgtctaccagctggagcagcagctgcacagcctgcaaggccgcaggagcagccggtag |
| |
| SEQ ID NO: 42: CD16. 7-CTT4154-4243 |
| MWQLLLPTALLLLVSAGMRTEDLPKAVVFLEPQWYSVLEKDSVTLKCQGAYSPEDNSTQWFHNESLISSQASSYFIDAATVNDSG EYRCQTNLSTLSDPVQLEVHIGWLLLQAPRWVFKEEDPIHLRCHSWKNTALHKVTYLQNGKDRKYFHHNSDFHIPKATLKDSGSY FCRGLVGSKNVSSETVNITITQGLAVSTISSFSPPGADPPRASALPAPPTGSALPDPQTASALPDPPAASALPAALAVISFLLGL GLGVACVLARTRRFEGMEPLPSRSSRGSKVSPDVPPPSAGSDASHPSTSSSQLDGLSVSLGRLGTRCEPEPSRLQAVFEALLTQF DRLNQATEDVYQLEQQL |
| |
| SEQ ID NO: 43: CD16. 7-CTT4154-4243 |
| ATGTGGCAGCTGCTCCTCCCAACTGCTCTGCTACTTCTAGTTTCAGCTGGCATGCGGACTGAAGATCTCCCAAAGGCTGTGGTGT |

### Table 5-5

**Table 5-5**

| |
|---|
| TCCTGGAGCCTCAATGGTACAGCGTGCTTGAGAAGGACAGTGTGACTCTGAAGTGCCAGGGAGCCTACTCCCCTGAGGACAATTC CACACAGTGGTTTCACAATGAGAGCCTCATCTCAAGCCAGGCCTCGAGCTACTTCATTGACGCTGCCACAGTCAACGACAGTGGA GAGTACAGGTGCCAGACAAACCTCTCCACCCTCAGTGACCCGGTGCAGCTAGAAGTCCATATCGGCTGGCTGTTGCTCCAGGCCC CTCGGTGGGTGTTCAAGGAGGAAGACCCTATTCACCTGAGGTGTCACAGCTGGAAGAACACTGCTCTGCATAAGGTCACATATTT ACAGAATGGCAAAGACAGGAAGTATTTTCATCATAATTCTGACTTCCACATTCCAAAAGCCACACTCAAAGATAGCGGCTCCTAC TTCTGCAGGGGGCTTGTTGGGAGTAAAAATGTGTCTTCAGAGACTGTGAACATCACCATCACTCAAGGTTTGGCAGTGTCAACCA TCTCATCATTCTCTCCACCTGGCGCGGATCCACCAAGGGCCTCTGCCCTCCCTGCCCCACCGACAGGCTCCGCCCTCCCTGACCC GCAGACAGCCTCTGCCCTCCCTGACCCGCCAGCAGCCTCTGCCCTCCCTGCGGCCCTGGCGGTGATCTCCTTCCTCCTCGGGCTG GGCCTGGGGGTGGCGTGTGTGCTGGCGAGGACGCGTcgctttgaagggatggagccgctgccctctcgctcctccaggggctcca aggtatccccggatgtgcccccacccagcgctggctccgatgcctcgcacccctccacctcctccagccagctggatgggctgag cgtgagcctgggccggctggggacaaggtgtgagcctgagccctcccgcctccaagccgtgttcgaggccctgctcacccagttt gaccgactcaaccaggccacagaggacgtctaccagctggagcagcagctgtag |
| |
| SEQ ID NO: 44: CD16. 7-CTT4193-4303 |
| MWQLLLPTALLLLVSAGMRTEDLPKAVVFLEPQWYSVLEKDSVTLKCQGAYSPEDNSTQWFHNESLISSQASSYFIDAATVNDSG EYRCQTNLSTLSDPVQLEVHIGWLLLQAPRWVFKEEDPIHLRCHSWKNTALHKVTYLQNGKDRKYFHHNSDFHIPKATLKDSGSY FCRGLVGSKNVSSETVNITITQGLAVSTISSFSPPGADPPRASALPAPPTGSALPDPQTASALPDPPAASALPAALAVISFLLGL GLGVACVLARTRQLDGLSVSLGRLGTRCEPEPSRLQAVFEALLTQFDRLNQATEDVYQLEQQLHSLQGRRSSRAPAGSSRGPSPG LRPALPSRLARASRGVDLATGPSRTPLRAKNKVHPSST |
| |
| SEQ ID NO: 45: CD16. 7-CTT4193-4303 |
| ATGTGGCAGCTGCTCCTCCCAACTGCTCTGCTACTTCTAGTTTCAGCTGGCATGCGGACTGAAGATCTCCCAAAGGCTGTGGTGT TCCTGGAGCCTCAATGGTACAGCGTGCTTGAGAAGGACAGTGTGACTCTGAAGTGCCAGGGAGCCTACTCCCCTGAGGACAATTC CACACAGTGGTTTCACAATGAGAGCCTCATCTCAAGCCAGGCCTCGAGCTACTTCATTGACGCTGCCACAGTCAACGACAGTGGA GAGTACAGGTGCCAGACAAACCTCTCCACCCTCAGTGACCCGGTGCAGCTAGAAGTCCATATCGGCTGGCTGTTGCTCCAGGCCC CTCGGTGGGTGTTCAAGGAGGAAGACCCTATTCACCTGAGGTGTCACAGCTGGAAGAACACTGCTCTGCATAAGGTCACATATTT ACAGAATGGCAAAGACAGGAAGTATTTTCATCATAATTCTGACTTCCACATTCCAAAAGCCACACTCAAAGATAGCGGCTCCTAC TTCTGCAGGGGGCTTGTTGGGAGTAAAAATGTGTCTTCAGAGACTGTGAACATCACCATCACTCAAGGTTTGGCAGTGTCAACCA TCTCATCATTCTCTCCACCTGGCGCGGATCCACCAAGGGCCTCTGCCCTCCCTGCCCCACCGACAGGCTCCGCCCTCCCTGACCC GCAGACAGCCTCTGCCCTCCCTGACCCGCCAGCAGCCTCTGCCCTCCCTGCGGCCCTGGCGGTGATCTCCTTCCTCCTCGGGCTG GGCCTGGGGGTGGCGTGTGTGCTGGCGAGGACGCGTcagctggatgggctgagcgtgagcctgggccggctggggacaaggtgtg agcctgagccctcccgcctccaagccgtgttcgaggccctgctcacccagtttgaccgactcaaccaggccacagaggacgtcta ccagctggagcagcagctgcacagcctgcaaggccgcaggagcagccgggcgcccgccggatcttcccgtggcccatccccgggc ctgcggccagcactgcccagccgccttgcccgggccagtcggggtgtggacctggccactggccccagcaggacaccccttcggg ccaagaacaaggtccaccccagcagcacttag |
| |
| SEQ ID NO: 46: CD16. 7-CTT4204-4303 |
| MWQLLLPTALLLLVSAGMRTEDLPKAVVFLEPQWYSVLEKDSVTLKCQGAYSPEDNSTQWFHNESLISSQASSYFIDAATVNDSG EYRCQTNLSTLSDPVQLEVHIGWLLLQAPRWVFKEEDPIHLRCHSWKNTALHKVTYLQNGKDRKYFHHNSDFHIPKATLKDSGSY |

### Table 5-6

**Table 5-6**

| |
|---|
| FCRGLVGSKNVSSETVNITITQGLAVSTISSFSPPGADPPRASALPAPPTGSALPDPQTASALPDPPAASALPAALAVISFLLGL GLGVACVLARTRLGTRCEPEPSRLQAVFEALLTQFDRLNQATEDVYQLEQQLHSLQGRRSSRAPAGSSRGPSPGLRPALPSRLAR ASRGVDLATGPSRTPLRAKNKVHPSST |
| |
| SEQ ID NO: 47: CD16. 7-CTT4204-4303 |
| ATGTGGCAGCTGCTCCTCCCAACTGCTCTGCTACTTCTAGTTTCAGCTGGCATGCGGACTGAAGATCTCCCAAAGGCTGTGGTGT TCCTGGAGCCTCAATGGTACAGCGTGCTTGAGAAGGACAGTGTGACTCTGAAGTGCCAGGGAGCCTACTCCCCTGAGGACAATTC CACACAGTGGTTTCACAATGAGAGCCTCATCTCAAGCCAGGCCTCGAGCTACTTCATTGACGCTGCCACAGTCAACGACAGTGGA GAGTACAGGTGCCAGACAAACCTCTCCACCCTCAGTGACCCGGTGCAGCTAGAAGTCCATATCGGCTGGCTGTTGCTCCAGGCCC CTCGGTGGGTGTTCAAGGAGGAAGACCCTATTCACCTGAGGTGTCACAGCTGGAAGAACACTGCTCTGCATAAGGTCACATATTT ACAGAATGGCAAAGACAGGAAGTATTTTCATCATAATTCTGACTTCCACATTCCAAAAGCCACACTCAAAGATAGCGGCTCCTAC TTCTGCAGGGGGCTTGTTGGGAGTAAAAATGTGTCTTCAGAGACTGTGAACATCACCATCACTCAAGGTTTGGCAGTGTCAACCA TCTCATCATTCTCTCCACCTGGCGCGGATCCACCAAGGGCCTCTGCCCTCCCTGCCCCACCGACAGGCTCCGCCCTCCCTGACCC GCAGACAGCCTCTGCCCTCCCTGACCCGCCAGCAGCCTCTGCCCTCCCTGCGGCCCTGGCGGTGATCTCCTTCCTCCTCGGGCTG GGCCTGGGGGTGGCGTGTGTGCTGGCGAGGACGCGTctggggacaaggtgtgagcctgagccctcccgcctccaagccgtgttcg aggccctgctcacccagtttgaccgactcaaccaggccacagaggacgtctaccagctggagcagcagctgcacagcctgcaagg ccgcaggagcagccgggcgcccgccggatcttcccgtggcccatccccgggcctgcggccagcactgcccagccgccttgcccgg gccagtcggggtgtggacctggccactggccccagcaggacaccccttcgggccaagaacaaggtccaccccagcagcacttag |
| |
| SEQ ID NO: 48: CD16.7-CTT4204-4253 |
| MWQLLLPTALLLLVSAGMRTEDLPKAVVFLEPQWYSVLEKDSVTLKCQGAYSPEDNSTQWFHNESLISSQASSYFIDAATVNDSG EYRCQTNLSTLSDPVQLEVHIGWLLLQAPRWVFKEEDPIHLRCHSWKNTALHKVTYLQNGKDRKYFHHNSDFHIPKATLKDSGSY FCRGLVGSKNVSSETVNITITQGLAVSTISSFSPPGADPPRASALPAPPTGSALPDPQTASALPDPPAASALPAALAVISFLLGL GLGVACVLARTRLGTRCEPEPSRLQAVFEALLTQFDRLNQATEDVYQLEQQLHSLQGRRSSR |
| |
| SEQ ID NO: 49: CD16. 7-CTT4204-4253 |
| ATGTGGCAGCTGCTCCTCCCAACTGCTCTGCTACTTCTAGTTTCAGCTGGCATGCGGACTGAAGATCTCCCAAAGGCTGTGGTGT TCCTGGAGCCTCAATGGTACAGCGTGCTTGAGAAGGACAGTGTGACTCTGAAGTGCCAGGGAGCCTACTCCCCTGAGGACAATTC CACACAGTGGTTTCACAATGAGAGCCTCATCTCAAGCCAGGCCTCGAGCTACTTCATTGACGCTGCCACAGTCAACGACAGTGGA GAGTACAGGTGCCAGACAAACCTCTCCACCCTCAGTGACCCGGTGCAGCTAGAAGTCCATATCGGCTGGCTGTTGCTCCAGGCCC CTCGGTGGGTGTTCAAGGAGGAAGACCCTATTCACCTGAGGTGTCACAGCTGGAAGAACACTGCTCTGCATAAGGTCACATATTT ACAGAATGGCAAAGACAGGAAGTATTTTCATCATAATTCTGACTTCCACATTCCAAAAGCCACACTCAAAGATAGCGGCTCCTAC TTCTGCAGGGGGCTTGTTGGGAGTAAAAATGTGTCTTCAGAGACTGTGAACATCACCATCACTCAAGGTTTGGCAGTGTCAACCA TCTCATCATTCTCTCCACCTGGCGCGGATCCACCAAGGGCCTCTGCCCTCCCTGCCCCACCGACAGGCTCCGCCCTCCCTGACCC GCAGACAGCCTCTGCCCTCCCTGACCCGCCAGCAGCCTCTGCCCTCCCTGCGGCCCTGGCGGTGATCTCCTTCCTCCTCGGGCTG GGCCTGGGGGTGGCGTGTGTGCTGGCGAGGACGCGTctggggacaaggtgtgagcctgagccctcccgcctccaagccgtgttcg aggccctgctcacccagtttgaccgactcaaccaggccacagaggacgtctaccagctggagcagcagctgcacagcctgcaagg ccgcaggagcagccggtag |
| |

### Table 5-7

**Table 5-7**

| |
|---|
| SEQ ID NO: 50: CD16. 7-CTT4254-4303 |
| MWQLLLPTALLLLVSAGMRTEDLPKAVVFLEPQWYSVLEKDSVTLKCQGAYSPEDNSTQWFHNESLISSQASSYFIDAATVNDSG EYRCQTNLSTLSDPVQLEVHIGWLLLQAPRWVFKEEDPIHLRCHSWKNTALHKVTYLQNGKDRKYFHHNSDFHIPKATLKDSGSY FCRGLVGSKNVSSETVNITITQGLAVSTISSFSPPGADPPRASALPAPPTGSALPDPQTASALPDPPAASALPAALAVISFLLGL GLGVACVLARTRAPAGSSRGPSPGLRPALPSRLARASRGVDLATGPSRTPLRAKNKVHPSST |
| |
| SEQ ID NO: 51: CD16. 7-C174254-4303 |
| ATGTGGCAGCTGCTCCTCCCAACTGCTCTGCTACTTCTAGTTTCAGCTGGCATGCGGACTGAAGATCTCCCAAAGGCTGTGGTGT TCCTGGAGCCTCAATGGTACAGCGTGCTTGAGAAGGACAGTGTGACTCTGAAGTGCCAGGGAGCCTACTCCCCTGAGGACAATTC CACACAGTGGTTTCACAATGAGAGCCTCATCTCAAGCCAGGCCTCGAGCTACTTCATTGACGCTGCCACAGTCAACGACAGTGGA GAGTACAGGTGCCAGACAAACCTCTCCACCCTCAGTGACCCGGTGCAGCTAGAAGTCCATATCGGCTGGCTGTTGCTCCAGGCCC CTCGGTGGGTGTTCAAGGAGGAAGACCCTATTCACCTGAGGTGTCACAGCTGGAAGAACACTGCTCTGCATAAGGTCACATATTT ACAGAATGGCAAAGACAGGAAGTATTTTCATCATAATTCTGACTTCCACATTCCAAAAGCCACACTCAAAGATAGCGGCTCCTAC TTCTGCAGGGGGCTTGTTGGGAGTAAAAATGTGTCTTCAGAGACTGTGAACATCACCATCACTCAAGGTTTGGCAGTGTCAACCA TCTCATCATTCTCTCCACCTGGCGCGGATCCACCAAGGGCCTCTGCCCTCCCTGCCCCACCGACAGGCTCCGCCCTCCCTGACCC GCAGACAGCCTCTGCCCTCCCTGACCCGCCAGCAGCCTCTGCCCTCCCTGCGGCCCTGGCGGTGATCTCCTTCCTCCTCGGGCTG GGCCTGGGGGTGGCGTGTGTGCTGGCGAGGACGCGTGCGCCCGCCGGATCTTCCCGTGGCCCATCCCCGGGCCTGCGGCCAGCAC TGCCCAGCCGCCTTGCCCGGGCCAGTCGGGGTGTGGACCTGGCCACTGGCCCCAGCAGGACACCCCTTCGGGCCAAGAACAAGGT CCACCCCAGCAGCACTTAG |
| |
| SEQ ID NO: 52: FAS-CTT4204-4303 |
| MLGIWTLLPLVLTSVARLSSKSVNAQVTDINSKGLELRKTVTTVETQNLEGLHHDGQFCHKPCPPGERKARDCTVNGDEPDCVPC QEGKEYTDKAHFSSKCRRCRLCDEGHGLEVEINCTRTQNTKCRCKPNFFCNSTVCEHCDPCTKCEHGIIKECTLTSNTKCKEEGS RSNLGWLCLLLLPIPLIVWVLGTRCEPEPSRLQAVFEALLTQFDRLNQATEDVYQLEQQLHSLQGRRSSRAPAGSSRGPSPGLRP ALPSRLARASRGVDLATGPSRTPLRAKNKVHPSST |
| |
| SEQ ID NO: 53: FAS-CTT4204-4303 |
| ATGCTGGGCATCTGGACCCTCCTACCTCTGGTTCTTACGTCTGTTGCTAGATTATCGTCCAAAAGTGTTAATGCCCAAGTGACTG ACATCAACTCCAAGGGATTGGAATTGAGGAAGACTGTTACTACAGTTGAGACTCAGAACTTGGAAGGCCTGCATCATGATGGCCA ATTCTGCCATAAGCCCTGTCCTCCAGGTGAAAGGAAAGCTAGGGACTGCACAGTCAATGGGGATGAACCAGACTGCGTGCCCTGC CAAGAAGGGAAGGAGTACACAGACAAAGCCCATTTTTCTTCCAAATGCAGAAGATGTAGATTGTGTGATGAAGGACATGGCTTAG AAGTGGAAATAAACTGCACCCGGACCCAGAATACCAAGTGCAGATGTAAACCAAACTTTTTTTGTAACTCTACTGTATGTGAACA CTGTGACCCTTGCACCAAATGTGAACATGGAATCATCAAGGAATGCACACTCACCAGCAACACCAAGTGCAAAGAGGAAGGATCC AGATCTAACTTGGGGTGGCTTTGTCTTCTTCTTTTGCCAATTCCACTAATTGTTTGGGTGCTGGGGACAAGGTGTGAGCCTGAGC CCTCCCGCCTCCAAGCCGTGTTCGAGGCCCTGCTCACCCAGTTTGACCGACTCAACCAGGCCACAGAGGACGTCTACCAGCTGGA GCAGCAGCTGCACAGCCTGCAAGGCCGCAGGAGCAGCCGGGCGCCCGCCGGATCTTCCCGTGGCCCATCCCCGGGCCTGCGGCCA GCACTGCCCAGCCGCCTTGCCCGGGCCAGTCGGGGTGTGGACCTGGCCACTGGCCCCAGCAGGACACCCCTTCGGGCCAAGAACA AGGTCCACCCCAGCAGCACTTAA |
| |

Table 5-8

**Table 5-8**

| |
|---|
| SEQ ID NO: 54: FAS-CTT4204-4253 |
| MLGIWTLLPLVLTSVARLSSKSVNAQVTDINSKGLELRKTVTTVETQNLEGLHHDGQFCHKPCPPGERKARDCTVNGDEPDCVPC QEGKEYTDKAHFSSKCRRCRLCDEGHGLEVEINCTRTQNTKCRCKPNFFCNSTVCEHCDPCTKCEHGIIKECTLTSNTKCKEEGS RSNLGWLCLLLLPIPLIVWVLGTRCEPEPSRLQAVFEALLTQFDRLNQATEDVYQLEQQLHSLQGRRSSR |
| |
| SEQ ID NO: 55: FAS-CTT4204-4253 |
| ATGCTGGGCATCTGGACCCTCCTACCTCTGGTTCTTACGTCTGTTGCTAGATTATCGTCCAAAAGTGTTAATGCCCAAGTGACTG ACATCAACTCCAAGGGATTGGAATTGAGGAAGACTGTTACTACAGTTGAGACTCAGAACTTGGAAGGCCTGCATCATGATGGCCA ATTCTGCCATAAGCCCTGTCCTCCAGGTGAAAGGAAAGCTAGGGACTGCACAGTCAATGGGGATGAACCAGACTGCGTGCCCTGC CAAGAAGGGAAGGAGTACACAGACAAAGCCCATTTTTCTTCCAAATGCAGAAGATGTAGATTGTGTGATGAAGGACATGGCTTAG AAGTGGAAATAAACTGCACCCGGACCCAGAATACCAAGTGCAGATGTAAACCAAACTTTTTTTGTAACTCTACTGTATGTGAACA CTGTGACCCTTGCACCAAATGTGAACATGGAATCATCAAGGAATGCACACTCACCAGCAACACCAAGTGCAAAGAGGAAGGATCC AGATCTAACTTGGGGTGGCTTTGTCTTCTTCTTTTGCCAATTCCACTAATTGTTTGGGTGCTGGGGACAAGGTGTGAGCCTGAGC CCTCCCGCCTCCAAGCCGTGTTCGAGGCCCTGCTCACCCAGTTTGACCGACTCAACCAGGCCACAGAGGACGTCTACCAGCTGGA GCAGCAGCTGCACAGCCTGCAAGGCCGCAGGAGCAGCCGGTAG |

## Claims

1. A polynucleotide comprising a sequence encoding a shortened polycystin-1 (PC1) having cyst formation inhibitory activity, wherein the shortened PC1 comprises a C-terminal tail (CTT) region of PC1 or a part thereof.

2. The polynucleotide according to claim 1, wherein the shortened PC1 is a membrane-localized polypeptide.

3. A polynucleotide comprising a sequence encoding a shortened polycystin-1 (PC1), wherein the shortened PC1 is a membrane-localized polypeptide comprising a C-terminal tail (CTT) region of PC1 or a part thereof.

4. The polynucleotide according to any one of claims 1 to 3, wherein the shortened PC1 comprises the CTT region of PC1.

5. The polynucleotide according to any one of claims 1 to 3, wherein the shortened PC1 comprises the part of a CTT region of PC1.

6. The polynucleotide according to any one of claims 1 to 5, wherein the shortened PC1 further comprises at least part of a stalk of PC1.

7. The polynucleotide according to claim 6, wherein the shortened PC1 further comprises at least one transmembrane region.

8. The polynucleotide according to any one of claims 1 to 5, wherein the shortened PC1 further comprises a signal sequence.

9. The polynucleotide according to claim 8, wherein the shortened PC1 further comprises at least one transmembrane region.

10. A shortened polycystin-1 (PC1) polypeptide encoded by the polynucleotide according to any one of claims 1 to 9.

11. A vector comprising the polynucleotide according to any one of claims 1 to 9.

12. A pharmaceutical composition for treating a PKD1-related disease, comprising the polynucleotide according to any one of claims 1 to 9, the polypeptide according to claim 10, or the vector according to claim 11.

13. A pharmaceutical composition for inhibiting cyst formation in kidney, comprising a polynucleotide encoding a shortened polycystin-1 (PC1) comprising a C-terminal tail (CTT) region of PC1 or a part thereof, or a vector comprising the polynucleotide, wherein the pharmaceutical composition inhibits the cyst formation by localizing the CTT region or the part thereof to cell membrane.
